# EUROPEAN PATENT SPECIFICATION

(11) **EP 1 042 320 B1**
(45) Date of publication and mention of the grant of the patent: **13.02.2002**
(21) Application number: 98957267.2
(22) Date of filing: 18.11.1998
(51) Int. Cl.: C07D 413/04, A61K 31/505

(54) **NEW COMPOUNDS WHICH ARE P2-PURINOCEPTOR 7-TRANSMEMBRANE (TM) G-PROTEIN COUPLED RECEPTOR ANTAGONISTS**
NEUE VERBINDUNGEN, WELCHE ANTAGONISTEN SIND FÜR P2-PURINOREZEPTOR 7-TRANSMEMBRAN (TM) G-PROTEIN GEKOPPELTE REZEPTOREN
NOUVEAUX COMPOSES ANTAGONISTES DU RECEPTEUR COUPLE A LA PROTEINE G TRANSMEMBRANAIRE-7 (TM) DU PURINORECEPTEUR P2

(30) Priority: 21.11.1997 SE 9704272; 21.04.1998 SE 9801397
(43) Date of publication of application: 11.10.2000
(73) Proprietor: AstraZeneca UK Limited, London W1Y 6LN (GB)
(72) Inventor: KINDON, Nicholas, Loughborough, Leics. LE11 5RH (GB); MEGHANI, Premji, Loughborough, Leics. LE11 5RH (GB); THOM, Stephen, Loughborough, Leics. LE11 5RH (GB)
(86) International application number: SE9802088
(87) International publication number: WO9926944

(56) References cited:
- WO-A-98/45309
- WO-A-98/54180
- WO-A-99/02501
- WO-A-99/05123
- WO-A1-97/08170
- KOROLKOVAS A.: 'Essentials of medicinal chemistry, page 74-84', JOHN WILEY & SONS, NEW YORK
- GRINGAUX A.: 'Introduction to medicinal chemistry', WILEY-VCH, NEW YORK

## Description

The invention provides new pharmaceutically active compounds, compositions containing them and processes for their preparation. The compounds are useful in therapy because they are P2-purinoceptor 7-transmembrane (TM) G-protein coupled receptor antagonists.

ATP receptors have been shown to be present on a wide number of different cell types (Dubyak *et al* Am J Physiol (1993) 265, C577-C606). Neutrophils, monocytes and macrophages have been isolated from several species including humans and ATP and/or UTP have been shown to increase intracellular calcium levels. Activation of these receptors on leukocytes can either directly stimulate certain types of inflammatory response or can prime the effector cells to other inflammatory mediators *in vivo*. ATP can upregulate the expression of adhesion molecules (Freyer *et al* J Immun. (1988) 141, 580-586) which causes enhanced adhesion of circulating leukocytes to endothelial cells and their enhanced migration into the tissue space. ATP has also been shown to promote chemotaxis of both neutrophils and eosinophils (Verghese *et al J.* B. C. (1996) 271, 15597-15601 and Burders *et al* Blood (1993) 81, 49-55) which may promote an inflammatory response. ATP priming of neutrophils can also potentiate superoxide production (Seifert *et al* Eur J Biochem (1989) 181, 277-285). ATP receptors are also present on a number of other cell types such as chondrocytes, keratinocytes, microglia and goblet cells (Leong *et al* BBA (1994) 1201, 298-304; Pillai *et al* J Clin Invest (1992) 90, 42-51; Walz *et al* J Neuroscience (1993) 13, 4403-4411 and Abdullah *et al* Biochem J (1996) 316, 943-951). Stimulation of the receptors on these cells can stimulate or enhance inflammatory responses and antagonist of the receptor may therefore be of use in a number of inflammatory diseases such as asthma, inflammatory bowel disease, ARDS, psoriasis, rheumatoid arthritis, myocardial ischaemia, COPD, cystic fibrosis, atherosclerosis, restenosis, peridontal disease, septic shock, osteoarthritis and stroke. ATP receptors have also been reported on tumour cells (Dubyak et al J. Biol. Chem., (1985) 260, 10653 - 10661 and Wagner *et al* Gastroenterology, (1997), 112(4) suppl. page A1198) and may be involved in the development of cancer. Antagonists may therefore be useful in treatment of cancer.

The invention provides new pharmaceutically active compounds, compositions containing them and processes for their preparation. The compounds are useful in therapy as P2-purinoceptor-7-transmembrane (TM) G-protein coupled receptor antagonists.

It has now been found that a series of pyrimidine derivatives are useful as P2-purinoceptor 7-transmembrane (TM) G-protein coupled receptor antagonists. In a first aspect the invention therefore provides a compound of formula (I) or a salt thereof: 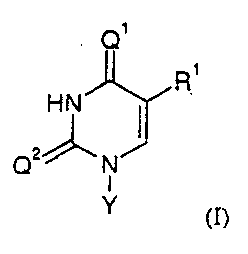 where
Y is hydrogen, C₁₋₄alkyl, C₁₋₄alkyl optionally substituted by hydroxy, alkoxy, amino, alkylamino, dialkylamino, phenyl, nitrogen and/or oxygen or optionally substituted by a C₃₋₈cycloalkyl ring which optionally contains I to 3 heteroatoms and optionally substituted by C₁₋₄ alkyl; or Y is a group of formula (i): 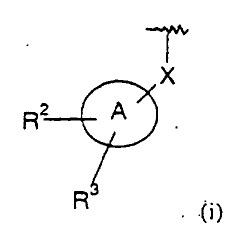 where
A is a pyridine, pyrimidine, thiazole, oxazole, thiophene, furan or pyridazine ring;
R¹ is a group of formula (ii): 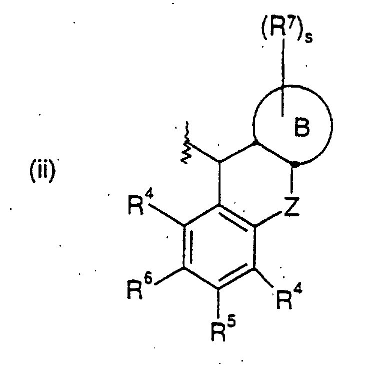 where
R⁴ is hydrogen, halogen, C₁₋₃alkoxy, C₁₋₃alkylthio or C₁₋₃alkyl (optionally substituted by one or more fluorine atoms);
R⁵ is hydrogen, hydroxy, halogen, C₁₋₃alkylthio, C₁₋₄alkyl (optionally substituted by one or more fluorine atoms), C₃₋₄cycloalkyl, MeOCH₂, MeSCH₂, phenyl, pyridyl, or C₁₋₃alkoxy; or R⁴ and R⁵ are -(CH₂)t- where t is 3 or 4 forming a fused ring;
R⁶ is hydrogen, halogen, C₁₋₃alkoxy, C₁₋₃alkylthio, or C₁₋₃alkyl (optionally substituted by one or more fluorine atoms); or R⁶ together with R⁴ is a group -(CH₂)t- where t is 3 or 4 forming a fused ring;
R⁷ is hydrogen, hydroxy, C₁₋₃alkoxy, amino. hydroxyC₁₋₃alkyl, -NHC₁₋₃alkyl, -NC₁₋₃dialkyl, -NHC₃₋₈cycloalkyl, -N₃₋₈cycloalkyl, -NHphenyl, -NHC₁₋₃alkylphenyl, (heterocycle) C₁₋₃alkyl-, (heterocycle)C₁₋₃alkylthio-, (heterocycle)C₁₋₃alkyloxy-, (heterocycle)C₁₋₃alkylamino-, (heterocycle)thio-, (heterocycle)oxy-, (heterocycle)amino-, C₁₋₃alkylthio-, cyano, thiol, C₁₋₃alkyl (optionally substituted by one or more fluorine atoms), -C₁₋₃alkylamino, -C₁₋₃alkylaminoalkyl, carboxamidoC₁₋₃alkyl, acetoxyC₁₋₃alkyl, or C₃₋₄cycloalkyl;
S is 1 or 2;
B is a pyrrole, thiophene, furan, oxazole, thiazole, pyridine, pyrimidine, pyridazine, pyrazine or triazine ring;
Z is a -CH=CH-, -CH₂CH₂-, or CH₂O;
R² is hydrogen. NO₂, NH₂, N(C₁₋₆alkyl)₂, CO₂H, CH₂OH, halogen, CO₂C₁₋₆alkyl, C₁₋₈alkyl optionally interrupted by one or more oxygen, nitrogen or sulphur atoms and optionally substituted by CO₂H or R² is hydroxy, imidazol-1-ylCH₂-, phenyl optionally substituted by CH₂CO₂H or CONR⁹R¹⁰ where R⁹ and R¹⁰ are independently hydrogen, C₁₋₆alkyl optionally substituted by hydroxy or CO₂H and/or optionally interrupted by oxygen, nitrogen or sulphur;
R³ is hydrogen, R¹¹CO₂H, R¹¹PO(OH)₂, R¹²tetrazol-5-yl, COR¹³, NR¹⁴R¹⁵, CH₂NR¹⁶CH₂CO₂H, C₁₋₈alkyl optionally interrupted by one or more oxygen, sulphur or nitrogen atoms and optionally substituted by CO₂H or R³ is a group of formula (iii):

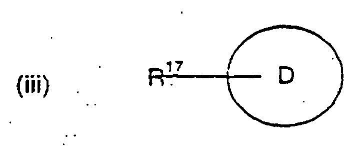

where D is a 4,5 or 6 membered saturated ring containing a nitrogen optionally substituted by hydroxy and substituted by CO₂H or CONH-het where het is tetrazol-5-yl or a thiazole or a thiadiazole ring substituted by CH₂CO₂H or D is a phenyl ring or a 5 membered aromatic heterocylic ring containing 1-3 heteroatoms selected from nitrogen, oxygen or sulphur optionally substituted by one or more groups selected from CF₃, CO₂H, CH₂OH, C₁₋₆alkyl optionally interrupted by one or more oxygen atoms, (CH₂)pCO₂H, C(CO₂H)=NOMe, tetrazol-5-yl, CH₂tetrazol-5-yl, CH₂CON(CH₂CO₂H)₂, or CH₂COR¹⁸;
where R¹¹ is OCH₂, (CH₂)p, SCH₂, CONHCH₂, NHCH(R¹⁹) or NR²⁰(CH₂)p;
R¹² is a bond, (CH₂)p, OCH₂, SCH₂, CONH, CONHCH₂, CONHCH₂CONH, NHCH₂CONH, NHCH(R⁹);
R¹³ is OH, N(CH₂CO₂H)₂, NHS(O)₂R²¹ or a group of formula (iv): 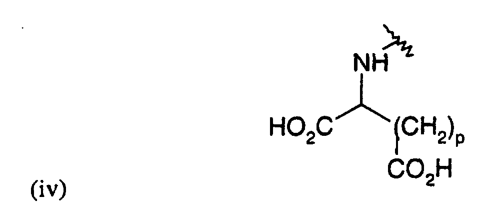
R¹⁴ and R¹⁵ are independently hydrogen, CH₂CO₂H, CHPh₂ or C(=S)CH₂CH₂CO₂H;
R¹⁶ is hydrogen, C₁₋₆alkyl or CO₂CH₂Ph;
R¹⁷ is a bond, sulphur atom, CONH, CH₂, CH₂O, OCH₂, a group -NR²²CH(CO₂H)CH₂- group or a group CONR²²(CH₂)pCONR²³ or NR²²(CH₂)pCONR²³;
R¹⁸ is a group of formula (iv) as defined above or a group of formula (v): 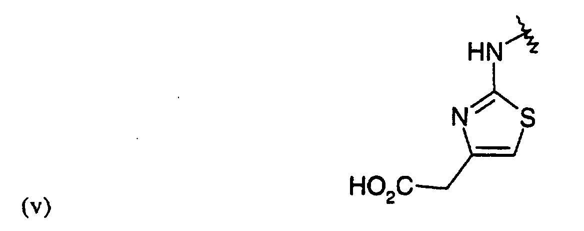
R¹⁹ is hydrogen, C₁₋₆ alkyl optionally substituted by hydroxy and/or optionally interrupted by oxygen, nitrogen or sulphur;
R²⁰ is hydrogen or C₁₋₆ alkyl;
p is 1 or 2;
R²¹ is NH₂ or C₁₋₆alkyl optionally interrupted by oxygen or nitrogen;
R²² and R²³ are independently hydrogen, C₁₋₆alkyl; and
Q¹ and Q² each independently represent an O or S atom.

Alkyl groups, whether alone or as part of another group, can be straight chain or branched.

Certain compounds of formula (I) are capable of existing in stereoisomeric forms including enantiomers and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

Suitably Y is hydrogen, C₁₋₄alkyl, C₁₋₄alkyl optionally substituted by hydroxy, alkoxy, amino, alkylamino, dialkylamino, phenyl, nitrogen and/or oxygen or optionally substituted by a C₃₋₈cycloalkyl ring which optionally contains 1 to 3 heteroatoms and optionally substituted by C₁₋₄ alkyl or Y is a group of formula (I) as defined above.
Preferably Y is hydrogen, C₁₋₄alkyl, C₁₋₄alkyl optionally substituted by hydroxy, nitrogen and/or oxygen or C₃₋₈cycloalkyl optionally containing 1 to 3 heteroatoms and optionally substituted by C₁₋₄ alkyl. More preferably Y is methyl, hydroxyethyl or methoxyethyl.

Suitably X is a bond or a C₁₋₃ alkylene group optionally interrupted by oxygen. Preferably X is a bond or a CH₂ group.

When Y is a group of formula (i) A is a pyridine, pyrimidine, thiazole, oxazole, thiophene, furan and pyridazine. Preferably A is furan, oxazole, pyrimidine or phenyl.

Suitably R⁴ is hydrogen, halogen, C₁₋₃alkoxy, C₁₋₃alkylthio or C₁₋₃alkyl (optionally substituted by one or more fluorine atoms). R⁴ at both positions can be the same or different. Preferably R⁴ is hydrogen.

Suitably R⁵ is hydrogen, hydroxy, halogen, C₁₋₃alkylthio, C₁₋₄alkyl (optionally substituted by one or more fluorine atoms), C₃₋₄cycloalkyl, MeOCH₂, MeSCH₂, phenyl. pyridyl. or C₁₋₃alkoxy; or R⁴ and the adjacent R⁶ group together form a group -(CH₂)t- where t is 3 or 4, that is R⁴ and R⁶ form a fused ring. Preferably R⁵ is halogen, hydrogen, hydroxy or C₁₋₄alkyl, more preferably halogen, methyl or ethyl.

Suitably R⁶ is hydrogen, halogen, C₁₋₃alkoxy, C₁₋₃alkylthio, or C₁₋₃alkyl (optionally substituted by one or more fluorine atoms): or R⁵ and R⁶ are -(CH₂)t- where t is 3 or 4 forming a fused ring. Preferably R⁶ is hydrogen or methyl.

Suitably R⁷ is hydrogen, hydroxy, C₁₋₃alkoxy, amino, hydroxyC₁₋₃alkyl, -NHC₁₋₃alkyl, -NC₁₋₃dialkyl, -NHC₃₋₈cycloalkyl, -N₃₋₈cycloalkyl, -NHphenyl, -NHC₁₋₃alkylphenyl. (heterocycle) C₁₋₃alkyl-, (heterocycle)C₁₋₃alkylthio-, (heterocycle)C₁₋₃alkyloxy-, (heterocycle)C ₁₋₃alkylamino-, (heterocycle)thio-, (heterocycle)oxy-, (heterocycle)amino-, C₁₋₃alkylthio-. cyano. thiol, C₁₋₃alkyl (optionally substituted by one or more fluorine atoms), -C₁₋₃alkylamino, -C₁₋₃alkylaminoalkyl, carboxamidoC₁₋₃alkyl, acetoxyC₁₋₃alkyl, or C₃₋₄cycloalkyl. The term (heterocycle) denotes a 5 or 6 membered saturated or unsaturated ring containing 1-5 heteroatoms selected from sulphur, oxygen or nitrogen. Examples of suitable heterocycles include morpholine, pyrrolidine, imidazoline. thiazoline, piperazine. pyrrole, thiophene, furan, oxazole, thiazole, imidazole, isoxazole, isthiazole, triazole, pyrazole, thiadiazole, tetrazole, pyridine, pyrimidine, pyridazine, pyrazine or triazine. Preferably R⁷ is hydrogen, hydroxy, amino, methylamino, dimethylamino, NHcyclopropyl, NHcyclobutyl, NHbenzyl, imidazol-2-ylthio-, thiadiazol-2-ylthio-, triazol-2-ylthio-, thiol, methyl, ethyl, cyclopropyl, methylthio, methoxy, hydroxyethyl, acetoxyethyl, imidazol-4-ylethyl, or imidazol-1-ylethoxy.

More preferably R⁷ is methyl, ethyl, hydrogen, hydroxy, hydroxyethyl, imidazol-4-ylethyl, or amino.

Suitably S is 1 or 2. Preferably S is 1 or 2.

Suitably B is a pyrrole. thiophene, furan, oxazole, thiazole. imidazole, isoxazole, isthiazole, triazole, pyrazole, thiadiazole, pyridine, pyrimidine, pyridazine, pyrazine. triazine. Preferably B is pyridine, oxazole, thiazole or imidazole

Suitably Z is a bond, -O-, -S-, -SO₂-, -CH₂-, -NH-, -Nalkyl-, -CH=CH-, -CF=CH-,-CH=CF-, -CF=CF-, -CH₂CH₂-, -CH=Calkyl-, -Calkyl=CH-, -CH=C(halogen)-,-C(halogen)=CH-, -NHCO-. -CONH-, -SO₂NH-, -NHSO₂-, or a group -R⁸CH₂- or -CH₂R⁸- where R⁸ is NH, Nalkyl, NCOalkyl, CO, O or S. Preferably Z is -CH=CH-, -CH₂CH₂- or -CH₂O-.

Suitably R² is hydrogen, NO₂, NH₂, N(C₁₋₆alkyl)₂, CO₂H, CH₂OH, halogen, CO₂C₁₋₆alkyl, C₁₋₈alkyl optionally interrupted by one or more oxygen, nitrogen or sulphur atoms and optionally substituted by CO₂H or R² is hydroxy, imidazol-1-ylCH₂-, phenyl optionally substituted by CH₂CO₂H or CONR⁹R¹⁰ where R⁹ and R¹⁰ are independently hydrogen. C₁₋₆alkyl optionally substituted by hydroxy or CO₂H and/or optionally interrupted by oxygen, nitrogen or sulphur. Preferably R² is hydrogen or CO₂H

Suitably R³ is hydrogen, C₁₋₈alkyl optionally interrupted by one or more oxygen, sulphur or nitrogen atoms and optionally substituted by CO₂H, or R³ is R¹¹PO(OH)₂ or R¹¹CO₂H where R¹¹ is OCH₂, (CH₂)p where p is 1 or 2, SCH₂, CONHCH₂; or R¹¹ is NHCH(R¹⁹) where R¹⁹ is hydrogen, C₁₋₆ alkyl optionally substituted by hydroxy and/or optionally interrupted by oxygen, nitrogen or sulphur; or R¹¹ is NR²⁰(CH₂)p where p is 1 or 2 and R²⁰ is hydrogen or C₁₋₆ alkyl; or R³ is R¹²tetrazol-5-yl where R¹² is a bond, (CH₂)p, OCH₂, SCH₂, CONH, CONHCH₂, CONHCH₂CONH, NHCH₂CONH, NHCH(R⁹); or R³ is COR¹³ where R¹³ is OH, N(CH₂CO₂H)₂, NHS(O)₂R²¹ where R²¹ is NH₂ or C₁₋₆alkyl optionally interrupted by oxygen or nitrogen or R¹³ is a group of formula (iv): 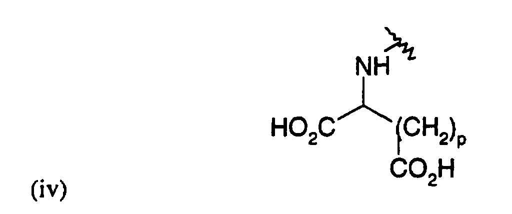 or R³ is NR¹⁴R¹⁵ where R¹⁴ and R¹⁵ are independently hydrogen, CH₂CO₂H, CHPh₂ or C(=S)CH₂CH₂CO₂H; or R³ is CH₂NR¹⁶CH₂CO₂H where R¹⁶ is hydrogen, C₁₋₆alkyl or CO₂CH₂Ph; or R³ is a group of formula (iii): 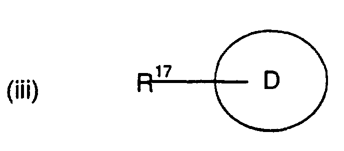 where D is a 4, 5 or 6 membered saturated ring containing a nitrogen atom and optionally substituted by hydroxy and substituted by CO₂H or CONH-het where het is tetrazol-5-yl or a thiazole or a thiadiazole ring substituted by CH₂CO₂H or D is a phenyl ring or a 5 membered aromatic heterocylic ring containing 1-3 heteroatoms selected from nitrogen, oxygen or sulphur optionally substituted by one or more groups selected from CF₃, CO₂H, CH₂OH, C₁₋₆alkyl optionally interrupted by one or more oxygen atoms, (CH₂)pCO₂H, C(CO₂H)=NOMe, tetrazol-5-yl, CH₂tetrazol-5-yl, CH₂CON(CH₂CO₂H)₂, or CH₂COR¹⁸ where R¹⁸ is a group of formula (iv) as defined above or a group of formula (v):

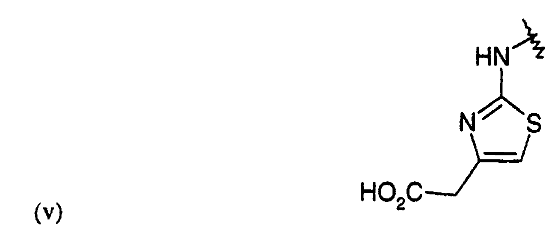

R¹⁷ is a bond, sulphur atom, CONH, CH₂, CH₂O, OCH₂, a group -NR²²CH(CO₂H)CH₂-group or a group CONR²²(CH₂)pCONR²³ or NR²²(CH₂)pCONR²³ where R²² and R²³ are independently hydrogen, C₁₋₆alkyl.

Preferably R³ is hydrogen, a group of formula (iii) where D is a 4-membered saturated ring containing a nitrogen atom and substituted by CO₂H, or D is a 5-membered aromatic heterocycle containing 2 or 3 heteroatoms selected from nitrogen and sulphur and optionally substituted by CH₂CO₂H or CF₃ and R¹⁷ is a bond, CONH, NHCH₂CONH or CH₂.

Suitably Q¹ and Q² each independently represent an O or S. Preferably Q¹ is S and Q² is O or S.

Particularly preferred compounds of the invention include those exemplified herein, both in free base form and as pharmaceutically acceptable salts.

In a further aspect the invention provides a process for the preparation of a compound of formula (I) which comprises:
(a) reacting a compound of formula (II): 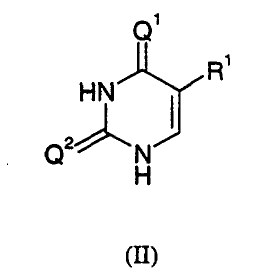 where Q¹ and Q² are as defined in formula (I) and R¹ is as defined in formula (I) or is a protected derivative thereof with a compound of formula (III): 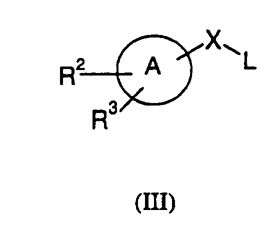 where R², R³ and A are as defined in formula (I) or are protected derivatives thereof, X is as defined in formula (I) and L is a leaving group, or
(b) reacting a compound of formula (IV): 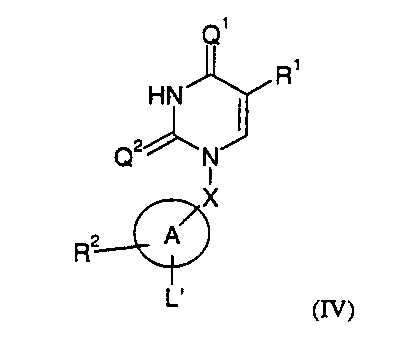 where Q¹, Q², R¹ and X are as defined in formula (I), R² and A are as defined in formula (I) or are protected derivatives thereof and L' is a leaving group with a compound of formula (V), (VI) or (VII):

   HNR²²-(CH₂)ₚ₋CONR²³-D (V)

   HNR²²-CH(CO₂H)-CH₂-D (VI)

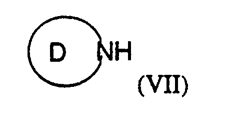

   where R²², R²³ and p are as defined in formula (I) and D is as defined in formula (I) or is a protected derivative thereof, or
(c) when R³ is a group -R¹¹-CO₂H and R¹¹ is SCH₂ or NR²⁰(CH₂)ₚ, reacting a compound of formula (iv) as defined above with a compound H-R¹¹-CO₂R²⁴ where R¹¹ is SCH₂ or NR²⁰(CH₂)ₚ and R²⁴ is hydrogen or an ester forming group;
and optionally thereafter (a), (b) or (c) in any order:
- removing any protecting groups
- converting the compound of formula (I) into a further compound of formula (I)
- forming a salt

Reaction of compounds of formulae (II) and (III) can be carried out in the presence of a suitable base, for example a metal carbonate such as potassium carbonate or cesium carbonate in a suitable polar solvent such as NMP, dimethylformamide or dimethylsulphoxide at ambient or elevated temperature, for example at about 80°C. Preferably L is halogen when X is a bond, in particular chloro or fluoro. Alternatively for compounds where X is CH₂ the compound of formula (II) can be silylated with a suitable silylating reagent such as a trialkylsilylchloride and/or 1,1,1,3,3,3-hexamethyldisilazane in a suitable solvent such as pyridine, toluene or 1,4-dioxane at a temperature of about 80°C to about 140°C followed by addition of the compound of formula (III) in a suitable solvent such as acetonitrile at elevated tempetature, for example at reflux. We prefer to silylate using bis(trimethylsilyl)trifluoroacetamide in refluxing 1,2-dichloroethane followed by treatment with the appropriate compound of formula (III) (where L is halogen, preferably bromide or chloride) in acetonitrile and 1,2-dichloroethane at reflux.

Compounds of formula (IV) can be reacted with compounds H-R¹¹-CO₂R²⁴ in the presence of a suitable base. When R¹¹ is SCH₂ the base is preferably NaH and when R¹¹ is NR²⁰(CH₂)ₚ the base is preferably an organic amine such as N,N-diisopropylethylamine. The group R²⁴ is hydrogen or a suitable ester forming group such as benzyl or C₁₋₆ alkyl. Compounds of formula (IV) can also be reacted with compounds of formulae (V), (VI) or (VII) using the procedure described above for when R¹¹ is NR²⁰(CH₂)ₚ.

It will be appreciated by those skilled in the art that in the process described above the functional groups of intermediate compounds may need to be protected by protecting groups.

Functional groups which it is desirable to protect include hydroxy, amino and carboxylic acid. Suitable protecting groups for hydroxy include organosilyl groups (e.g. *tert*-butyldimethylsilyl, *tert*-butyldiphenylsilyl or trimethylsilyl), benzyl and tetrahydropyranyl. Suitable protecting groups for amino include *tert*-butoxycarbonyl or benzyloxy carbonyl. Suitable protecting groups for carboxylic acid include C₁₋₆ alkyl or benzyl esters.

The protection and deprotection of functional groups may take place before or after a reaction step.

The use of protecting groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wutz, Wiley-Interscience (1991).

In particular compounds of formula (III) where R² and R³ contains a carboxylic acid group can be protected as esters, particularly as C₁₋₆alkyl esters. Basic hydrolysis of such esters can be performed using metal hydroxides or quaternary ammonium hydroxides such as sodium hydroxide in a solvent such as an aqueous alcohol, 1,4-dioxane, tetrahydrofuran or dimethylformamide at a temperature between 10°C and 100°C. When Q¹/Q² are oxygen, acidic hydrolysis may also be performed using mineral acid such as HCl or a strong organic acid such as trifluoroacetic acid in a suitable solvent such as 1,4-dioxane. We prefer basic hydrolysis using lithium hydroxide in aqueous tetrahydrofuran or aqueous methanol at ambient temperature.

Compounds of formula (I) can be converted into further compounds of formula (I) using standard procedures, for example using known alkylation, hydrolysis, esterification and amide formation chemistry, for example the use of a coupling reagent. Coupling reagents which may be used include 1,1'-carbonyldiimidazole and 1,3-dicyclohexylcarbodiimide in a suitable solvent such as dimethylformamide, dichloromethane, tetrahydrofuran or acetonitrile at about 0 °C to 30 °C. We prefer to use bromo-tris(pyrrolidino)-phosphonium hexafluorophosphate with a trialkylamine such as N,N-diisopropylaminopyridine in dimethylformamide at ambient temperature. Compounds of formula (I) where Q¹ is oxygen can be converted to a corresponding compound of formula (I) where Q¹ is sulphur using standard thiation conditions for conversion of uridine and thymidine nucleosides in their corresponding thio-nucleoside derivatives (see "Chemistry of Nucleosides and Nucleotides" edited by Leroy B. Townsend, Plenum Press volume 1). Thiation may be achieved using reagents such as diphosphorus pentasulphide or Lawesson's reagent in a solvent such as pyridine, 1,4-dioxane, toluene, xylene, or tetrahydrofuran at a temperature of about 50°C to about 130°C. We prefer to use Lawesson's reagent in 1,4-dioxane at about 100°C when selectivity can be achieved.

Thiation can also be achieved by displacement of a suitable leaving group in the 2- or 4-position of the uracil by hydrogen sulphide in a suitable solvent such as pyridine and triethylamine at ambient temperature. Suitable leaving groups include alkylthio or halogen, preferably alkylthio, more preferably methylthio.

Compounds of formula (II) where Q¹ and Q² are oxygen can be prepared by reaction of a compound of formula (VIII): 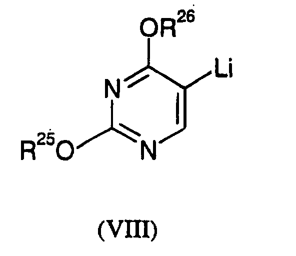 where R²⁵ and R²⁶ are independently C₁₋₆alkyl or benzyl with a compound of formula (IX): 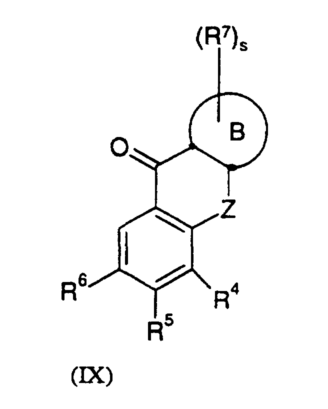 where Z, R⁴, R⁵, R⁶ and R⁷ groups are as defined in formula (I) followed by reduction of the resulting alcohol. Compounds of formula (VIII) are prepared by treating the corresponding halide with an alkyl lithium reagent (alkyl=n-Butyl, sec-Butyl, tert-Butyl) in solvents such as tetrahydrofuran or diethyl ether at low temperature e.g. -40°C to -78°C.

The resulting alcohol can then be reduced to compounds of type (II) by treatment with a trialkylsilane such as triethylsilane in a suitable solvent such as dichloromethane, chloroform, or 1,2-dichloroethane and an acid or Lewis acid such as trifluoroacetic acid or borontrifluoride diethyl ether complex. We preferred to perform the metal halogen exchange on 5-bromo-2,4-bis(1,1-dimethylethoxy)pyrimidine using n-butyl lithium at about -78°C in tetrahydrofuran.

When the lithio species is quenched with a compound of formula (IX) where Z is CH₂CH₂ the resulting alcohol can then be converted to a compound of formula (II) where Z is CH=CH by refluxing in a carboxylic acid solvent (D. Hellwinkel and T. Becker, Chem. Ber., 1989, 122, 1595). Preferred acids include acetic acid. In some cases further treatment with trifluoroacetic acid at reflux may be required for the dehydration to give the substituted uracil .
Compounds of formula (II) can also be prepared from uracil or 1-alkyluracil and the appropriately substituted alcohols of formula (XII) 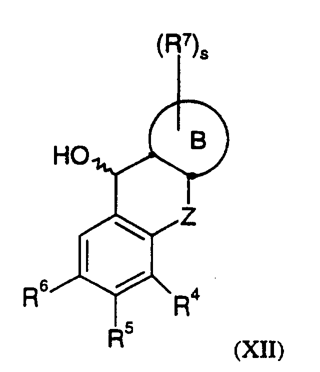 in which Z,, R⁴, R⁵, R⁶ and R⁷ groups are as defined in formula (I) by refluxing in a carboxylic acid solvent such as acetic acid or trifluoroacetic acid (J.O.C., 1974, 39, 587) or in a siutable solvent such as acetonitrile and a Lewis acid such as boron trifluoride etherate from ambient temperature to reflux.
Compounds of formula (XII) can be prepared from compounds of formula (IX) using a reducing agent such as sodium borohydride in a suitable solvent such as an alcohol, for example ethanol or diisobutylaluminium hydride in a suitable solvent such as tetrahydrofuran or diethyl ether or benzene or toluene from 0°C to 50 °C .

Compounds of type (IX) can be prepared from compounds of type (X) by treatment with polyphosphoric acid at high temperature (100°C - 200 °C) or by treatment of the corresponding acid chloride under Friedel-Crafts type conditions using a suitable Lewis acid such as the halide salts of Boron, Tin, Iron, Zinc or Aluminium. We preferred to use anhydrous AlCl₃ in a suitable solvent such as dichloromethane, carbon disulphide or 1,2-dichloroethane heated under reflux

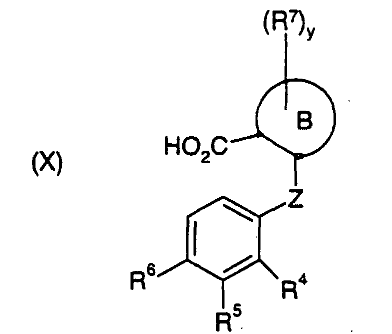

Alternatively when B is oxazole, thiazole or imidazole, (IX) can be prepared from the corresponding cycloheptanone (XI) in several steps using the methods of Galantay et al (J.Med.Chem., 1974, 17, 1316).

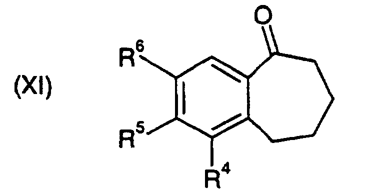

Salts of the compounds of formula (I) may be formed by reacting the free acid, or a salt thereof, with one or more equivalents of the appropriate base (for example ammonium hydroxide optionally substituted by C₁₋₆-alkyl or an alkali metal or alkaline earth metal hydroxide). The reaction may be carried out in a solvent or medium in which the salt is insoluble or in a solvent in which the salt is soluble, e.g. water, alcohol or acetone, which may be removed *in vacuo*, or by freeze drying. The reaction may also be ametathetical process or it may preferably be carried out on an ion exchange resin. The non-toxic pharmaceutically acceptable salts are preferred, although other salts may be useful, e.g. in isolating or purifying the product.

All novel intermediates form a further aspect of the invention.

The compounds of the invention have been submitted to the assay outlined below and have been found to be P2 7-TM G-protein receptor antagonists, particularly to the P2Y₂ receptor. Accordingly they are useful in therapy and are, in particular, indicated for use as anti-inflammatory agents. The invention therefore provides a pharmaceutical composition comprising a compound of formula I as defined herein or a pharmaceutically acceptable salt thereof in association with a pharmaceutically acceptable adjuvant, diluent or carrier.

The invention provides in a further aspect a method of treating an inflammatory condition which comprises administering to a patient in need of therapy, a therapeutically effective amount of a compound of the invention. If desired the compounds of the invention can be administered with other anti-inflammatory agents for example NSAIDS, cPLA₂ inhibitors COX-2 inhibitors or iNOS inhibitors.

According to the invention there is further provided use of the compounds of the invention in the manufacture of a medicament for use in the treatment of an inflammatory condition.

The compounds may be administered orally, topically e.g. to the lung and/or the airways, dermally, in the form of solutions, suspensions, HFA areosols and dry powder formulations, e.g. Turbuhaler® formulations or by parenteral administration in the form of sterile parenteral solutions or suspensions.

The invention further provides a pharmaceutical composition comprising a compound according to the present invention in association with a pharmaceutically acceptable excipient and/or adjuvant. Particularly preferred are compositions not containing material capable of causing an adverse, e.g. an allergic, reaction. For example a chelating or sequestering agent, an antioxidant, a tonicity adjusting agent, a pH modifying agent and/or a buffering agent are suitable additives.

The compounds of the invention may also be administered by means of a dry powder inhaler. The inhaler may be a single or a multi dose inhaler, and may be a breath actuated dry powder inhaler.

A pharmaceutical composition according to the present invention could optionally be prepared in freeze dried form using any lyophilisation techniques commonly used within the pharmaceutical area. Upon use but before administration, such pharmaceutical compositions are generally reconstituted in a pharmaceutically acceptable excipient. Preferably a solution of the pharmaceutical composition according to the invention obtained after reconstitution is an isotonic solution. Such a pharmaceutical composition according to the present invention when reconstituted is preferably administered by injection, for example intravenously, subcutaneously or intramuscularly.

The invention is illustrated by the following examples. In the examples the NMR spectra were measured on a Varian Unity Inova 300 or 400 MHz spectrometer and the MS spectra measured as follows: EI spectra were obtained on a VG 70-250S or Finnigan Mat Incos-XL spectrometer, ESI and APCI spectra were obtained on Finnigan Mat SSQ7000 or a Micromass Platform spectrometer. Where necessary, the reactions were performed under an inert atmosphere of either nitrogen or argon. Where necessary, preparative HPLC separations were generally performed using a Novapak® , Bondapak® , or Hypersil® column packed with BDSC-18 reverse phase silica gel. Chromatography was generally performed using Matrex Silica 60® (35-70 micron)or Prolabo Silica gel 60® (35-75 micron) suitable for flash silica gel chromatography. Reverse phase chromatography was generally performed using C18 sep-pak® (55-105 micron).

### Example 1

### (±)-5-[[5-(2-Ethyl-7-methyl-4H-benzo[5,6]cyclohepta[1,2-d]oxazol-4-yl)-3,4-dihydro-2,4-dioxo-1(2H)-pyrimidinyl)methyl)-2-furancarboxylic acid

### i) 5-(3-Methylphenyl)penta-2,4-dienoic acid

A mixture of potassium *tert*-butoxide (68g) and *tert*-butanol (130ml) was treated dropwise with a mixture of 3-methylbenzaldehyde (33.8g) and methyl crotonate (45ml). The temperature of the reaction mixture rose to 65°C during the addition and this was maintained for a further 3 hours. The reaction mixture was cooled to room temperature, poured into ice/water and washed with diethyl ether. The aqueous phase was acidified with concentrated HCl and extracted with diethyl ether. The organic phase was washed with water, dried (MgSO₄) and evaporated under reduced pressure. Purification was by trituration with isohexane and filtration. Yield 36.8g.
1H NMR: δ (CDCl₃) 8.06 (d of d,1H), 7.4-7.3 (m,3H), 7.15 (d,1H), 6.85 (m,2H), 5.74 (d,1H), 2.38 (s,3H).

### ii) 5-(3-Methylphenyl)pentanoic acid

A solution of the product from step (i) (36.8g) in ethanol (300ml) was hydrogenated over 10% palladium on carbon (1g) at 1 atmosphere pressure for 3 days. The catalyst was removed by filtration and the mother liquor was evaporated under reduced pressure. Yield 34.2g.
1H NMR: δ (DMSO) 12.05 (s,1H), 7.15 (t,1H), 6.98 (d,3H), 2.50 (m,2H), 2.25 (m,5H), 1.52 (m,4H).

### iii)6,7,8,9-Tetrahydro-2-methyl-5H-benzocyclohepten-5-one

A mixture of the product from step (ii) (14g) and polyphosphoric acid (150ml) was heated at 100°C for 16 hours. The reaction mixture was poured onto ice/water and extracted with diethyl ether. The organic phase was washed with dilute NaOH and saturated brine. The organic phase was dried (MgSO₄) and the solvent evaporated under reduced pressure. Purification was by chromatography eluting with 10% ethyl acetate in toluene. Yield 4.54g.
MS: APCI(+ve): 175 (M+1, 100%).

### iv) 8,9-Dihydro-2-methyl-5H-benzocycloheptene-5,6(7H)-dione, 6-oxime.

A solution of the product from step (iii) (4.5g) in diethyl ether (200ml) and 1M HCI in diethyl ether (32ml) was treated dropwise with isoamyl nitrite (4.5ml) at -15°C. The reaction mixture was stirred at 0°C for 72 hours and the solvent was evaporated under reduced pressure. Purification was by recrystallisation from isohexane/ethyl acetate. Yield 2.2g.
MS: APCI(+ve): 204 (M+1, 100%).

### v) 2-Ethyl-9,10-dihydro-7-methyl-4H-benzo[5,6]cyclohepta[1,2-d]oxazol-4-one

Propionyl chloride (4.7ml) was added dropwise to a mixture of propionic acid (25ml), propionic anhydride (2.5ml) and the product from step (iv) (2.2g) at 85°C. The reaction mixture was heated at this temperature for a further 1 hour then poured into ice/water/NaOH (sufficient to maintain pH>7) and extracted with diethyl ether. The organic phase was dried (MgSO₄) and the solvent was evaporated under reduced pressure. Purification was by chromatography eluting with 25% ethyl acetate in toluene. Yield 1.26g.
MS: APCI(+ve): 242 (M+1, 100%).

### vi) 5-Bromo-2,4-bis(1,1-dimethylethoxy)pyrimidine

To a solution of potassium *tert*-butoxide (67.5g) in tetrahydrofuran (500ml) was added 5-bromo-2,4-dichloropyrimidine (55g) (*J. Am. Chem. Soc*., 1934, **56**, 134) in tetrahydrofuran (100ml) dropwise. After 1.5 hours water (100ml) was added carefully and the mixture extracted with ethyl acetate. The combined organic solution was washed with water, dried (MgSO₄) and evaporated under reduced pressure. Purification was by chromatography eluting with isohexane containing 1% triethylamine. Yield 52.4g.
MS: GC-MS: 304/302 (M⁺)

### vii) (±)-5-(2-Ethyl-7-methyl-4H-benzo[5,6]cyclohepta[1,2-d]oxazol-4-yl)-2,4(1H,3H)-pyrimidinedione

To a solution of the product of step (vi) (1.74g) in dry tetrahydrofuran (20ml) at -78°C was added *n*-butyllithium (2.3ml of a 2.5M solution in hexanes) dropwise. After 0.5 hours a solution of the product from step (v) (1.26g) in tetrahydrofuran (10ml) was added. The reaction mixture was allowed to warm to room temperature and saturated brine was added. The mixture was extracted with ethyl acetate, the organic phase was dried (MgSO₄) and the solvent was evaporated under reduced pressure. The residue was dissolved in acetic acid (20ml) and the solution was heated at reflux for 64 hours. The solvent was evaporated under reduced pressure and the residue was then dissolved in trifluoroacetic acid (20ml) and the solution heated at reflux for 64 hours. The solvent was evaporated under reduced pressure. Purification was by trituration with diethyl ether and filtration. Yield 1.42g.
MS: APCI(+ve): 336 (M+1, 100%).

### viii) (±)-5-[[5-(2-Ethyl-7-methyl-4H-benzo[5,6]cyclohepta[1,2-d]oxazol-4-yl)-3,4-dihydro-2,4-dioxo-1(2H)-pyrimidinyl]methyl]-2-furancarboxylic acid, ethyl ester

To a slurry of the product of step (vii) (1.42g) in dry 1,2-dichloroethane (15ml) was added bis(trimethylsilyl)trifluoroacetamide (3.4ml). The mixture was heated at reflux for 2 hours until the mixture became a homogeneous solution. The solution was allowed to cool to room temperature and a solution of 5-[bromomethyl]-2-furancarboxylic acid ethyl ester (*J*. *Chem. Soc. Perkin Trans. I*, 1981, 1125, *Bull. Chem. Soc. Jpn*., 1987, **60**, 1807) (1.1g) in dry acetonitrile (15ml) was added. The solution was then heated at reflux for 14 hours. The reaction mixture was allowed to cool and methanol (15ml) was added. The solvents were evaporated under reduced pressure and the residue was triturated with diethyl ether. The resultant solid was collected by filtration and partitioned between saturated aqueous sodium bicarbonate and ethyl acetate. The organic phase was dried (MgSO₄) and the solvent was evaporated under reduced pressure. Purification was by chromatography eluting with ethyl acetate. Yield 0.26g.
MS: APCI(+ve): 488 (M+1, 100%).

### ix) (±)-5-[[5-(2-Ethyl-7-methyl-4H-benzo[5,6]cyclohepta[1,2-d]oxazol-4-yl)-3,4-dihydro-2,4-dioxo-1(2H)-pyrimidinyl]methyl]-2-furancarboxylic acid

A mixture of the product of step (viii) (0.26g) and lithium hydroxide monohydrate (0.063g) in methanol (4ml) and water (4ml) was stirred at room temperature for 1.5 hours. The solution was neutralised by the addition of 2M hydrochloric acid and the solvents were evaporated under reduced pressure. Purification was by preparative HPLC followed by lyophilisation. Yield 0.035g.
MS: APCI(-ve): 458 (M-1, 100%).
1H NMR: δ (DMSO) 7.37 (d,1H), 7.18 (d,2H), 7.03 (d,1H), 6.84 (s,1H), 6.76 (d,2H), 6.42 (d,1H), 5.19 (s,1H), 4.90 (d,1H), 4.77 (d,1H), 2.74 (q,2H), 2.26 (s,3H), 1.22 (t,3H).
MP: 168-169°C.

### Example 2

### (±)-2-[[5-(2-Ethyl-7-methyl-4H-benzo[5,6]cyclohepta[1,2-d]oxazol-4-yl)-3,4-dihydro-2-oxo-4-thioxo-1(2H)-pyrimidinyl]methyl]-4-oxazolecarboxylic acid

### i) (±)-2-[[5-(2-Ethyl-7-methyl-4H-benzo[5,6]cyclohepta[1,2-d]oxazol-4-yl)-3,4-dihydro-2,4-dioxo-1(2H)-pyrimidinyl]methyl]-4-oxazolecarboxylic acid, ethyl ester

The subtitle compound was prepared from the product from example 1 step (vii) (6.5g) and 2-[bromomethyl]-4-oxazolecarboxylic acid ethyl ester (*Coll. Czech Chem. Comm*., 1967, **32**, 2155) (1.69g) by the method of example 1 step (viii). Purification was by chromatography eluting with 60-66% ethyl acetate in toluene. Yield 1.26g.
MS: APCI(+ve): 489 (M+1, 100%).

### ii) (±)-2-[[5-(2-Ethyl-7-methyl-4H-benzo[5,6]cyclohepta[1,2-d]oxazol-4-yl)-3,4-dihydro-2-oxo-4-thioxo-1(2H)-pyrimidinyl]methyl]-4-oxazolecarboxylic acid, ethyl ester

A mixture of the product of step (i) (1.26g) and Lawesson's reagent (1.56g) in 1,4-dioxane (30ml) was heated at reflux for 16 hours. The solvent was evaporated under reduced pressure. The residue was partitioned between ethyl acetate and saturated brine. The organic phase was collected, dried (MgSO₄) and solvent evaporated under reduced pressure. Purification was by chromatography eluting with 50% ethyl acetate in toluene. Yield 0.55g.
MS: APCI(+ve): 505 (M+1, 100%).

### iii) (±)-2-[[5-(2-Ethyl-7-methyl-4H-benzo[5,6]cyclohepta[1,2-d]oxazol-4-yl)-3,4-dihydro-2-oxo-4-thioxo-1(2H)-pyrimidinyl]methyl]-4-oxazolecarboxylic acid.

A mixture of the product of step (ii) (0.55g) and lithium hydroxide monohydrate (0.46g) in methanol (10ml) and water (10ml) was stirred at room temperature for 3 hours. The solution was concentrated under reduced pressure to 10ml and partitioned between 2M hydrochloric acid and ethyl acetate. The organic phase was dried (MgSO₄) and the solvent evaporated under reduced pressure. Purification was by reverse phase chromatography eluting with 50% methanol in 0.1% aqueous ammonium acetate followed by lyophilisation. Yield 0.060g.
MS: APCI(+ve): 477 (M+1, 100%).
1H NMR: δ (DMSO) 8.61 (s,1H), 7.63 (d,1H), 7.28 (s,1H), 7.20 (m,2H), 6.86 (d,1H), 6.76 (d,1H), 5.92 (s,1H), 5.11 (d,1H), 5.01 (d,1H), 2.76 (q,2H), 2.27 (s,3H), 1.22 (t,3H).

### Example 3

### (±)-2-[2-[[4-[5-(7-Ethyl-2-methyl-4H-benzo[5,6]cyclohepta[1,2-d]oxazol-4-yl)-3,4-dihydro-2,4-dioxo-1(2H)-pyrimidinyl]pyrimidin-2-yl]amino]acetylamino)-4-thiazoleacetic acid

### i) 3-Ethylbenzaldehyde

A solution of 3-bromo-ethylbenzene (25g) in dry tetrahydrofuran (350ml) at -70°C was treated dropwise with *n*-butyllithium (59ml of a 2.5M solution in hexanes) and stirred for 1 hour. *N,N*-Dimethylformamide (30ml) was added and the solution was stirred at -70°C for 1 hour and allowed to warm to room temperature. The reaction mixture was partitioned between saturated ammonium chloride solution and ethyl acetate. The organic phase was washed with 1N hydrochloric acid, dried (MgSO₄) and evaporated. Yield 18.55g.
1H NMR: δ (DMSO) 10.00(s,1H), 7.71(m,2H), 7.47(m,2H), 2.74(q,2H), 1.28(t,3H)

### ii) 5-(3-Ethylphenyl)penta-2,4-dienoic acid

The subtitle compound was prepared from the product of step (i) (18.55g) according to the method of example 5 steps (i) and (ii). Yield 25g.
MS: APCI(-ve): 201 (M-1,100%)

### iii) 5-(3-Ethylphenyl)pentanoic acid

The subtitle compound was prepared from the product of step (ii) (25g) according to the method of example 1 step (ii). Yield 21.70g.
MS: APCI(-ve): 205 (M-1,100%)

### iv) 2-Ethyl-6,7,8,9-tetrahydro-5H-benzocyclohepten-5-one

The subtitle compound was prepared from the product of step (iii) (19.81g) according to the method of example 1 step (iii). Yield 12.12g.
1H NMR: δ (CDCl₃) 7.68(d,1H), 7.13(dd,1H), 7.02(d,1H), 2.92(t,2H), 2.68(m,4H), 1.84(m,4H), 1.24(t,3H)

### v) 2-Ethyl-8,9-dihydro-5H-benzocycloheptene-5,6(7H)-dione, 6-oxime

The subtitle compound was prepared from the product of step (iv) (11.5g) according to the method of example 1 step (iv). Yield 9.8g.
1H NMR: δ (CDCl₃) 8.80(br s,1H), 7.80(d,1H), 7.20(dd,1H), 7.04(d,1H), 2.86(t,2H), 2.70(m,5H), 2.04(m,2H), 1.27(t,3H)

### vi) 7-Ethyl-9,10-dihydro-2-methyl-4H-benzo[5,6]cyclohepta[1,2-d]oxazol-4-one

The subtitle compound was prepared from the product of step (v) (9.20g) according to the method of example 1 step (v) using acetic anhydride (4.9ml), acetyl chloride (7.2ml) and acetic acid (47ml) (instead of propionic anhydride, propionyl chloride, and propionic acid). Yield 3.49g.
1H NMR: δ (CDCl₃) 7,93(d,1H), 7.21(dd,1H), 7.07(d,1H), 3.18(m,2H), 3.11(m,2H), 2.49(s,3H), 2.70(q,2H), 1.26(t,3H)

### vii) (±)-5-(7-Ethyl-2-methyl-4H-benzo[5,6]cyclohepta[1,2-d]oxazol-4-yl)-2,4(1H,3H)-pyrimidinedione

The subtitle compound was prepared from the product of step (vi) (3.38g) according to the method of example 1 step (vii). Yield 4.52g.
MS: APCI(-ve): 334 (M-1,100%)

### viii) (±)-1-(2-Chloropyrimidin-4-yl)-5-(7-ethyl-2-methyl-4H-benzo[5,6]cyclohepta[1,2-d]oxazol-4-yl)-2,4(1H,3H)-pyrimidinedione

A solution of the product from step (vii) (4.50g), cesium carbonate (4.37g) and 2,4-dichloropyrimidine (3.00g) in dry *N*,*N*-dimethylformamide (30ml) was stirred for 25 hours. The reaction mixture was partitioned between ethyl acetate and water and the organic layer dried (MgSO₄). Evaporation of the solvent gave an oil which was purified by column chromatography eluting with 50-100% ethyl acetate in 40-60 petroleum ether. Yield 0.65g.
MS: APCI(-ve): 446 (M-1,100%)

### ix) 2-[(2-Aminoacetyl)amino]-4-thiazoleacetic acid ethyl ester

A mixture of *N*-(tert-butoxycarbonyl)glycine (3.1g), 2-amino-4-thiazoleacetic acid ethyl ester (3g) and bromo-tris(pyrrolidino)-phosphonium hexafluorophosphate (7.54g) in *N,N*-dimethylformamide (20ml) was treated with *N,N*-diisopropylethylamine (6ml). After 25 minutes 4-(dimethylamino)pyridine (1.96g) was added. After 24 hours the mixture was partitioned between ethyl acetate and brine. The organic phase was washed with saturated aqueous sodium bicarbonate, brine, 1N HCl and brine, dried (MgSO₄) and evaporated. The residue was purified by chromatography eluting with 50% ethyl acetate in isohexane. The product (0.77g) was dissolved in dichloromethane (25ml) and trifluoroacetic acid (10ml). After 2 hours the mixture was evaporated. The residue was partitioned between ethyl acetate and sodium bicarbonate solution. The aqueous phase was repeatedly extracted with ethyl acetate. The combined extracts were dried (MgSO₄) and evaporated. Yield 0.41g.
MS: APCI(+ve): 244 (M+1)

### x) (±)-2-[2-[[4-[5-(7-Ethyl-2-methyl-4H-benzo[5,6]cyclohept[1,2-d]oxazol-4-yl)-3,4-dihydro-2,4-dioxo-1(2H)-pyrimidinyl]pyrimidin-2-yl]amino]acetylamino]-4-thiazoleacetic acid, ethyl ester

A mixture of the products from steps (viii) (0.65g) and (ix) (1.025g) and *N,N*-diisopropylethylamine (1.26ml) in *N*-methylpyrrolidin-2-one (10ml) was stirred at 90°C for 24 hours. A further 0.342g of product from step (ix) and *N*,*N*-diisopropylethylamine (0.5ml) was added and heating continued for a further 24 hours. The solution was partitioned between 1N hydrochloric acid and ethyl acetate. The organic layer was washed with water, dried (MgSO₄) and evaporated to give a brown oil which was purified by chromatography eluting with ethyl acetate. Yield 0.61g.
1H NMR: δ (DMSO) 12.04(br s,1H), 11.34(s,1H), 8.30(d,1H), 7.83(s,1H), 7.42(d,1H), 7.30(s,1H), 7.19(m,3H), 6.85(m,3H), 5.34(s,1H), 4.24(d,1H), 4.06(q,2H), 3.68(s,2H), 2,58(q,2H), 2,35(s,3H), 1.16(m,6H)

### xi) (±)-2-[2-[[4-[5-(7-Ethyl-2-methyl-4H-benzo[5,6]cyclohepta[1,2-d]oxazol-4-yl)-3,4-dihydro-2,4-dioxo-1(2H)-pyrimidinyl]pyrimidin-2-yl]amino]acetylamino]-4-thiazoleacetic acid

The title compound was prepared from the product of step (x) (0.6g) according to the method of example 1 step (ix). Purification was by reverse phase chromatography eluting with 50% methanol in 0.1% aqueous ammonium acetate. Yield 0.04g.
MS: APCI(-ve): 625 (M-1,100%)
1H NMR: δ (DMSO) 12.02(br s,1H), 11.34(s,1H), 8.31(d,1H), 7.83(s,1H), 7.41(d,1H), 7.30(s,1H), 7.18(m,3H), 6.85(m,3H), 5.34(s,1H), 4.24(d,1H), 3.60(s,2H), 2.57(q,2H), 2,35(s,3H), 1.15(t,3H)
MP: 205-210°C

### Example 4

### (±)-5-[[3,4-Dihydro-5-(2-methyl-4H-benzo[5,6]cyclohepta[1,2-d]oxazol-4-yl)-2-oxo-4-thioxo-1 (2H)-pyrimidinyl]methyl]-2-furancarboxylic acid

### i) (±)-5-(2-Methyl-4H-benzo[5,6]cyclohepta[1,2-d]oxazol-4-yl)-2,4(1H,3H)-pyrimidinedione

The subtitle compound was prepared from 9,10-dihydro-2-methyl-4*H*-benzo[5,6]cyclohepta[1,2-*d*]oxazol-4-one (2.98g) (J. Med. Chem., 1974, 17, 1316) by the method of example 1 step (vii). Yield 4.0g. Used directly in the next step.

### ii) (±)-5-[[3,4-Dihydro-5-(2-methyl-4H-benzo[5,6]cyclohepta[1,2-d]oxazol-4-yl)-2,4-dioxo-1(2H)-pyrimidinyl]methyl]-2-furancarboxylic acid, ethyl ester

The subtitle compound was prepared from the product of step (i) (2.0g) by the method of example 1 step (viii). Yield 0.66g.
1H NMR: δ (CDCl₃) 8.21 (s,1H), 7.57 (d,1H), 7.40 (t,1H), 7.33 (d,1H), 7.29 (d,1H), 7.10 (d,1H), 6.83 (d,1H), 6.72 (d,1H), 6.70 (s,1H), 6.41 (d,1H), 5.46 (s,1H), 4.77 (q,2H), 4.20 (q,2H), 2.48 (s,3H), 1.43 (t,3H).

### iii) (±)-5-[[3,4-Dihydro-5-(2-methyl-4H-benzo[5,6]cyclohepta[1,2-d]oxazol-4-yl)-2-oxo-4-thioxo-1(2H)-pyrimidinyl]methyl]-2-furancarboxylic acid, ethyl ester

The subtitle compound was prepared from the product of step (ii) (0.66g) by the method of example 2 step (ii). Yield 0.13g.
1H NMR: δ (DMSO) 7.70 (d, 1H), 7.38 (m,2H), 7.30 (m,2H), 7.18 (s, 1H), 6.82 (d, 1H), 6.78 (d, 1H), 6.63 (d, 1H), 5.92 (s,1H), 4.97 (q, 2H), 4.32 (q,2H), 2.41 (s,3H), 1.31 (t,3H).

### iv) (±)-5-[[3,4-Dihydro-5-(2-methyl-4H-benzo[5,6]cyclohepta[1,2-d]oxazol-4-yl)-2-oxo-4-thioxo-1(2H)-pyrimidinyl]methyl]-2-furancarboxylic acid

A mixture of the product from step (iii) (0.13g) and lithium hydroxide monohydrate (0.042g) in methanol (20ml) and water (10ml) was stirred at room temperature for 72 hours and heated at reflux for 1 hour. The solution was concentrated under reduced pressure and the residue partitioned between 2M hydrochloric acid and ethyl acetate. The organic phase was dried (MgSO₄) and the solvent was evaporated under reduced pressure. The residue was triturated with ethyl acetate and the resultant solid collected by filtration. Yield 0.08g. MS: APCI (+ve) 448 (M+1,100%)
1H NMR: δ (DMSO) 13.23 (br s, 1H), 12.82 (s, 1H), 7.71 (d, 1H), 7.36 (m,2H), 7.29-7.18 (m,3H), 6.86 (d,1H), 6.76 (d, 1H), 6.60 (d, 1H), 5.92 (s, 1H), 4.95 (q,2H), 2.41 (s,3H).
MP: >230°C

### Example 5

### (±)-N-[4-[3,4-Dihydro-5-(7-hydroxy-2-methyl-4H-henzo[5,6]cyclohepta[1,2-d]oxazol-4-yl)-2,4-dioxo-1(2H)-pyrimidinyl]pyrimidin-2-yl]-3-azetidinecarboxylic acid

### i) 5-(3-Methoxyphenyl)penta-2,4-dienoic acid, methyl ester

A mixture of potassium *tert*-butoxide (52g) and *tert*-butanol (170ml) was treated dropwise with a mixture of 3-methoxybenzaldehyde (30g) and methyl crotonate (35ml). The temperature of the reaction mixture rose to 80°C during the addition and was then maintained for a further 3 hours at 65°C. The reaction mixture was cooled to room temperature, poured into ice/water and washed with diethyl ether. The aqueous phase was acidified with concentrated HCl and extracted with ethyl acetate. The organic phase was washed with brine, dried (MgSO₄) and evaporated under reduced pressure to yield an orange oil. Yield 53.2g. Used directly in the next step.

### ii) 5-(3-Methoxyphenyl)penta-2,4-dienoic acid

A mixture of the product from step (i) (53.2g) and lithium hydroxide monohydrate (52g) in methanol (250ml) and water (100ml) was stirred at room temperature for 24 hours and partitioned between ethyl acetate and 2M HCI. The organic phase was washed with brine, dried (MgSO₄), and evaporated under reduced pressure to afford an orange oil.
Yield 55.0g.
MS: APCI(-ve): 203 (M-1,100%).

### iii) 5-(3-Methoxyphenyl)pentanoic acid

The subtitle compound was prepared from the product of step (ii) (55g) according to the method of example 1 step (ii). Yield 50.0g.
MS: APCI(-ve): 207 (M-1, 100%).

### iv) 6,7,8,9-Tetrahydro-2-methoxy-5H-benzocyclohepten-5-one

The subtitle compound was prepared from the product of step (iii) (50.0g) according to the method of example 1 step (iii). Yield 28g.
MS: APCI(+ve): 191 (M+1,100%).

### v) 8,9-Dihydro-2-methoxy-5H-benzocycloheptene-5,6(7H)-dione, 6-oxime

The subtitle compound was prepared from the product of step (iv) (28.0g) according to the method of example 1 step (iv). Yield 23.9g.
MS: APCI(+ve): 220 (M+1,100%).

### vi) 9,10-Dihydro-7-methoxy-2-methyl-4H-benzo[5,6]cyclohepta[1,2-d]oxazol-4-one

The subtitle compound was prepared from the product of step (v) (23.9g) according to the method of example 3 step (vi). Yield 3.8g.
MS: APCI(+ve): 244 (M+1)

### vii) (±)-5-(7-Methoxy-2-methyl-4H-benzo[5,6]cyclohepta[1,2-d]oxazol-4-yl)-2,4(1H,3H)-pyrimidinedione

The subtitle compound was prepared from the product of step (vi) (3.0g) according to the method of example 1 step (vii). Yield 3.8g. Used directly in the next step.

### viii) (±)-5-[7-[(1,1-Dimethylethyl)dimethylsiloxy]-2-methyl-4H-benzo[5,6]cyclohepta[1,2-d]oxazol-4-yl]-2,4(1H,3H)-pyrimidinedione

To a suspension of the product from step (vii) (1.5g) in dichloromethane (20ml) was added a solution of boron tribromide (1M in dichloromethane) (6ml). The mixture was stirred at room temperature for 2 hours. A further 10ml of boron tribromide solution was added and the mixture was stirred for 2 hours before quenching with water. The pH was adjusted to 7 with sodium bicarbonate solution and the aqueous solution extracted with ethyl acetate. The organic phase was dried (MgSO₄) and evaporated under reduced pressure to give a brown solid. The solid was dissolved in *N,N*-dimethylformamide (20ml) and treated with imidazole (0.68g) followed by *tert*-butyldimethylsilyl chloride (0.68g). The mixture was stirred overnight then partitioned between water and ethyl acetate. The organic phase was dried (MgSO₄) and evaporated under reduced pressure. Purification was by precipitation with methanol/dichloromethane/isohexane. Yield 0.36g.
MS: APCI(+ve): 438 (M+1,100%).

### ix) (±)-1-(2-Chloropyrimidin-4-yl)-5-[7-[(1,1-dimethylethyl)dimethylsiloxy]-2-methyl-4H-benzo[5,6]cyclohepta[1,2-d]oxazol-4-yl]-2,4(1H,3H)-pyrimidinedione

A mixture of the product from step (viii) (0.35g), 2,4-dichloropyrimidine (0.18g) and cesium carbonate (0.26g) in *N,N*-dimethylformamide (10ml) was stirred at room temperature for 4 hours. The mixture was partitioned between water and ethyl acetate, the organic phase washed with water, dried (MgSO₄) and evaporated under reduced pressure. Purification was by chromatography eluting with 50-80% ethyl acetate in isohexane. Yield 0.367g.
MS: APCI(+ve): 550 (M+1, 100%).

### x) (±)-N-[4-[3,4-Dihydro-5-(7-hydroxy-2-methyl-4H-benzo[5,6]cyclohepta[1,2-d]oxazol-4-yl)-2,4-dioxo-1(2H)-pyrimidinyl)pyrimidin-2-yl]-3-azetidinecarboxylic acid

The product from step (ix) was treated with 3-azetidinecarboxylic acid (0.096g) and *N,N*-diisopropylethylamine (0.445ml) in *N,N*-dimethylformamide (10ml) at 85°C for 4 hours. The mixture was partitioned between 1N HCl and ethyl acetate. The aqueous layer was adjusted to pH 6 and extracted with ethyl acetate. The organic phase was dried (MgSO₄) and evaporated under reduced pressure to give a solid. Purification was by reverse phase chromatography eluting with 50% methanol in 0.1% aqueous ammonium acetate solution. Yield 0.059g.
MS: APCI(+ve): 501 (M+1)
1H NMR: δ (DMSO) 12.77(s,1H), 11.61(s,1H), 9.51(s,1H), 8.36(d,1H), 7.87(s,1H), 7.33(d,1H), 7.27(d,1H), 6.85-6.77(m,4H), 5.26(s,1H), 4.24(t,2H), 4.11-4.07(m,2H), 3.63-3.57(m,1 H), 2.41(s,3H).
MP:285°C

### Example 6

### (±)-2-[[5-(2-Ethyl-7-methyl-4H-benzo[5,6]cyclohepta[1,2-d]oxazol-4-yl)-3,4-dihydro-2-oxo-4-thioxo-1(2H)-pyrimidinyl]methyl]-N-[5-(trifluoromethyl)-1H-1,2,4-triazol-3-yl]-4-oxazolecarboxamide

A solution of the product of example 2 step (iii) (0.34g), 4-(dimethylamino)pyridine (0.34g), 3-amino-5-trifluoromethyl-1,2,4-triazole (J. Gen. Chem. USSR (Engl. Transl.) 1983, vol53, part 7, p1514) (0.42g) and *N,N*-diisopropylethylamine (1.46ml) in *N,N*-dimethylformamide (6ml) was treated with bromo-tris(pyrrolidino)phosphonium hexafluorophosphate (1.3g). After 2 hours the solution was quenched with aqueous tartaric acid. The solution was extracted with ethyl acetate. The combined extracts were washed with brine, dried (MgSO₄) and evaporated under reduced pressure. Purification was by chromatography eluting with 30% ethyl acetate in toluene. The product was triturated with ethyl acetate/ isohexane and the solid collected. Yield 0.12g.
MS: APCI(+ve): 611 (M+1, 100%)
1HNMR: δ (DMSO) 14.48(br s, 1H), 12.87(br s, 1H), 12.18(br s, 1H), 7.64(d, 1H), 7.30(br s, 1H), 7.22(m, 2H), 6.87(d, 1H), 6.77(d, 1H), 6.21(s, 1H), 5.21(d, 1H), 5.10(d, 1H), 2.74(q, 2H), 1.22(t, 3H).
MP: 188-190°C

### Example 7

### (±)-3-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]-5-[imidazol-1-ylmethyl]benzoic acid

### i) 3,5-(Dichloromethyl)benzoic acid, ethyl ester

Ethyl 3,5-dimethylbenzoate (15g) was heated at reflux with *N*-chlorosuccinimide (25g) in dry benzene (140ml) in the presence a catalytic amount (∼5mg) of benzoyl peroxide whilst being irradiated with a 500 watt halogen lamp. After 6 hours the cooled reaction mixture was partitioned with saturated brine. The organic layer was collected, dried (MgSO₄) and the solvent evaporated under reduced pressure. The crude residue was purified by chromatography eluting with 10% ethyl acetate in isohexane to give the subtitle product as a colourless solid. Yield: 3.7g.
1H NMR: δ (CDCl₃) 8.05(m, 2H), 7.62(s, 1H), 4.63(s, 4H), 4.20(q, 2H), 1.40(t, 3H).

### ii) 3-Chloromethyl-5-(imidazol-1-ylmethyl)benzoic acid, ethyl ester hydrochloride

The product of step (i) (3.7g) was dissolved in dry DMF (16ml) at 0°C and treated with a preformed mixture of imidazole (1g) and 60% sodium hydride (0.59g) in DMF (16ml) by syringe over 10 minutes. The reaction mixture was stirred for 20 minutes and carefully quenched with brine (20ml). The product was extracted into diethyl ether. The organic phase was dried (MgSO₄) and treated with a 1.0M HCl solution in diethyl ether (20ml) producing a white precipitate. The solvents/reagents were evaporated under reduced pressure and the residue azeotroped with diethyl ether twice. The residue was triturated with diethyl ether and filtered to leave the crude product as a white solid. Yield 1.92g.
MS: APCI(+ve): 279 (M+1) (100%), 315 (M+HCl) (100%)

### iii) (±)-3-[[5-(7-Ethyl-2-methyl-4H-benzo[5,6]cyclohepta[1,2-d]oxazol-4-yl)-3,4-dihydro-2,4-dioxo-1(2H)-pyrimidinyl]methyl]-5-[imidazol-1-ylmethyl]benzoic acid, ethyl ester

A solution of the product of example 3 step (vii) (2.5g) in dry dimethylsulphoxide (30ml) was treated with cesium carbonate (10g) with stirring. After 20 min a solution of the product of step (ii) (1.5g) in dimethylsulphoxide (10ml) was added dropwise over 2 min. After 1.5 hours the reaction mixture was quenched with saturated brine and the product extracted into ethyl acetate. The organic phase was dried (MgSO₄) and the solvent was evaporated under reduced pressure to leave a brown gum. Column chromatography eluting with 10% methanol in ethyl acetate containing 1% triethylamine gave the product as a glass. Yield: 0.92g.
MS: APCI(+ve): 578(M+1) (100%)

### iv) (±)-3-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]-5-[imidazol-1-ylmethyl]benzoic acid, ethyl ester

The subtitle compound was prepared from the product of step (iii) (0.92g) according to the method of example 2 step (ii) as a yellow foam. Yield: 0.45g.
MS: APCI(+ve): 594(M+1) (100%)

### v) (±)-3-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]-5-[imidazol-1-ylmethyl]benzoic acid

The title compound was prepared from the product of step (iv) (0.45g) according to the method of example 1 step (ix) as a yellow solid. Yield 0.32g.
A small portion of this material (0.09g) was subjected to purification by reverse phase chromatography eluting with 50% methanol in 1% aqueous ammonium acetate. Yield 0.03g.
MS: APCI(+ve): 566(M+1) (100%)
1H NMR: δ (DMSO) 7.99(2xs, 3H), 7.6(d, 1H), 7.40(s, 1H), 7.20(m, 4H), 6.95(s,1H), 6.62(q, 2H), 5.86(s, 1H), 5.33(s, 2H), 4.95(q, 2H), 2.5(q, 2H), 2.40(s, 3H), 1.10(t, 3H). MP: 204°C dec.

### Example 8

### (±)-2-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]-4-oxazolecarboxylic acid

### (i) (±)-2-[[5-(7-Ethyl-2-methyl-4H-benzo[5,6]cyclohepta[1,2-d]oxazol-4-yl)-3,4-dihydro-2,4-dioxo-1(2H)-pyrimidinyl]methyl]-4-oxazolecarboxylic acid, ethyl ester

The subtitle compound was prepared from the product of example 3 step (vii) (2.2g) and 2-bromomethyl-4-oxazolecarboxylic acid ethyl ester (0.52g) using the method of example 1 step (viii). Purification was by chromatography eluting with ethyl acetate. Yield 0.67g. MS: APCI(+ve): 489 (M+1, 100%)

### (ii) (±)-2-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]-4-oxazolecarboxylic acid, ethyl ester

The subtitle compound was prepared from the product of step (i) (0.67g) using the method of example 2 step (ii). Purification was by chromatography eluting with 50% ethyl acetate in toluene. Yield 0.51g.
MS: APCI(+ve): 505 (M+1, 100%)

### (iii) (±)-2-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]-4-oxazolecarboxylic acid

The title compound was prepared from the product of step (ii) (0.45g) using the method of example 1 step (ix). Yield 0.4g.
MS: APCI(+ve): 477 (M+1, 100%)
1 H NMR: δ(DMSO) 12.87(s, 1H), 8.8(s, 1H), 7.65(d, 1H), 7.3(s, 1H), 7.2-7.25(m, 2H), 6.84(d, 1H), 6.74(d, 1H), 5.93(s, 1H), 5.08(d, 1H), 5.04(d, 1H), 2.58(q, 2H), 2.4(s, 3H), 1.15(t, 3H).
MP: 210°C

### Example 9

### (±)-3-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]benzoic acid

### i) (±)-3-[[5-(7-Ethyl-2-methyl-4H-benzo[5,6]cyclohepta[1,2-d]oxazol-4-yl)-3,4-dihydro-2,4-dioxo-1(2H)-pyrimidinyl]methyl]benzoic acid, methyl ester

The subtitle compound was prepared from the product of example 3 step (vii) (2.g) and 3-bromomethyl benzoic acid methyl ester (0.5g) using the method of example 1 step (viii). Purification was by chromatography eluting with 60% ethyl acetate in toluene. Yield 0.58g.
MS: APCI(+ve) 484 (M+1, 100%)

### ii) (±)-3-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]benzoic acid, methyl ester

The subtitle compound was prepared from the product of step (i) (0.56g) using the method of example 2 step (ii). Purification was by chromatography eluting with 30% ethyl acetate in toluene, Yield 0.5g.
MS: APCI(+ve): 500(M+1, 100%)

### iii) (±)-3-[[5-(7-Ethyl-2-methyl-4H-benzo[5,6]cyclohepta[1,2-d]oxazol-4-yl)-3,4-dihydro-2-oxo-4-thioxo-1(2H)-pyrimidinyl]methyl]benzoic acid

The title compound was prepared from the product of step (ii) (0.5g) using the method of example 1 step (ix). Yield 0.43g.
MS: APCI(+ve): 486(M+1, 100%)
1H NMR: δ(DMSO) 13.05(br s, 1H), 12.75(s, 1H), 7.9-8.0(m, 1H), 7.84(s, 1H), 7.59(d, 1H), 7.5-7.6(m, 2H), 7.27(s, 1H), 7.1-7.2(m, 2H), 6.75(d, 1H), 6.68(d, 1H), 5.86(s, 1H), 4.99(d, 1H), 4.85(d, 1H), 2.55(q, 2H), 2.4(s, 3H), 1.14(t, 3H).
MP: 168-180 °C (decomp)

### Example 10

### (±)-3-[[5-(7-Ethyl-2-methyl-4H-benzo[5,6]cyclohepta[1,2-d]oxazol-4-yl)-3,4-dihydro-2-oxo-4-thioxo-1(2H)pyrimidinyl]methyl]-N-[5-(trifluoromethyl)-1H-1,2,4-triazol-3-yl]benzenecarboxamide

The title compound was prepared from the product of example 9 step (iii) (0.35g) using the method of example 6. Yield 0.035g.
MS: APCI(+ve): 620(M+1, 100%)
1H NMR: δ (DMSO) 12.75(s, 1H), 8.20(br s, 2H), 8.03(dt, 1H), 7.8(s, 1H), 7.7-7.5(m, 3H), 7.23(s, 1H), 7.17(dd, 1H), 7.09(d, 1H), 6.64(s, 2H), 5.87(s, 1H), 2.5(q, 2H), 2.35(s, 3H), 1.15(t, 3H).
MP: 142-145°C

### Example 11

### (±)-3-[[5-(7-Ethyl-9,10-dihydro-2-methyl-4H-benzo[5,6]cyclohepta[1,2-d]oxazol-4-yl)-3,4-dihydro-2-oxo-4-thioxo-1(2H)-pyrimidinyl]methyl]benzoic acid

### i) (±)-4-(2,4-Bis(1,1-dimethylethoxy)pyrimidin-5-yl)-7-ethyl-2-methyl-9,10-dihydro-4H-benzo[5,6]cyclohepta[1,2-d]oxazol-4-ol

A stirred solution of the product from example 1 step (vi) (3.60g) in tetrahydrofuran (30ml) at -70°C was treated dropwise with *n*-butyllithium (4.75ml of a 2.5M solution in hexanes) and stirred for 30 minutes. A solution of the product from example 3 step (vi) (2.60g) in tetrahydrofuran (30ml) was added dropwise and the reaction stirred at room temperature for 3 hours. The reaction was partitioned between brine and ethyl acetate and the organics dried (MgSO₄) and evaporation under reduced pressure gave a brown oil. Purification was by column chromatography eluting with 50% ethyl acetate in isohexane. Yield 4.06g.
1H NMR: δ (CDCl₃) 7.87(d,1H), 7.78(s,1H), 7.12(dd,1H), 6.98(s,1H), 4.71(s,1H), 2.92(m,2H), 2.67(m,4H), 2.39(s,3H), 1.56(s,9H), 1.44(s,9H), 1.23(m,3H)

### ii) (±)-5-(7-Ethyl-9,10-dihydro-2-methyl-4H-benzo[5,6]cyclohepta[1,2-d]oxazol-4-yl)-2,4(1H,3H)-pyrimidinedione

A stirred solution of the product from step (i) (0.60g) in dichloromethane (15ml) was treated with triethylsilane (0.225ml) and cooled to 0°C. Boron trifluoride etherate (0.65ml) was added in four equal portions at 5 minute intervals and the reaction stirred at room temperature for 16 hours. The reaction was poured into saturated sodium bicarbonate solution (200ml) and extracted with ethyl acetate. The extracts were dried (MgSO₄) and evaporated to give a yellow solid which was purified by reverse phase chromatography eluting with 50% to 80% methanol in 0.1 % aqueous ammonium acetate. Yield 0.38g.
MS: APCI(+ve) 338 (M+1, 100%)

### iii) (±)-3-[[5-(7-Ethyl-9,10-dihydro-2-methyl-4H-benzo[5,6]cyclohepta[1,2-d]oxazol-4-yl)-3,4-dihydro-2,4-dioxo-1(2H)-pyrimidinyl]methyl]benzoic acid, methyl ester

The subtitle compound was prepared from the product of step (ii) (0.38g) and methyl 3-[bromomethyl]benzoate according to the method of example 1 step (viii).
Yield 0.105g.
MS: APCI(+ve) 486 (M+1, 100%)

### iv)(±)-3-[[5-(7-Ethyl-9,10-dihydro-2-methyl-4H-benzo[5,6]cyclohepta[1,2-d]oxazol-4-yl)-3,4-dihydro-2-oxo-4-thioxo-1(2H)-pyrimidinyl]methyl]benzoic acid, methyl ester

The subtitle compound was prepared from the product of step (iii) (0.1g) according to the method of example 2 step (ii). Yield 0.045g.
MS: APCI(+ve) 502 (M+1, 100%)

### v)(±)-3-[[5-(7-Ethyl-9,10-dihydro-2-methyl-4H-benzo[5,6]cyclohepta[1,2-d]oxazol-4-yl)-3,4-dihydro-2-oxo-4-thioxo-1(2H)-pyrimidinyl]methyl]benzoic acid

The title compound was prepared from the product of step (iv) (0.045g) according to the method of example 2 step (iii). Purification was by reverse phase chromatography eluting with 50% to 60% methanol in 0.1% aqueous ammonium acetate. Yield 0.01g.
MS: APCI(-ve) 486 (M-1, 100%)
1H NMR: δ (DMSO) 7.90(m,2H), 7.65(s,1H), 7.45(m,3H), 6.94(m,2H), 5.61(s,1H), 5.08(d,1H), 4.96(d,1H), 2.94(m,2H), 2.70(m,2H), 2.50(q,2H), 2.28(s,3H), 1.12(t,3H)

### Example 12

### (±)-3-[[5-(7-Ethyl-2-methyl-4H-benzo[5,6]cyclohepta[1,2-d]thiazol-4-yl)-3,4-dihydro-2,4-dioxo-1(2H)-pyrimidinyl]methyl]benzoic acid

### i) 7-Ethyl-9,10-dihydro-2-methyl-4H-benzo[5,6]cyclohepta[1,2-d]thiazol-4-one

A stirred suspension of potassium *tert*-butoxide (1.80g) in dry *N,N*-dimethylformamide (20ml) was cooled to 5°C and saturated with hydrogen sulphide. The product from example 3 step (vi) (1.00g) was added and the reaction stirred at room temperature for 1 hour. The mixture was poured into ice, acidified to pH4 with 2N hydrochloric acid and extracted with ethyl acetate. The extracts were washed with brine, dried (MgSO₄) and evaporated to give an orange solid which was purified by chromatography eluting with 50% ethyl acetate in isohexane. Yield 0.84g.
MS: APCI(+ve) 258 (M+1, 100%)

### ii) (±)-4-(2,4-Bis(1,1-dimethylethoxy)pyrimidin-5-yl)-7-ethyl-2-methyl-9,10-dihydro-4H-benzo[5,6]cyclohepta[1,2-d]thiazol-4-ol

The subtitle compound was prepared from the product of step (i) according to the method of example 11 step (i). Purification was by chromatography eluting with 20% ethyl acetate in isohexane. Yield 0.907g.
1H NMR: δ (DMSO) 7.89(s,1H), 7.23(d,1H), 7.04(d,1H), 6.98(dd,1H), 5.95(s,1H), 3.19(m,1H), 2.91(t,2H), 2.81(m,1H), 2.56(q,2H), 1.55(s,9H), 1.16(m,12H)

### iii) (±)-5-(7-Ethyl-9,10-dihydro-4-hydroxy-2-methyl-4H-benzo[5,6]cyclohepta[1,2-d]thiazol-4-yl)-2,4(1H,3H)-pyrimidinedxone

A solution of the product from step (ii) (0.44g) in glacial acetic acid (10ml) was stirred at room temperature overnight. The reaction mixture was evaporated to dryness (azeotroped twice with toluene) and the residue triturated with diethyl ether to give a pink solid. Yield 0.303g.
MS: APCI(-ve) 369 (M-1, 100%)

### iv) (±)-3-[[5-(7-Ethyl-9,10-dihydro-4-hydroxy-2-methyl-4H-benzo[5,6]cyclohepta[1,2-d]thiazol-4-yl)-3,4-dihydro-2,4-dioxo-1(2H)-pyrimidinyl]methyl]benzoic acid, methyl ester

The subtitle compound was prepared from the product of step (iii) (0.35g) according to the method of example 15 step (i). Yield 0.185g.
1H NMR: δ (DMSO) 11.17(s,1H), 7.93(m,2H), 7.60(m,3H), 7.20(d,1H), 7.01(d,1H), 6.93(dd,1H), 5.95(s,1H), 5.06(s,2H), 3.87(s,3H), 3.38(m,1H), 2.93(m,3H), 2.53(q,2H), 2.46(s,3H), 1.15(t,3H)

### v) (±)-3-[[5-(7-Ethyl-2-methyl-4H-benzo[5,6]cyclohepta[1,2-d]thiazol-4-yl)-3,4-dihydro-2,4-dioxo-1(2H)-pyrimidinyl]methyl]benzoic acid, methyl ester

A solution of the product from step (iv) (0.18g) in glacial acetic acid (6ml) was stirred at 100°C for 2 hours. The solution was evaporated to dryness (azeotroped twice with toluene) and the residue purified by chromatography eluting with ethyl acetate. Yield 0.15g.
MS: APCI(+ve) 500 (M+1, 100%)

### vi)(±)-3-[[5-(7-Ethyl-2-methyl-4H-benzo[5,6]cyclohepta[1,2-d]thiazol-4-yl)-3,4-dihydro-2,4-dioxo-1(2H)-pyrimidinyl]methyl]benzoic acid

The title compound was prepared from the product of step (v) according to the method of example 2 step (iii). Purification was by reverse phase chromatography eluting with 50% to 60% methanol in 0.1% aqueous ammonium acetate. Yield 0.014g.
MS: APCI(+ve) 486 (M+1, 100%)
1H NMR: δ (DMSO) 7.90(m,1H), 7.80(s,1H), 7.44(m,3H), 7.22(dd,1H), 7.14(d,1H), 6.98(s,1H), 6.79(dd,2H), 5.46(s,1H), 4.92(d,1H), 4.80(d,1H), 2.60(s,3H), 2.56(q,2H), 1.14(t,3H)
MP: 159-161°C

### Example 13

### (±)-3-[[5-(2,7-Dimethyl-10H-benzo[4,5]cyclohepta[1,2-d]oxazol-10-yl)-3,4-dihydro-2-oxo-4-thioxo-1(2H)-pyrimidinyl]methyl]benzoic acid

### i) 5-(3-Methylphenyl)-3-oxopentanoic acid, ethyl ester

Ethyl acetoacetate (74.5ml) was added dropwise to a solution of lithium diisopropylamide (2.0M solution in heptane/tetrahydrofuran/ethylbenzene,600ml) in tetrahydrofuran (2.5L) at 0°C. The mixture was stirred for 30 min then 3-methylbenzylbromide (79ml) was added dropwise. The mixture was stirred for 1 hour at 0°C then quenched with 2M HCl. The mixture was extracted with ethyl acetate, the extracts washed with water, dried (MgSO₄) and evaporated to give an oil which was purified by chromatography eluting with 5-10% ethyl acetate in isohexane. Yield 84.7g.
MS: APCI(-ve) 233 (M-1, 100%)

### ii) 2-Acetoxy-5-(3-methylphenyl)-3-oxopentanoic acid, ethyl ester

Lead tetraacetate (60g) was added in 10g portions over 1 hour to a stirred solution of the product from step (i) (40g) in benzene (200ml) at room temperature. The mixture was stirred for 0.5 hour, quenched with ice-cold water and extracted with ether. The extracts were washed with aqueous sodium bicarbonate, dried (MgSO₄) and evaporated to give an oil which was purified by chromatography eluting with 5-10% ethyl acetate in isohexane. Yield 19.7g.
MS: APCI(-ve) 291 (M-1, 100%)

### iii) 2-Methyl-4-[2-(3-methylphenyl)ethyl]-5-oxazolecarboxylic acid, ethyl ester

A mixture of the product from step (ii) (19.6g) and ammonium acetate (25.8g) in acetic acid (150ml) was heated under reflux for 2 hours. The reaction mixture was evaporated to dryness and the residue partitioned between water and ethyl acetate. The organic phase was washed with aqueous sodium bicarbonate and water, dried (MgSO₄) and evaporated under reduced pressure. The product was used without purification in the next step.
MS: APCI(+ve) 274 (M+1, 100%)

### iv) 2-Methyl-4-[2-(3-methylphenyl)ethyl]-5-oxazolecarboxylic acid

The subtitle compound was prepared from the product from step (iii) according to the method of example 1 step (ix). Purification was by trituration with ethyl acetate and isohexane. Yield 11.0g.
MS: APCI(-ve): 244 (M-1,100%)

### v) 4,5-Dihydro-2,7-dimethyl-10H-benzo[4,5]cyclohepta[1,2-d]oxazol-10-one

Oxalyl chloride (4.8ml) was added dropwise to a stirred suspension of the product from step (iv) (9.0g) and *N,N*-dimethylformamide (3 drops) in dichloromethane (100ml) at room temperature. After 2 hours the solvent was removed under reduced pressure. The residue was dissolved in 1,2-dichloroethane (140ml), aluminium trichloride (14.7g) added and the mixture heated under reflux for 1 hour. The reaction mixture was added slowly to a stirred mixture of ice and 1M HCl, then extracted with chloroform. The extracts were dried (MgSO₄) and evaporated under reduced pressure to 40ml. The product was obtained by precipitation with isohexane. Yield 5.52g.
MS: APCI(+ve) 228 (M+1, 100%)

### vi)(±)-5-(4,5-Dihydro-10-hydroxy-2,7-dimethyl-10H-benzo[4,5]cyclohepta(1,2-d]oxazol-10-yl)-2,4-bis(1,1-dimethylethoxy)pyrimidine

The subtitle compound was prepared from the product from step (v) (6.0g) according to the method of example 11 step (i). The product was used without further purification in the next step. Yield 11.37g.

### vii)(±)-5-(2,7-Dimethyl-10H-benzo[4,5]cyclohepta[1,2-d]oxazol-10-yl)-2,4(1H,3H)-pyrimidinedione

A solution of the product from step (vi) (5.7g) in acetic acid (20ml) was added dropwise over 5 min to acetic acid heated at 100°C. The mixture was heated for a further 3 hours at 100°C, the solvent removed under reduced pressure and azeotroped with toluene. Purification was by chromatography eluting with 5% methanol in dichloromethane. Yield 0.67g
MS: APCI(+ve) 322 (M+1, 100%)

### viii)(±)-3-[[5-(2,7-Dimethyl-10H-benzo[4,5]cyclohepta[1,2-d]oxazol-10-yl)-3,4-dihydro-2,4-dioxo-1(2H)-pyrimidinyl]methyl]benzoic acid, methyl ester

The subtitle compound was prepared from the product of step (vii) and methyl 3-(bromomethyl)benzoate (690mg) by the method of example 1 step (viii). Yield 309mg.
MS: APCI(+ve) 470 (M+1, 100%)

### ix)(±)-3-[[5-(2,7-Dimethyl-10H-benzo[4,5]cyclohepta[1,2-d]oxazol-10-yl)-3,4-dihydro-2-oxo-4-thioxo-1(2H)-pyrimidinyl]methyl]benzoic acid, methyl ester

The subtitle compound was prepared from the product of step (viii) (0.47g) by the method of example 2 step (ii). Purification was by chromatography eluting with 30-50% ethyl acetate in isohexane. Yield 245mg.
MS: APCI(+ve) 486 (M+1, 100%)

### x)(±)-3-[[5-(2,7-Dimethyl-10H-benzo[4,5]cyclohepta[1,2-d]oxazol-10-yl)-3,4-dihydro-2-oxo-4-thioxo-1(2H)-pyrimidinyl]methyl]benzoic acid

The title compound was prepared from the product of step (ix) according to the method of example 2 step (iii). Purification was by precipitation from ethyl acetate with isohexane. Yield 0.06g.
MS: APCI(+ve) 472 (M+1, 100%)
1H NMR: δ (DMSO) 13.15(s,1H), 12.83(s,1H), 7.93(d,1H), 1.80(s,1H), 7.54-7.46(m,3H), 7.17(d,1H), 7.14(s,1H), 7.07(s,1H), 6.57(AB quartet,2H), 6.12(s, 1H), 4.87(AB quartet,2H), 2.37(s,3H), 2.26(s,3H)
MP: 180°C

### Example 14

### (±)-5-(2,7-Dimethyl-4H-benzo[5,6]cyclohepta[1,2-d]oxazol-4-yl)-1-(3-methyl-5-(5-trifluoromethyl)-1H-1,2,4-triazol-3-yl)phenylmethyl-3,4-dihydro-4-thioxo-2(1H)pyrimidinone

### i) 2-(Hydroxyimino)-5-(3-methylphenyl) -3-oxopentanoic acid, ethyl ester

Sodium nitrite (32g) in water (60ml) was added dropwise over 1hour to a stirred solution of the product from example 13 step (i) (99.4g) in acetic acid (200ml) at a rate such that the internal temperature did not rise above 30 °C. The solution was allowed to stir a further 1hour before adding water (500ml). After stirring for a further 24h the reaction mixture was extracted with diethyl ether (X4). The combined ether extracts were washed with saturated sodium bicarbonate solution, and finally water before collecting the organic layer and drying over magnesium sulphate. The solution was filtered and the solvent evaporated under reduced pressure to leave the subtitle product. Yield:88g
1H NMR: δ (DMSO) 13.23 (s, 1H), 7.15-7.20 (m, 1H), 6.95-7.05 (m, 3H), 4.25 (q,2H), 3.1 (t, 2H), 2.8 (t, 2H), 2.22 (s, 3H), 1.2 (t, 3H)

### ii) 2-(Acetylamino) -5-(3-methylphenyl)-3-oxopentanoic acid, ethyl ester

Zinc dust (10 micron, 150g) was added portionwise to a stirred solution of the product from step (i) (88g) in acetic acid (400ml) and acetic anhydride (125ml) keeping the internal temperature below 40°C. The solution was stirred at room temperature for a further 1hour before adding water (500ml) dropwise at such a rate so as to keep the internal temperature below 40 °C. The mixture was stirred at room temperature for a further 16h then filtered and the mother liquor extracted with chloroform. The organic extracts were further washed with brine and water, collected dried over magnesium sulphate and solvent removed under reduced pressure to give the subtitle product. Yield:70g
MS: APCI(+ve); 290 (M-1, 100%)

### iii) 2-Methyl-5-(2-(3-methylphenyl)ethyl)-4-oxazole carboxylic acid, ethyl ester

Thionyl chloride (200ml) was added dropwise over 15min to the product from step (ii) (70g) with cooling provided by an ice bath. The mixture was then set at reflux for 1h. After cooling to room temperature the thionyl chloride was evaporated under reduced pressure and the residue partitioned between ethyl acetate and saturated sodium bicarbonate solution. The organic layer was collected, dried over magnesium sulphate and solvent removed under reduced pressure. Purification of the residue was performed by flash chromatography eluting with 2% ethanol in dichloromethane. Yield:39g
MS: APCI(+ve); 274 (M+1, 30%, 228, 100%)

### iv) 2-Methyl - 5-(2-(3-methylphenyl)ethyl)-4-oxazolecarboxylic acid

The product from step (iii) (39g) was stirred in a solution of methanol (100ml), water (50ml) and lithium hydroxide hydrate (16.8g). After 16h the slurry was evaporated to remove the methanol and the residue partitioned between ethyl acetate and 2M hydrochloric acid. The organic layer collected, dried over magnesium sulphate and solvent removed under reduced pressure. Yield: 33g
MS: APCI(+ve); 246 (M+1, 10%, 228, 100%)

### v) 9,10-Dihydro-2,7-dimethyl-4H-benzo[5,6]cyclohepta[1,2-d]oxazol-4-one

The product of step (iv) (31g) was stirred with oxalyl chloride (16.6ml) in dichloromethane (300ml) at room temperature for 2h. The solvent/reagents were evaporated under reduced pressure to dryness. The residue was suspended in 1,2-dichloroethane (200ml) and aluminium chloride (65g) added. The mixture was then set at reflux for 1h. The cooled reaction mixture was added to 1N HCl (1.5L) and ice and then extracted with dichloromethane (X4). The combined extracts were dried over magnesium sulphate and solvent evaporated under reduced pressure. Purification was by flash chromatography eluting with 1% ethanol in dichloromethane to give the subtitle product. Yield: 9g
MS: APCI(+ve); 228 (M+1, 100%)

### vi) (±)-5-(9,10-Dihydro-4-hydroxy-2,7-dimethyl-4H-benzo[5,6]cyclohepta[1,2-d]oxazol-4-yl)-2,4(1H,3H)-pyrimidinedione

To a solution of the product from example 1 step (vi) (9g) in dry tetrahydrafuran (100ml) under nitrogen at -78 °C was added n-butyllithium (2.5M, 14.3ml) dropwise over 1min. After 30min a warm solution of the product from step (v) (6.73g) in tetrahydrofuran (350ml) was added rapidly via cannula. After further stirring for 1h the cooling bath was removed and the mixture allowed to stir at room temperature for 30min. The reaction mixture was quenched with saturated brine and extracted with ethyl acetate (X2). The combined organic extracts were dried over magnesium sulphate and solvent removed under reduced pressure to leave a pale yellow foam. This was dissolved in acetic acid (100ml) and stired at room temperature for 16h. The solvent was evaporated under reduced pressure and the residue azeotroped with toluene (X2) to remove last traces of acetic acid. The remaining residue was triturated with diethyl ether / isohexane mixtures and filtered to give the subtitle product as a beige solid.Yield: 11 g
MS: APCI(-ve); 338 (M-1, 100%)

### vii) N-(2-Cyanoethyl)-3,5-dimethylbenzenecarboxamide

A stirred suspension of 3,5-dimethylbenzoic acid (50g) in dry dichloromethane (500ml) was treated dropwise with oxalyl chloride (100ml) and then set at reflux for 16h. Another aliquot of oxalyl choride (50ml) was added and reflux continued for a further 6h. The solvents/reagents were removed under reduced pressure and the residue azeotroped with benzene (X2). The resultant yellow oil was dissolved in dry tetrahydrofuran to a final volume of 250ml. This solution was added dropwise in five 50ml portions alternating with five 67ml portions of 1.0N aqueous sodium hydroxide into a stirred solution of 3-aminopropionitrile fumarate (42.7g) in 1.0N aqueous sodium hydroxide (333ml) at 0 °C under nitrogen. After addition was complete the reaction mixture was allowed to attain room temperature and further stirred for 16h. Water (500ml) was added and the mixture extracted with ethyl acetate (3X500ml). The organic layer was further washed with saturated sodium bicarbonate solution (2X500ml). The organic layer was collected, dried over magnesium sulphate and solvent evaporated under reduced pressure to give the subtitle product as a white solid. Yield:48.85g
MS: APCI(+ve); 203 (M+1, 100%), APCI(-ve); 201 (M-1, 100%)

### viii) N-(2-Cyanoethyl)-3,5-dimethylbenzenecarbohydrazonamide

A mixture of the product from step (vii) (35.68g) and phosphorus pentachloride (36.86g) was stirred under a water-aspirator vacuum and heated at 100°C giving rise to a yellow oil. The oil was further stirred at 100 °C under vacuum for a further 30 min. The boiling phosphorus oxychloride liberated was collected by distillation to leave a dark brown oil which was cooled to room temperature and dissolved in dry 1,4-dioxane (50ml). This solution was transferred rapidly via cannula to a stirring solution of hydrazine in tetrahydrofuran (1.0M, 882ml) under nitrogen at room temperature. An exotherm was produced raising the reaction temperature to ∼ 40°C. The resultant opaque yellow solution was further stirred for 16h. The solvent was removed under reduced pressure and the residue partitioned between ethyl acetate and water. The organic layer was collected, dried over magnesium sulphate and solvent removed under reduced pressure to give the subtitle product as a red oil (34.7g). This was not purified but used directly in the next step

### ix) 3-(3,5-Dimethylphenyl)-5-trifluoromethyl -1H-1,2,4-triazole

A solution of the crude product from step (viii) (34.7g) in dichoromethane (50ml) was added to trifluoroacetic anhydride (1200ml) at 0°C, under nitrogen with stirring. The cooling bath was removed and the resultant bright-orange solution stirred at room temperature for a further 16h. The solvent/reagent was removed under reduced pressure and the residue dissolved in ethyl acetate (500ml) and extracted with 1.0N sodium hydroxide solution (3X200ml) and saturated brine (400ml), organic layer collected, dried over magnesium sulphate and solvent removed under reduced pressure to leave an orange solid.(42g). This solid was dissolved in tetrahydrofuran (600ml) and treated with N sodium hydroxide (157ml). The resultant dark solution was stirred for 5h. The reaction mixture was concentrated to dryness under reduced pressure and the residue treated with water (500ml) and partitioned between ethyl acetate and 2N hydrochloric acid. The combined organic extracts were collected, dried over magnesium sulphate and solvent evaporated to leave a viscous red oil. Purification by flash chromatography eluting with 4:1 isohexane/ethyl acetate gave the subtitle product as a pale pink solid. Yield: 17.47g
MS: APCI(+ve); 242 (M+1, 100%), APCI(-ve); (M-1, 100%)

### x) 3-(3,5-Dimethylphenyl)-5-trifluoromethyl-4-(2-(trimethylsilyl)ethoxymethyl)-1,2,4-triazole

A stirred solution of the product from step (ix) (17.46g) in dry N,N-dimethylformamide (300ml) was treated with 60% dispersion of sodium hydride (3.22g) in portions at room temperature under nitrogen. After the initial effervescence had subsided the resultant orange oil was stirred for a further 30min before being treated with 2-(trimethylsilyl)ethoxymethyl chloride (14.3ml) dropwise giving a pale yellow suspension which was further stirred for 30min. The mixture was carefully poured onto water (700ml) and extracted with ethyl acetate (3X250ml). The combined organic extracts were further washed with water (3X250ml) and saturated brine (250ml) before collecting, drying over magnesium sulphate and evapoaration of the solvent under reduced pressure to give the subtitle product as a yellow oil. Yield: 26.1g. This was not purified but used directly in the next step.

### xi) 3-(3-Bromomethyl-5-methylphenyl)-5-trifluoromethyl -4-(2-(trimethylsilyl)ethoxymethyl)-1,2,4-triazole

The product from step (x) (2g), N-bromosuccinamide (1g) in dry ethyl acetate (25ml) was irradiated at reflux with a 500 Watt halogen lamp for 1h. The cooled reaction mixture was partitioned with dilute sodium bicarbonate solution. The organic layer collected, dried (MgSO₄) and solvent removed by evaporation under reduced pressure. Yield:2.5g
The product was not purified but used directly in the next step.

### xii) (±)-5-(4-Hydroxy-9,10-dihydo 2,7-dimethyl- -4H-benzo[5,6]cyclohepta[1,2-d]oxazol-4-yl)-1-(3-methyl-5-(5-trifluoromethyl-4-(2-(trimethylsilyl)ethoxymethyl)-1,2,4-triazol-3-yl)phenylmethyl-3,4-dihydro-2,4(1H, 3H)-pyrimidinedione

The product from step (xi) (2.5g) was added to a stirred solution of the product of step (vi) (2.8g), cesium carbonate (2.7g) in dry dimethylsulphoxide (20ml) at room temperature. After 30 min. the reaction mixture was partitioned between brine and ethyl acetate. The organic layer was further washed with brine (X4), collected, dried over MgSO₄ and solvent removed under reduced pressure to leave a pale yellow foam. The crude product was purified by silica gel chromatography eluting with ethyl acetate to give the subtitle product as a colourless foam. Yield: 0.75g
MS: APCI (+ve); 691 (M-18, 100%)

### xiii) (±)-5-(2,7-Dimethyl-4H-benzo[5,6]cyclohepta[1,2-d]oxazol-4-yl)-1-(3-methyl-5-(5-trifluoromethyl)-1,2,4-triazol-3-yl)phenylmethyl-3,4-dihydro-2,4-dioxo-1(2H)pyrimidinedione

The product from step (xii) (0.75g) was dissolved in acetic acid (50ml) and heated at reflux for 16h. The solvent was removed and reduced pressure and replaced with trifluoroacetic acid (50ml) and heated at reflux for 1h. The solvent removed under reduced pressure . The residue purified by flash chromatography to give the subtitle product. Yield: 0.32g
MS: APCI (+ve); 561 (M+1, 100%)

### xiv) (±)-5-(2,7-Dimethyl-4H-benzo[5,6]cyclohepta[1,2-d]oxazol-4-yl)-1-(3-methyl-5-(5-trifluoromethyl)-1H-1,2,4-triazol-3-yl)phenylmethyl-3,4-dihydro-4-thioxo-2(1H)pyrimidinone

The title compound was prepared from the product of step (xiii) (0.32g) according to the method of example 2 step (ii). Purification was by flash chromatography eluting with 60/40 iso-hexane/ethyl acetate. Yield: 0.05g
MS: APCI (+ve): 577 (M+1, 100%, APCI (-ve); 575 (M-1, 100%)
1H NMR: δ (DMSO) 15.32 (bs, 1H), 12.76 (bs, 1H), 7.87 (s, 1H), 7.70 (s, 1H), 7.58 (d, 1H), 7.25 (s, 1H), 7.20 (s, 1H), 7.15 (d, 1H), 7.0 (s, 1H), 6.60 (dd, 2H), 5.87 (s, 1H), 4.90 (q, 2H), 2.50 (s, 3H), 2.40 (s, 3H), 2.20 (3, 3H)
MP: 184 °C

### Example 15

### (±)-3-[5-(2,7-Dimethyl-4H-benzo[5,6]cyclohepta[1,2-d]oxazol-4-yl)-3,4-dihydro-2-oxo-4-thioxo-1(2H)-pyrimidinylmethyl]benzoic acid

### i) (±) -3-[9,10-Dihydro-4-hydroxy-2,7-dimethyl-4H-benzo[5,6]cyclohepta[1,2-d]oxazol-4-yl)-2,4-dioxo-(1H,3H)-pyrimidinylmethyl]benzoic acid methyl ester

A mixture of the product from example 14 step (vi) (3.57g) and cesium carbonate (3.43g) in dry dimethyl sulphoxide (40ml) was stirred for 20 minutes and treated dropwise with a solution of methyl 3-(bromomethyl)benzoate (1.21g) in dry dimethyl sulphoxide (10ml) and stirred for 4 hours. The solution was partitioned between brine and ethyl acetate. The organic extracts were washed with brine, dried (MgSO₄) and evaporated under reduced pressure to give a yellow foam. Purification was by flash column chromatography eluting with ethyl acetate. Yield 1.08g.
1H NMR: δ (DMSO) 11.21(s,1H), 7.95(d,1H), 7.91(d,1H), 7.76(s,1H), 7.65(d,1H), 7.56(t,1H), 7.05(m,2H), 6.89(dd,1H), 5.91(s,1H), 5.08(s,2H), 3.87(s,3H), 2.86(m,4H), 2.26(s,3H), 2.24(s,3H)

### ii) (±) -3-[5-(2,7-Dimethyl-4H-benzo[5,6]cyclohepta[1,2-d]oxazol-4-yl)-3,4-dihydro-2,4-dioxo-1(2H)-pyrimidinylmethyl]benzoic acid, methyl ester

A solution of the product from step (i) (1.07g) in trifluoroacetic acid (30ml) was stirred under reflux for 48hrs. The solution was evaporated under reduced pressure and azeotroped twice with toluene. The residue was purified by flash column chromatography eluting with ethyl acetate. Yield 0.41g.
MS: APCI(+ve):470 (M+1, 100%), APCI(-ve):468 (M-1, 100%)

### iii) (±) 3-[5-(2,7-Dimethyl-4H-benzo[5,6]cyclohepta[1,2-d]oxazol-4-yl)-3,4-dihydro-2-oxo-4-thioxo-1(2H)-pyrimidinylmethyl]benzoic acid, methyl ester

The subtitle compound was prepared from the product of step (ii) according to the method of example 2 step (ii). Purification was by flash column chromatography eluting with 30% to 100% ethyl acetate in isohexane. Yield 0.26g.
MS: APCI(+ve):486 (M+1, 100%), APCI(-ve):484 (M-1, 100%)

### iv) (±)-3-[5-(2,7-Dimethyl-4H-benzo[5,6]cyclohepta[1,2-d]oxazol-4-yl)-3,4-dihydro-2-oxo-4-thioxo-1(2H)-pyrimidinylmethyl]benzoic acid

The title compound was prepared from the product of step (iii) (0.16g) according to the method of example 1 step (ix). Purification was by reverse phase chromatography eluting with 40%-60% methanol in 0.1 % aqueous ammonium acetate. Yield 0.053g
MS: APCI(+ve):472 (M+1, 100%), APCI(-ve):470 (M-1, 100%)
1H NMR: δ (DMSO) 7.91(d,1H), 7.84(s,1H), 7.57(d,1H), 7.35(t,1H), 7.24(d,1H), 7.20(s,1H), 7.13(d,2H), 6.73(dd,2H), 5.85(s,1H), 4.94(d,1H), 4.74(d,1H), 2.39(s,3H), 2.21(s,3H).

### Example 16

### (±)-1-Methyl-5-(2,7-dimethyl-4H-benzo[5,6]cyclohepta[1,2-d]oxazol-4-yl)-3,4-dihydro-4-thioxo-2(1H)-pyrimidinone

### i) (±)-1-Methyl-5-(9,10-dihydro-4-hydroxy-2,7-dimethyl-4H-benzo[5,6]cyclohepta[1,2-d]oxazol-4-yl)-2,4(1H,3H)-pyrimidinedione

The subtitle compound was prepared from the product of example 14 step (vi) (1.55g) and methyl iodide (0.284ml) according to the method of example 15 step (i). Purification was by flash column chromatography eluting with 5% methanol in ethyl acetate. Yield 0.20g MS: APCI(-ve):352 (M-1,100%)

### ii) (±)-1-Methyl-5-(2,7-dimethyl-4H-benzo[5,6]cyclohepta[1,2-d]oxazol-4-yl)-2,4(1H,3H)-pyrimidinedione

The subtitle compound was prepared from the product of step (i) (0.55g) according to the method of example 15 step (ii). Purification was by flash column chromatography eluting with 5% methanol in ethyl acetate. Yield 0.20g.
MS: APCI(+ve):336 (M+1,100%)

### iii) (±)-1-Methyl-5-(2,7-dimethyl-4H-benzo[5,6]cyclohepta[1,2-d]oxazol-4-yl)-3,4-dihydro-4-thioxo-2(1H)-pyrimidinone

The title compound was prepared from the product from step (ii) (0.20g) according to the method of example 2 step (ii). Purification was by flash column chromatography eluting with 2% methanol in dichloromethane. Yield 0.039g.
MS: APCI(+ve):352 (M+1, 100%), APCI(-ve):350 (M-1, 100%)
1H NMR: δ (DMSO) 12.67(s,1H), 7.62(d,1H), 7.24(s,1H), 7.18(d,2H), 6.91(d,1H), 6.82(d,1H), 5.99(s,1H), 3.18(s,3H), 2.42(s,3H), 2.27(s,3H).
MP: 146-150°C

### Example 17

### (±)-1-Methyl-5-(2,6-dimethyl-4H-benzo[5,6]cyclohepta[1,2-d]oxazol-4-yl)-3,4-dihydro-4-thioxo-2(1H)-pyrimidinone

### i) 5-(4-Methylphenyl)-3-oxopentanoic acid, ethyl ester

The subtitle compound was prepared from 4-methylbenzyl bromide (76.7g) according to the method of example 13 step (i). Purification was by flash column chromatography eluting with 10% diethyl ether in isohexane. Yield 62g.
MS: APCI(+ve):235 (M+1, 100%)

### ii) 2-(Hydroxyimino)-5-(4-methylphenyl)-3-oxo-pentanoic acid, ethyl ester

The subtitle compound was prepared from the product of step (i) (62g) according to the method of example 14 step (i). Yield 69g
MS: APCI(-ve):262 (M-1, 100%)

### iii) 2-Acetylamino-5-(4-methylphenyl)-3-oxo-pentanoic acid, ethyl ester

The subtitle compound was prepared from the product of step (ii) (70g) according to the method of example 14 step (ii). Yield 73g.
1H NMR: δ (DMSO) 8.65(1H,d), 7.10-7.00(m,4H), 5.21(d,1H), 4.10(q,2H), 2.95-2.90(m,2H), 2.80-2.70(m,2H), 2.25(s,3H), 1.91(s,3H), 1.17(t,3H)

### iv) 5-[2-(4-Methylphenyl)ethyl]-2-methyloxazole-4-carboxylic acid, ethyl ester

The subtitle compound was prepared from the product from step (iii) according to the method of example 14 step (iii). Yield 26g
MS: APCI(+ve):274 (M+1, 100%)

### v) 5-[2-(4-Methylphenyl)ethyl]-2-methyloxazole-4-carboxylic acid

The subtitle compound was prepared from the product of step (iv) (26g) according to the method of example 14 step (iv). Yield 21.40g
MS: APCI(-ve):244 (M-1, 100%)

### vi) 9,10-Dihydro-2,6-dimethyl-4H-benzo[5,6]cyclohepta[1,2-d]oxazole-4-one

A mixture of the product from step (v) (21.5g), polyphosphoric acid (120g) and sulpholane (60ml) was stirred at 130°C for 2 days. The cooled mixture was poured onto ice/water and extracted with ethyl acetate. The extracts were washed with water, dried (MgSO₄) and evaporated. Purification was by flash column chromatography eluting with 2% ethyl acetate in dichloromethane followed by recrystallisation from ethyl acetate. Yield 4.00g.
1H NMR: δ (DMSO) 7.55(br s,1H), 7.33(d,1H), 7.28(d,1H), 3.20-3.00(m,4H), 2.49(s,3H), 2.33(s,3H)

### vii) (±)-4-(2,4-Bis(1,1-dimethylethoxy)pyrimidin-5-yl)-9,10-dihydro-2,6-dimethyl-4H-benzo[5,6]cyclohepta[1,2-d]oxazol-4-ol

The subtitle compound was prepared from the product of step (vi) (7.80g) according to the method of Example 11 step (i). Purification was by flash column chromatography eluting with 1:1 ethyl acetate:isohexane. Yield 10.65g.
1H NMR: δ (DMSO) 8.13(s,1H), 7.12(d,1H), 6.99(d,1H), 6.93(s,1H), 5.90(s,1H), 3.29(m,1H), 2.74(m,3H), 2.29(s,3H), 2.17(s,3H), 1.57(s.9H), 1.14(s,9H).

### viii) (±)-5-(9,10-Dihydro-4-hydroxy-2,6-dimethyl-4H-benzo[5,6]cyclohepta[1,2-d]oxazol-4-yl)-2,4(1H,3H)-pyrimidinedione

The subtitle compound was prepared from the product of step (vii) (10.00g) according to the method of example 12 step (iii). Yield 7.15g
MS: APCI(-ve):338 (M-1, 100%)

### ix) (±)-1-Methyl-5-(9,10-dihydro-4-hydroxy-2,6-dimethyl-4H-benzo[5,6]cyclohepta[1,2-d]oxazol-4-yl)-2,4(1H,3H)-pyrimidinedione

The subtitle compound was prepared from the product of step (viii) (7.10g) according to the method of example 21 step (vi). Purification was by flash column chromatography eluting with 5% methanol in dichloromethane. Yield 1.96g
MS: APCI(-ve):352 (M-1, 100%)

### x) (±)-1-Methyl-5-(2,6-dimethyl-4H-benzo[5,6]cyclohepta[1,2-d]oxazol-4-yl)-2,4(1H,3H)-pyrimidinedione

The subtitle compound was prepared from the product of step (ix) (1.93g) according to the method of example 15 step (ii). Purification was by flash column chromatography eluting with 2% methanol in dichloromethane. Yield 0.275g
MS: APCI(+ve):336 (M+1, 100%), APCI(-ve):334 (M-1, 100%)

### xi) (±)-1-Methyl-5-(2,6-dimethyl-4H-benzo[5,6]cyclohepta[1,2-d]oxazol-4-yl)-3,4-dihydro-4-thioxo-2(1H)-pyrimidinone

The title compound was prepared from the product of step (x) (0.245g) according to the method of example 2 step (ii). Purification was by flash column chromatography eluting with 30% acetone in isohexane. Yield 0.057g
MS: APCI(+ve):352 (M+1, 100%), APCI(-ve):350 (M-1, 100%)
1H NMR: δ (DMSO) 12.68(s,1H), 7.55(s,1H), 7.33(d,1H), 7.19(s,1H), 7.10(dd,1H), 6.92(d,1H), 6.78(d,1H), 6.00(s,1H), 3,18(s,3H), 2.41(s,3H), 2.29(s,3H)
MP: 238-240 °C

### Example 18

### (±)-1-Methyl-5-(2,7-dimethyl-4H-benzo[5,6]cyclohepta[1,2-d]thiazol-4-yl)-3,4-dihydro-4-thioxo-2(1H)-pyrimidinone

### i) 2,7-Dimethyl-9,10-dihydro-4H-benzo[5,6]cyclohepta[1,2-d]thiazol-4-one.

A stirred suspension of potassium tert-butoxide (6.4g), in N,N-dimethylformamide (60ml) was saturated at 5°C with hydrogen sulphide. The mixture was treated with the product from example 14 step (v) (3.4g), stirred for 2h and poured onto ice. The mixture was acidified to pH 4 with dilute hydrochloric acid, and extracted with ethyl acetate. The organic phase was collected, dried (MgSO₄) and evaporated under reduced pressure to give a solid. Recrystallised from ethyl acetate/isohexane gave a buff solid. Yield 1.5g
MS: APCI(+ve):244 (M+1, 100%)

### ii) (±)-5-[9,10-Dihydro-4-hydroxy-2,7-dimethyl-4H-benzo[5,6]cyclohepta[1,2-d]thiazol-4-yl]-2,4(1H,3H)-pyrimidinedione

The subtitle compound was prepared from the product of step (i) (1.9g), by the method of example 14, step (vi). Yield 1.5g
MS: APCI(-ve):354 (M-1, 100%)

### iii) (±)-5-[9,10-Dihydro-4-hydroxy-2,7-dimethyl-4H-benzo[5,6]cyclohepta[1,2-d]thiazol-4-yl]-1-methyl-2,4(1H,3H)-pyrimidinedione

The subtitle compound was prepared from the product of step (ii) (0.7g), by the method of example 21, step (vi). Yield 0.22g
MS: APCI(+ve):366 (M+1, 100%)

### iv) (±)-1-Methyl-5-[2,7-dimethyl-4H-benzo[5,6]cyclohepta[1,2-d]thiazol-4-yl]-2,4(1H,3H)-pyrimidinedione.

A stirred solution of the product from step (iii) (0.22g), in glacial acetic acid (5ml) was stirred at 100°C for 3h and evaporated to give an oil. Yield: 190mg. Used directly in the step.

### v) (±)-1-Methyl-5-(2,7-dimethyl-4H-benzo[5,6]cyclohepta[1,2-d]thiazol-4-yl)-3,4-dihydro-4-thioxo-2(1H)-pyrimidinone

The title compound was prepared from the product from step (iv) (0.19g) according to the method of example 2 step (ii). Purification was by flash column chromatography eluting with 0 to 1% methanol in dichloromethane, followed by a second flash column eluting with 30% ethyl acetate in toluene. Yield: 0.07g.
MS: APCI(+ve): 368 (M+1, 100%)
1H NMR: δ (DMSO) 2.27(s,3H), 2.62(s,3H), 3.26(s,3H), 6.05(s,1H), 7.03(d of d, 2H), 7.16-7.25(mult, 2H), 7.50-7.53(mult, 2H), 12.65 (s1,H). MP: 235-237 "C

### Example 19

### (±)-1-Methyl-5-(2,7-dimethyl-10H-benzo[4,5]cyclohepta[1,2-d]oxazol-10-yl)-2,4-(1H,3H)-pyrimidinedione

The title compound was prepared from the product of example 13 step (vii) (0.66g) and iodomethane (0.49ml) according to the method of example 1 step (viii). Purification was by chromatography eluting with 5% methanol in dichloromethane. Yield 0.315g.
MS: APCI(+ve) 336 (M+1, 100%)
1H NMR: δ (DMSO) 11.32(s,1H), 7.37(d,1H), 7.25(s,1H), 7.22(d,1H), 6.90(d,1H), 6.67(s,1H), 6.64(d,1H), 5.47(s,1H), 3.08(s,3H), 2.39(s,3H), 2.30(s,3H)

### Example 20

### (±)-1-Methyl-5-(2,7-dimethyl-10H-benzo[4,5]cyclohepta[1,2-d]oxazol-10-yl)-3,4-dihydro-2-oxo-4-thioxo-1(2H)-pyrimidine

The title compound was prepared from the product of example 19 (0.29g) according to the method of example 2 step (ii). Purification was by chromatography eluting with 2% methanol in dichloromethane followed by a second column eluting with ethyl acetate. Yield 0.080g.
MS: APCI(+ve) 352 (M+1, 100%)
1H NMR: δ (DMSO) 12.75(s,1H), 7.58(d,1H), 7.27(s,1H), 7.20(d,1H), 7.06(s,1H), 6.91(d,1H), 6.66(d,1H), 6.30(s,1H), 3.14(s,3H), 2.38(s,3H), 2.29(s,3H)
MP: 235°C

### Example 21

### (±)-1-Methyl-5-(2,7-dimethyl-10H-benzo[4,5]cyclohepta[1,2-d]thiazol-10-yl)-3,4-dihydro-4-thioxo-2(1H)-pyrimidinone

### i) 2-Methyl-4-[2-(3-methylphenyl)ethyl]-5-thiazolecarboxylic acid, ethyl ester

Bromine (4ml) was added dropwise to a stirred mixture of the product from example 13 step (i) (15g) in water at 0°C. After 15min the mixture was partitioned between ether and water. The organic phase was separated, dried(MgSO₄) and evaporated under reduced pressure. The residue was dissolved in ethanol (200ml), thioacetamide (4.8g) added and the mixture heated at reflux for 3h. The solvent was removed under reduced pressure and the residue partitioned between ethyl acetate and aqueous sodium hydrogencarbonate. The organic phase was separated, dried(MgSO₄) and evaporated under reduced pressure. Purification was by chromatography eluting with 5-10% ethyl acetate in isohexane. Yield 12.85g.
MS: APCI(+ve) 290 (M+1, 100%)

### ii) 2-Methyl-4-[2-(3-methylphenyl)ethyl]-5-thiazolecarboxylic acid

The subtitle compound was prepared from the product of step (i) (12.84g) according to the method of example 1 step (ix). Purification was by trituration with ethyl acetate and isohexane. Yield 8.23g.
1H NMR: δ (DMSO) 7.11(t, 1H), 6.99-6.94(m,3H), 3.24-3.20(m,2H), 2.85-2.81(m,2H), 2.60(s,3H), 2.22(s,3H)

### iii) 4,5-Dihydro-2,7-dimethyl-10H-benzo[4,5]cyclohepta[1,2-d]thiazol-10-one

The subtitle compound was prepared from the product of step (ii) (7.0g) according to the method of example 1 step (iii). Purification was by trituration with ethyl acetate and isohexane. Yield 3.5g.
MS: APCI(+ve) 244 (M+1, 100%)

### iv) (±)-10-(2,4-Bis(1,1-dimethylethoxy)pyrimidin-5-yl)-4,5-dihydro-2,7-dimethyl-10H-benzo[4,5]cyclohepta[1,2-d]thiazol-10-ol

The subtitle compound was prepared from the product of step (iii) (3.45g) according to the method of example 11 step (i). Purification was by chromatography eluting with 1% methanol in dichloromethane. Yield 3.22g.
MS: APCI(+ve) 468 (M+1)

### v) (±)-5-(4,5-Dihydro-10-hydroxy-2,7-dimethyl-10H-benzo[4,5]cyclohepta[1,2-d]thiazol-10-yl)-2,4(1H,3H)-pyrimidinedione

A solution of the product from step (iv) in acetic acid was stirred overnight. The solvent was removed under reduced pressure and the residue purified by chromatography eluting with 5-6% methanol in dichloromethane. Yield 2.04g.
MS: APCI(+ve) 356 (M+1)

### vi) (±)-1-Methyl-5-(4,5-dihydro-10-hydroxy-2,7-dimethyl-10H-benzo[4,5]cyclohepta[1,2-d]thiazol-10-yl)-2,4(1H,3H)-pyrimidinedione

Sodium hydride(60% dispersion by wt) (112mg) was added to a stirred solution of the product from step (v) (1.0g) in *N,N*-dimethylformamide (20ml) at room temperature. After 0.75h, methyl iodide (0.175ml) was added and stirring continued for 1h. The mixture was quenched with water and extracted with ethyl acetate. The organic phase was washed with water ; dried (MgSO₄) and evaporated under reduced pressure. Purification was by chromatography eluting with 5% methanol in dichloromethane. Yield 0.14g.
MS: APCI(+ve) 370 (M+1)

### vii) (±)-1-Methyl-5-(2,7-dimethyl-10H-benzo[4,5]cyclohepta[1,2-d]thiazol-10-yl)-2,4(1H,3H)-pyrimidinedione

The subtitle compound was prepared from the product of step (vi) (0.18g) by the method of example 12 step (v). Purification was by chromatography eluting with 3% methanol in dichloromethane.
MS: APCI(-ve) 350 (M-1)

### viii) (±)-1-Methyl-5-(2,7-dimethyl-10H-benzo[4,5]cyclohepta[1,2-d]thiazol-10-yl)-4-thioxo-2(1H)-pyrimidinone

The title compound was prepared from the product of step (vii) according to the method of example 2 step (ii). Purification was by chromatography eluting with ethyl acetate. Yield 0.008g.
MS: APCI(+ve) 368 (M+1, 100%)
1H NMR: δ (DMSO) 12.75(s, 1H), 7.31(s, 1H), 7.29(d, 1H), 7.22(d,1H), 7.12(s,1H), 7.04(d, 1H), 6.96(d, 1H), 6.08(s,1H), 3.17(s,3H), 2.56(s,3H), 2.31(s,3H)

### Example 22

### (±)-5-[8-Methyl-5H-benzo[4,5]cyclohepta[1,2-b]pyridin-5-yl]-3,4-dihydro-4-thioxo-2(1H)-pyrimidinone

### i) (E)-2-[2-(3-Methylphenyl)ethenyl]pyridine-3-carboxylic acid.

A mixture of methyl 2-methylnicotinate (24.5g), 3-methylbenzaldehyde (58ml) and anhydrous zinc chloride (25g) were heated at 180 °C under nitrogen for 0.5hr. The reaction mixture was allowed to cool to room temperature and diluted with toluene (100ml) and 10% NaOH solution (100ml). This mixture was stirred for 0.5hr and the resultant precipitate was removed by filtration. The aqueous phase was washed with toluene (3x50ml) and acidified to pH5 by the addition of glacial acetic acid. The resultant precipitate was collected by filtration, washed with water and dried. Yield 10.88g. The aqueous filtrate was extracted with chloroform (3x50ml), the combined extracts were dried (MgSO₄) and evaporated under reduced pressure.Yield 6.02g. Both crops were combined. Total yield:16.9g
MS: APCI(+ve): 240 (M+1, 100%).

### ii) 8-Methyl-5H-benzo[4,5]cyclohepta[1,2-b]pyridin-5-one

A mixture of the product from step (i) (16.90g) and polyphosphoric acid (300ml) was heated at 180°C for 4hr. After cooling to room temperature the reaction mixture was added to ice/water and insoluble material was removed by filtration through Celite. The filtrate was adjusted to pH5 by the addition of 1M NaOH solution. and extracted with ethyl acetate (3x50ml). The combined extracts were dried (Na₂SO₄) and evaporated under reduced pressure. Purification was by chromatography eluting with 30% ethyl acetate in toluene. Yield 6.50g.
(The product was contaminated with 33 mole% of the corresponding 6-methyl isomer)
MS: APCI(+ve): 222 (M+1, 100%).

### iii) (±)-8-Methyl-5H-benzo[4,5]cyclohepta[1,2-b]pyridin-5-ol

A solution of the product from step (ii) (5.87g) in dry tetrahydrofuran (50ml) was treated with diisobutylaluminum hydride (4.7ml) in one portion. The solution was stirred under nitrogen for 1.75hr then partitioned between ethyl acetate and saturated brine. The organic phase was separated, dried (Na₂SO₄) and the solvent evaporated under reduced pressure. Purification was by chromatography eluting with 50% ethyl acetate in toluene. Yield 3.56g
MS: APCI(+ve): 224 (M+1, 100%).

### iv) (±)-5-[8-Methyl-5H-benzo[4,5]cyclohepta[1,2-b]pyridin-5-yl)-2,4(1H,3H)-pyrimidinedione

A solution of uracil (1.87g) and the product of step (iii) (3.73g) in glacial acetic acid (30ml) was heated at 120 °C under nitrogen for 40hr. The solvent was evaporated under reduced pressure and the residue was partitioned between ethyl acetate and saturated. sodium bicarbonate solution. The organic phase was separated, dried (MgSO₄) and evaporated under reduced pressure. Purification was by chromatography eluting with 0-20% methanol in ethyl acetate. Yield 3.5g.
MS: APCI(+ve): 318 (M+1, 100%).

### v) (±)-5-[8-Methyl-5H-benzo[4,5]cyclohepta[1,2-b]pyridin-5-yl]-3,4-dihydro-4-thioxo-2(1H)-pyrimidinone

The title compound was prepared from the product of step (iv) (0.53g) and Lawesson's reagent (0.675g) according to the method of example 2 step (ii).
Purification was by chromatography eluting with 50% acetone in *iso*-hexane followed by trituration with acetonitrile. Yield 0.18g.
MS: APCI(+ve): 334 (M+1, 100%).
1H NMR: δ (DMSO) 12.39(s,1H), 11.26(s,1H), 8.44(d of d,1H), 8.08(d of d,1H), 7.45(d,1H), 7.34(m,2H), 7.26(d of d,1H), 7.17(d,1H), 7.01(d,1H), 6.65(s,1H), 5.73(s,1H), 2.32(s,3H).
MP: >250 °C

### Example 23

### (±)-1-Methyl-5-[8-Methyl-5H-benzo[4,5]cyclohepta[1,2-b]pyridin-5-yl]-3,4-dihydro-4-thioxo-2(1H)-pyrimidine

### i) (±)-1-Methyl-5-[8-methyl-5H-benzo[4,5]cyclohepta[1,2-b]pyridin-5-yl]-2,4-(1H,3H)-pyrimidinedione

A solution of the product of example 22 step (iv) (0.68g) in dry *N*,*N*-dimethylformamide (10ml) was treated in one portion with sodium hydride (60% dispersion by wt, 0.086g). After lhr iodomethane (0.13ml) was added and the solution was stirred for 16hr. The reaction mixture was diluted with water (50ml) and the mixture was extracted with ethyl acetate (3x20ml). The combined extracts were dried (Na₂SO₄) and the solvent was evaporated under reduced pressure. Purification was by chromatography eluting with 50-60% acetone in isohexane. Yield 0.15g.
MS: APCI(+ve): 332 (M+1, 100%).

### ii) (±)-1-Methyl-5-[8-methyl-5H-benzo[4,5]cyclohepta[1,2-b]pyridin-5-yl]-3,4-dihydro-4-thioxo-2(1H)-pyrimidinone

The title compound was prepared from the product of step (i) (0.2g) and Lawesson's reagent (0.29g) according to the method of example 2 step (ii). Purification was by chromatography eluting with 50% acetone in isohexane followed by trituration with isohexane/ethyl acetate. Yield 0.044g
MS: APCI(+ve): 348 (M+1, 100%).
1H NMR: δ (DMSO) 12.59(s,1H), 8.44(d,1H), 8.08(d,1H), 7.44(d,1H), 7.32(m,2H), 7.25(m,2H), 7.03(d,1H), 6.97(s,1H), 5.78(s,1H), 3.19(s,3H), 2.32(s,3H).
MP: 157-160°C

### Example 24

### (±)-1-Methyl-5-[2,7-dimethyl-4H-benzo[5,6]cyclohepta[1,2-d]thiazol-4-yl]-2,4(1H,3H)-pyrimidinedione

A solution of the product from example 18 step (iii) (0.11g) in glacial acetic acid (5ml) was stirred at 100°C for 3h and evaporated. Purification of the residue was by flash chromatography, eluting with 40% acetone in isohexane to give the title compound.
MS: APCI(+ve): 352 (M+1, 100%)
1H NMR: δ (DMSO) 2.28(s, 3H), 2.62(s,3H), 3.12(s,3H), 5.49(s, 1H), 6.86 (fine d, 1H), 6.96(d of d, 2H), 7.20-7.21(mult, 2H), 7.36-7.39(mult, 1H)

### Example 25

### (±)-5-[7-Chloro-2-methyl-4H-benzo[5,6]cyclohepta[1,2-d]oxazol-4-yl]-1-methyl-2,4(1H,3H)-pyrimidinedione

### i) 5-(3-Chlorophenyl)-3-oxopentanoic acid, ethyl ester

To a solution of LDA (2.0M solution in heptanes/tetrahydrofuran/ethylbenzene, 500ml) in tetrahydrofuran (2L) at 0°C was added ethyl acetoacetate (62ml). After 40 min 3-chlorobenzyl bromide (100g) was added dropwise and the mixture was stirred at 0°C for 1 hour. The reaction mixture was acidified with 2N HCl and the organic phase was concentrated under reduced pressure. The residue was dissolved in ethyl acetate and washed with water and brine, dried (MgSO₄) and evaporated. Purification was by chromatography eluting with 5-10% ethyl acetate in isohexane. Yield 84.4g.
MS:APCI (-ve) 253 (M-1,100%)

### ii) 5-(3-Chlorophenyl)-2-hydroxyimino-3-oxo-pentanoic acid, ethyl ester

A solution of sodium nitrite (12.6g) in water (25ml) was added to a stirred solution of the product from step (i) (42.2g) in acetic acid (50ml) at <30°C. The reaction mixture was stirred at room temperature for 30 min. Water (75ml) was added and the mixture was stirred for 2 hours and extracted with diethyl ether. The combined extracts were washed with water and saturated aqueous sodium bicarbonate solution. The organic layer was collected, dried (MgSO₄) and solvent evaporated under reduced pressure to give the subtitle product.
The product was used directly in the next step without further purification.
MS: APCI (-ve) 282 (M-1,100%)

### iii) 2-Acetylamino-5-(3-chlorophenyl)-3-oxo-pentanoic acid, ethyl ester

To a stirred solution of the product from step (ii) in acetic acid (125ml) and acetic anhydride (40ml) was added zinc dust (45g) portionwise at a rate to maintain the internal temperature <40°C. The mixture was stirred for 30 min and water (125ml) was added at such a rate as to maintain the temperature at around 40°C. The mixture was stirred at room temperature for 2 hours and filtered. The mother liquor was extracted with dichloromethane and further washed with water. The organic layer was collected, dried (MgSO₄) and solvent evaporated under reduced pressure to give the subtitle product.
Yield 45.5g.
MS:APCI(-ve) 310 (M-1,100%)

### iv) 5-[2-(3-Chlorophenyl)ethyl]-2-methyloxazole-4-carboxylic acid, ethyl ester

Thionyl chloride (180ml) was added to the product of step (iii) (45.5g) at 0°C. The mixture was stirred at room temperature for 2 hours and heated at reflux for 40 min. The residue after evaporation under reduced pressure was dissolved in ethyl acetate and washed with saturated sodium bicarbonate solution. The organic phase was dried (MgSO₄) and solvent evaporated under reduced pressure. Purification was by chromatography eluting with 1-2% ethanol in dichloromethane to give the subtitle product as an orange oil. Yield 24.56g.
MS: APCI (+ve) 294 (M+1, 100%)

### v) 5-[2-(3-Chlorophenyl)ethyl]-2-methyloxazole-4-carboxylic acid

A solution of the product from step (iv) (24.36g) and potassium hydroxide (9g) in ethanol (250ml) and water (20ml) was stirred at room temperature for 4 hours and concentrated under reduced pressure. The residue was suspended in water, acidified with 2N HCl and extracted with ethyl acetate. The combined extracts were washed with water and saturated brine. The organic layer was collected, dried (MgSO₄) and solvent evaporated to give the subtitle product. Yield 20g.
MS: APCI(+ve) 266 (M+1), 248 (100%)

### vi) 7-Chloro-9,10-dihydro-2-methyl-4H-benzo[5,6]cyclohepta[1,2-d]oxazol-4-one

A suspension of the product from step (v) (19.5g) in benzene (150ml) and thionyl chloride (50ml) was heated at reflux for 1 hour. The resultant solution was evaporated to dryness and the residue was dissolved in 1,2-dichloroethane (150ml) and treated with aluminium chloride (50g). The reaction mixture was heated at reflux for 2 hours, allowed to cool and poured onto ice. The resultant suspension was repeatedly extracted with dichloromethane. The combined organic extracts were dried (MgSO₄) and solvent evaporated under reduced pressure. Purification was by chromatography eluting with 1% ethanol in dichloromethane. Yield 5g.
MS: APCI (+ve) 248 (M+1, 100%)

### vii) (±)-5-(7-Chloro-9,10-dihydro-4-hydroxy-2-methyl-4H-benzo[5,6]cyclohepta[1,2-d]oxazol-4-yl]-2,4(1H,3H)-pyrimidinedione

*n*-BuLi (2.5M in hexanes, 8.4ml) was added to a solution of the product from example 1 step (vi) (5.76g) in tetrahydrofuran (50ml) at -78°C. The reaction mixture was stirred at -65°C for 30min. A solution of the product from step (vi) (4.8g) in tetrahydrofuran (100ml) was added and the mixture was stirred at -70°C for 1.5 hours and room temperature for 1 hour. The reaction mixture was quenched with aqueous ammonium chloride and partitioned between ethyl acetate and water. The organic phase was washed with water and saturated brine, dried (MgSO₄) and solvent evaporated. The residue was dissolved in glacial acetic acid and the solution stirred for 16h at room temperature. The solvent was evaporated and the residue was triturated with ethyl acetate/isohexane. The solid was filtered off and the filtrate purified by chromatography eluting with 5-10% methanol in dichloromethane to give a pale yellow solid. Yield 2.05g.
MS: APCI(-ve) 358 (M-1, 100%)

### viii) (±)-5-[7-Chloro-9,10-dihydro-4-hydroxy-2-methyl-4H-benzo[5,6]cyclohepta[1,2-d]oxazol-4-yl]-1-methyl-2,4(1H,3H)-pyrimidinedione

To a stirred solution of the product from step (vii) (2.0g) in tetrahydrofuran (20ml) at 0°C was added sodium hydride (60% dispersion by wt, 0.22g). The mixture was stirred at 0°C for 40min and treated with methyl iodide (0.35ml). The mixture was further stirred at 0°C for 2 hours and room temperature for 24 hours then partitioned between ethyl acetate and water. The organic phase was washed with water and saturated brine, dried (MgSO₄) and solvent evaporated. Purification was by chromatography eluting with 10% ethanol in dichloromethane to give the product as a pale yellow solid. Yield 0.92g.
1H NMR: δ (CDCl₃) 8.17 (br s, 1H), 7.84 (d,1H), 7.29 (dd,1H), 7.21 (d,1H), 6.94 (s,1H), 4.96 (s,1H), 3.29 (s,3H), 3.14-2.73 (m, 4H), 2.42 (s,3H).

### ix) (±)-5-[7-Chloro-2-methyl-4H-benzo[5,6]cyclohepta[1,2-d]oxazol-4-yl]-1-methyl-2,4(1H,3H)-pyrimidinedione

A solution of the product from step (viii) (0.85g) in trifluoroacetic acid (20ml) was heated at reflux for 8 days. The solvent was evaporated and the residue triturated with ethyl acetate/ isohexane to give a solid. Yield 0.5g.
1H NMR: δ (DMSO) 11.29 (s,1H), 7.55-7.52 (m,2H), 7.43 (dd,1H), 6.90 (s,2H), 6.89 (s,1H), 5.23 (s,1H), 3.12 (s,3H), 2.44 (s,3H)

### Example 26

### (±)-5-[7-Chloro-2-methyl-4H-benzo[5,6]cyclohepta[1,2-d]oxazol-4-yl]-1-methyl-3,4-dihydro-4-thioxo-2(1H)-pyrimidinone

The title compound was prepared from the product of example 25 step (ix) (0.48g) according to the method of example 2 step (ii). Purification was by HPLC (SFC;0-45% methanol).
Yield 0.08g.
MS: APCI(+ve) 372 (M+1, 100%)
1H NMR: δ (DMSO) 12.72 (s,1 H), 7.76 (d,1H), 7.55 (d,1H), 7.42 (dd,1H), 7.23 (s,1H), 6.94 (q,2H), 5.96 (s,1H), 3.20 (s,3H), 2.43 (s,3H).

### Example 27

### (S)- 5-[7-Chloro-2-methyl-4H-benzo[5,6]cyclohepta[1,2-d]oxazol-4-yl]-1-methyl-3,4-dihydro-4-thioxo-2(1H)-pyrimidinone

The title compound was obtained from the product of example 26 by resolution of the racemate on HPLC (SFC; Chiralpak® AD column, 30-45% isopropanol in liquid CO₂). The title compound was the first enantiomer to be eluted off the column.
MS: APCI(+ve) 372 (M+1, 100%)
1H NMR: δ (DMSO) 12.72 (s,1H), 7.76 (d,1H), 7.55 (d,1H), 7.42 (dd,1H), 7.23 (s,1H), 6.94 (q,2H), 5.96 (s,1H), 3.20 (s,3H), 2.43 (s,3H).

### Example 28

### (±)-5-(7-Chloro-2-(2-(imidazol-1-yl)ethoxy)-4H-benzo[5,6]cyclohepta[1,2-d]thiazol-4-yl)-1-methyl-3,4-dihydro-4-thioxo-2(1H)-pyrimidinone

### i) 5-(7-Chloro-2-(2-(imidazol-1-yl)ethoxy)-4H-benzo[5,6]cyclohepta[1,2-d]thiazol-4-yl)-1-methyl-2,4(1H,3H)-pyrimidinedione

The product from example 44 step (viii) (0.2g), 1-(2-hydroxyethyl)imidazole (0.114g) and 60% sodium hydride (0.048g) in *N*,*N*-dimethylformamide (10ml) was stirred under nitrogen at room temperature for 16hours. The reaction mixture was partitioned between ethyl acetate and water. The organic phase was collected, dried (MgSO₄) and evaporated under reduced pressure to leave a yellow oil. Purification was by chromatography eluting with 10:1 dichloromethane/methanol to give the subtitle product as a yellow oil.
MS: APCI(+ve): 468(M+1, 100%), APCI(-ve):466(M-1, 100%)

### ii) (±)-5-(7-Chloro-2-(2-(imidazol-1-yl)ethoxy)-4H-benzo[5,6]cyclohepta[1,2-d]thiazol-4-yl)-1-methyl-3,4-dihydro-4-thioxo-2(1H)-pyrimidinone

The title product was prepared from the product of step (i) (0.15g) according to the method of example 70. Purification was by chromatography eluting with 9:1 ethyl acetate/methanol to give a yellow solid. Yield: 0.055g
MS: APCI(+ve):485(M+1, 100%)
1HNMR: δ (DMSO): 12.69(brs, 1H), 7.68-6.85(m, 8H), 5.77-5.75(s, 1H), 4.69-4.66(t, 2H), 4,41-4.39(m, 2H), 3.29(s, 3H)
MP: 190-193 °C.

### Example 29

### (±)-1-[(1-Methyl)-1H-imidazol-2-ylmethyl]-5-(2,6-dimethyl-4H-benzo[5,6]cyclohepta[1,2-d]oxazol-4-yl)-3,4-dihydro-4-thioxo-2(1H)-pyrimidinone

### i) (±)-1-[(1-Methyl)-1H-imidazol-2-ylmethyl]-5-(9,10-dihydro-4-hydroxy-2,6-dimethyl-4H-benzo[5,6]cyclohepta[1,2-d]oxazol-4-yl)-2,4(1H,3H)-pyrimidinedione

A solution of the product from example 17 step (viii) (1.39g) in dry *N,N*-dimethylformamide (20ml) was treated with sodium hydride(0.328g of a 60% dispersion by wt.) and stirred for 0.5h. 2-Chloromethyl-1-methyl-imidazole hydrochloride (J. Chem. Soc., Perkinl., 1993, 2298) (0.684g) was added and stirring continued for a further 2 hours before being partitioned between ethyl acetate and saturated brine. The aqueous layer was continuously extracted with chloroform for 24 hours. The continuous extracts were evaporated and the residue triturated with 1:1 diethyl ether:isohexane to give a pale yellow solid. Yield 0.60g
MS: APCI(-ve):432 (M-1, 100%)

### ii) (±)-1-[(1-Methyl)-1H-imidazol-2-ylmethyl]-5-(2,6-dimethyl-4H-benzo[5,6]cyclohepta[1,2-d]oxazol-4-yl)-2,4(1H,3H)-pyrimidinedione

The subtitle compound was prepared from the product of step (i) (0.60g) according to the method of example 15 step (ii). Purification was by flash column chromatography eluting with 5% methanol in dichloromethane (+0.1% triethylamine). Yield 0.15g
MS: APCI(+ve):416 (M-1, 100%)

### iii) (±)-1-[(1-Methyl)-1H-imidazol-2-ylmethyl]-5-(2,6-dimethyl-4H-benzo[5,6]cyclohepta[1,2-d]oxazol-4-yl)-3,4-dihydro-4-thioxo-2(1H)-pyrimidinone

The title compound was prepared from the product from step (ii) (0.163g) according to the method of example 2 step (ii). Purification was by flash column chromatography eluting with 5% methanol in dichloromethane. Yield 0.02g
MS: APCI(+ve):432 (M+1, 100%)
1H NMR: δ (DMSO) 12.77(s,1H), 7.51(s,1H), 7.29(d,1H), 7.18(s,1H), 7,14(s,1H), 7.09(d,1H), 6.88(s,1H), 6.76(d,1H), 6.61(d,1H), 5.93(s,1H), 4.94(d,1H), 4.82(d,1H), 3.54(s,3H), 2.40(s,3H), 2.29(s,3H)

### Example 30

### (±)-5-(2,7-Dimethyl-4H-benzo[5,6]cyclohepta[1,2-d]thiazol-4-yl)-1-(2-(N-pyrrolidinyl)ethyl)-3,4-dihydro-4-thioxo-2(1H)-pyrimidinone

### i) (±)-5-(2,7-Dimethyl-4H-benzo[5,6]cyclohepta[1,2-d]thiazol-4-yl)-1-(2-(N-pyrrolidinyl)ethyl)-2,4(1H,3H)-pyrimidinedione

The product from example 18 step (ii) (1.44g) was dissolved in dry *N,N*-dimethylformamide (20ml) and treated cautiously with sodium hydride(60% dispersion by wt, 0.5g) under nitrogen. After 10min 1-(2-chloroethyl)pyrrolidine hydrochloride (0.8g) was added and the whole heated at 50 °C for 16h. The reaction mixture was partitioned between water and ethyl acetate. The organic layer was collected, dried over magnesium sulphate and solvent evaporated under reduced pressure. Purification was by flash chromatography eluting with 9:1 dichloromethane/methanol. The product obtained was dissolved in acetic acid (10ml) and heated at 100 °C for 2h. The solvent was removed under reduced pressure. Purification was by flash chromatography eluting with dichloromethane/methanol mixtures to give the subtitle product as pale yellow foam. Yield: 0.1g
MS: APCI+ve: (435 (M+1), 100%)

### ii) (±)-5-(2,7-Dimethyl-4H-benzo[5,6]cyclohepta[1,2-d]thiazol-4-yl)-1-(2-(N-pyrrolidinyl)ethyl)-3,4-dihydro-4-thioxo-2(1H)-pyrimidinone

The title product was prepared from the product of step (i) according to the method of example 2 step (ii). Purification was by flash chromatography eluting with dichloromethane/methanol/triethylamine mixtures followed by SFC chromatography eluting with a 20-45% CO₂/methanol gradient. Yield. 0.011g
MS: APCI+ve: (451 (M+1), 100%), APCI(-ve): (449(M-1, 100%)
NMR: δ (CDCl₃): 9.35 (bs, 1H), 7.6 (d, 1H), 7.3-7.2 (m, 2H), 7.19 (d, 1H), 7.0-6.85 (dd, 2H), 6.21 (s, 1H), 3.8-3.6 (br hump, 2H), 2.7 (bs, 1H), 2.5 (bs, 2H), 1.8-1.4 (b multiplet, 4H), 2.7 (s, 3H), 2.30 (s, 3H)
MP: 198 °C

### Example 31

### (±)-5-(2-Ethyl-7-methyl-4H-benzo[5,6]cyclohepta[1,2-d]thiazol-4-yl)-3,4-dihydro-4-thioxo-2(1H)-pyrimidinone

### i) 2-Ethyl-9,10-dihydro-7-methyl-4H-benzo[5,6]cyclohepta[1,2-d]thiazol-4-one

Hydrogen sulphide was passed through a suspension of potassium tert-butoxide (9.31g) in *N,N*-dimethylformamide (80ml) at 5 "C until the mixture was saturated. The product from example 1 step (v) (5.0g) was added and the reaction mixture stirred at room temperature for 2h. The mixture was poured onto ice, acidified to pH3 and extracted with ethyl acetate. The organic phase was washed with water, dried (MgSO₄) and evaporated under reduced pressure. Purification was by chromatography eluting with 40-50% ethyl acetate in isohexane. Yield 3.15g.
MS: APCI(+ve) 258 (M+1, 100%)

### ii) 2-Ethyl-7-methyl-4H-benzo[5,6]cyclohepta[1,2-d]thiazol-4-one

A solution of the product from step (i) (3.14g) and N-bromosuccinimide (2.18g) in benzene (40ml) was irradiated with a 500 Watt halogen lamp for 2h. The reaction mixture was partitioned between dichloromethane and water. The organic phase was further washed with water, collected, dried (MgSO₄) and solvent evaporated under reduced pressure. The residue was dissolved in dichloromethane (25ml) and triethylamine (10ml) added. After stirring for 16h the solvent/reagent were evaporated under reduced pressure. Purification was by chromatography eluting with 30-40% ethyl acetate in isohexane.
Yield 0.88g.
MS: APCI(+ve) 256 (M+1, 100%)

### iii) (±)-2-Ethyl-7-methyl-4H-benzo[5,6]cyclohepta[1,2-d]thiazol-4-ol

Sodium borohydride (78mg) was added to a stirred solution of the product from step (ii) (0.87g) in dichloromethane (4ml) and ethanol (16ml) at 5°C. The reaction mixture was stirred for 2.5h, warmed to room temperature and left for 2h before a further 200mg of sodium borohydride was added. The reaction mixture was quenched with 1M sodium hydroxide solution then partitioned between dichloromethane and water. The organic phase was washed with water, dried(MgSO₄) and evaporated under reduced pressure.
Yield 0.845g.
MS: APCI(+ve) 258 (M+1)

### iv) (±)-5-(2-Ethyl-7-methyl-4H-benzo[5,6]cyclohepta[1,2-d]thiazol-4-yl)-2,4(1H,3H)-pyrimidinedione

A solution of the product from step (iii) (785mg) in acetic acid (5ml) was added to a suspension of uracil (0.673g) in acetic acid (25ml) at 90°C over 3min. After 1.5h the solvent was removed under reduced pressure and the residue partitioned between ethyl acetate and water. The organic phase was washed with water, dried(MgSO₄) and evaporated under reduced pressure. Purification was by chromatography eluting with ethyl acetate. Yield 0.408g.
MS: APCI(+ve): 352 (M+1, 100%)

### v) (±)-5-(2-Ethyl-7-methyl-4H-benzo[5,6]cyclohepta[1,2-d]thiazol-4-yl)-3,4-dihydro-4-thioxo-2(1H)-pyrimidinone

The title compound was prepared from the product of step (iv) (0.385g) by the method of example 2 step (ii). Purification was by chromatography eluting with 70% ethyl acetate in isohexane. Yield 0.268g.
MS: APCI(+ve) 368 (M+1, 100%)
1H NMR: δ (DMSO) 12.47(s,1H), 11.40(d,1H), 7.50(d,1H), 7.27(d,1H), 7.22(s,1H), 7.19(d,1H), 7.08-6.66 (AB, 2H), 6.04(s,1H), 2.94(q, 2H), 2.28(s,3H), 1.27(t,3H)

### Example 32

### (±)5-(2-Ethyl-7-methyl-4H-benzo[5,6]cyclohepta[1,2-d]thiazol-4-yl)-1-(N-methyl)imidazol-2-ylmethyl)-3,4-dihydro-4-thioxo-2(1H)-pyrimidinone

### i) (±)-5-(2-Ethyl-7-methyl-4H-benzo[5,6]cyclohepta[1,2-d]thiazol-4-yl)-4-methylthio-2(1H)-pyrimidinone

Methyl iodide (0.044ml) was added to a stirred solution of the product from example 31 step (v) (0.250g) and sodium bicarbonate (0.057g) in ethanol (5ml) and water (3ml). After 3h the reaction mixture was partitioned between ethyl acetate and water. The organic phase was washed with water, dried(MgSO₄) and evaporated under reduced pressure. Purification was by trituration with ethyl acetate and isohexane. Yield 0.140g.
MS: APCI(+ve): 382 (M+1, 100%)

### ii) (±)-5-(2-Ethyl-7-methyl-4H-benzo[5,6]cyclohepta[1,2-d]thiazol-4-yl)-1-(N-methyl)imidazol-2-ylmethyl)-3,4-dihydro-4-thioxo-2(1H)-pyrimidinone

Sodium hydride (60% dispersion by wt. 25mg) was added to a stirred solution of the product from step (i) in *N*,*N*-dimethylformamide (3ml) at room temperature. After 0.25h a solution of 2-chloromethyl-1-methyl-imidazole hydrochloride (J. Chem. Soc. Perkin1., 1993, 2298) (41mg) in *N,N*-dimethylformamide (1ml) was added and the mixture stirred for 3h at room temperature. Pyridine (5ml), diisopropylethylamine (1ml) and triethylamine (1ml) were added and hydrogen sulphide gas bubbled through the mixture for 0.5h. The reaction mixture was stirred at room temperature for a further 16h and the solvent evaporated under reduced pressure. Purification was by chromatography eluting with triethylamine-methanol-dichloromethane (2:50:950), followed by HPLC (SFC; 20-45% 0.1% diethylamine in methanol), followed by a C-18 Sep-Pak column eluting with 50-60% methanol-water with 0.1% 0.88 ammonia). Yield 0.01 1g.
MS: APCI(+ve): 462 (M+1, 100%)
1H NMR: δ (DMSO) 9.52(s, 1H), 7.64(d,1H), 7.20(s,1H), 7.18(d,1H), 7.08(s,1H), 7.06(d,1H), 6.91(1H,d), 6.75-6.67 (AB, 2H), 6,19(s,1H), 4.99(d,1H), 4.84(d,1H), 3.58(s,3H), 2.96(q, 2H), 2.31(s,3H), 1.34(t,3H)

### Example 33

### (±)-5-(2-Ethyl-7-methyl-4H-benzo[5,6]cyclohepta[1,2-d]thiazol-4-yl)-1-(2-(N,N-dimethyl)ethyl)-3,4-dihydro-4-thioxo-2(1H)-pyrimidinone

Sodium hydride (60% dispersion by wt. 83mg) was added to a stirred solution of the product from example 32 step (i) (318mg) in dry N,N-dimethylformamide (10ml) at room temperature under nitrogen. After 20min 2-dimethylaminoethyl chloride hydrochloride (130mg) was added with stirring at room temperature for 1h. The temperature was then raised to 80 °C for 2h. A further 40mg of 60% dispersion of sodium hydride and 2-dimethylaminoethyl chloride hydrochloride (60mg) was added and heating continued at 80 °C for a further 1h. The cooled mixture was then treated with NaSH.hydrate (200mg) and the mixture heated at 80 °C for 1.5h. The solvent was removed under reduced pressure and the residue purified by a C18 Sep-Pak column eluting with 30-70% methanol/water (containing 0.2% aq. Ammonia) to leave a solid residue after lyophilisation. Further purification was performed by SFC chromatography eluting with 8 to 30% gradient of methanol (containing 0.1% diethylamine)/CO₂ to give the give the title product as a yellow solid after trituration with isohexane/ethyl acetate and filtration. Yield: 51mg MS:APCI(+ve); 439 (M+1, 100%)
1HNMR δ (DMSO): 12.62 (s, 1H), 7.58 (s, 1H), 7.53 (d, 1H), 7.22 (s, 1H), 7.19 (d, 1H), 7.08-6.99 (d, 2H), 6.03 (s, 1H), 3.80-3.75 (m, 2H), 2.94 (m, 2H), 2.42 (t, 2H), 2.28 (s, 3H), 2.13 (s, 6H), 1.29 (t, 3H)
MP: 155 °C

### Example 34

### (±)-5-[2-methyl-4H-benzo[5,6]cyclohepta[1,2-d]oxazol-4-yl)-1-methyl-3,4-dihydro-4-thioxo-2(1H)-pyrimidinone

### i) (±)-5-(9,10-Dihydro-4-hydroxy-2-methyl-4H-benzo[5,6]cyclohepta[1,2-d]oxazol-4-yl)-2,4(1H,3H)-pyrimidinedione

The subtitle compound was prepared from 9,10-dihydro-2-methyl-4*H*-benzo[5,6]cyclohepta[1,2-*d*]oxazol-4-one (2.0g) (J. Med. Chem.,1974, **17**, 1316) according to the method of example 14 step (vi). Yield 2.0g.
MS: APCI(-ve): 324 (M-1, 100%)

### ii) (±)-5-(9,10-Dihydro-4-hydroxy-2-methyl-4H-benzo[5,6]cyclohepta[1,2-d]oxazol-4-yl)-1-methyl-2,4(1H,3H)-pyrimidinedione

The subtitled compound was prepared from the product from step (i) (1.9g) according to the method of example 21, step (vi). Purification was by flash column chromatography on silica eluting with 3 to 8% ethanol in dichloromethane to give the subtitle product.
Yield: 0.29g
MS: APCI(-ve): 338 (M-1, 100%)

### iii) (±)-5-(2-Methyl-4H-benzo[5,6]cyclohepta[1,2-d]oxazol-4-yl)-1-methyl-2,4(1H,3H)-pyrimidinedione

A stirred solution of the product from step (ii) (280mg) in trifluoroacetic acid (5ml), was heated under reflux for 96h and evaporated. The residue was slurried in toluene and evaporated (twice). Purification was by flash chromatography eluting 50% acetone in isohexane, followed by a second column eluting with 4% ethanol in dichloromethane to give a yellow solid. Yield 75mg. Used directly in the next step.

### iv) (±)-5-[2-methyl-4H-benzo[5,6]cyclohepta[1,2-d]oxazol-4-yl)-1-methyl-3,4-dihydro-4-thioxo-2(1H)-pyrimidinone

A mixture of the product from step (iii), (0.075g) and Lawesson's reagent (0.094g) in 1,4-dioxane (5ml) under nitrogen was heated at reflux 16h. The reaction mixture was partitioned between ethyl acetate and saturated brine. The organic phase was collected, dried over magnesium sulphate and solvent evaporated under reduced pressure. Purification was by flash chromatogaphy eluting with 0 to 2% ethanol in dichloromethane followed by a second column eluting with 40% ethyl acetate in toluene to give the title compound as a yellow solid. Yield: 0.037g
MS: APCI(+ve): 338 (M+1, 100%)
1H NMR: δ(DMSO) 2.42(s, 3H), 3.19(s, 3H), 6.01(s, 3H), 6.86(d, 1H), 6.98(d, 1H), 7.24(s, 1H), 7.28(d of t, 1H), 7.37(d of t, 1H), 7.44(d of d, 1H), 7.75(d, 1H), 12.96(s, 1H).

### Example 35

### (±)-1-Methyl-5-(2-methyl-7-ethyl-4H-benzo[5,6]cyclohepta[1,2-d]thiazol-4-yl)-3,4-dihydro-4-thioxo-2(1H)-pyrimidinone

### 3-Ethylphenylmethanol

*n*-Butyllithium (34ml of a 2.5M solution in hexanes) was added to a solution of 1-bromo-3-ethylbenzene (15g) in tetrahydrofuran (320ml) at -78°C. After 1 hour *N,N*-dimethylformamide (15ml) was added. The mixture was stirred at -78°C for 1 hour and allowed to warm to room temperature. The mixture was quenched with aqueous ammonium chloride and partitioned between ethyl acetate and water. The organic phase was dried (MgSO₄) and evaporated. The residue was dissolved in methanol (250ml) and treated with sodium borohydride (1.51g) portionwise. The mixture was stirred at room temperature for 16 hours, quenched with 2M HCl (100ml) and stirred for 4 hours. The mixture was concentrated under reduced pressure and the residue partitioned between ethyl acetate and 2M HCl. The organic phase was dried (MgSO₄), evaporated and the residue purified by chromatography eluting with 5-10% ethyl acetate in isohexane. Yield: 7.6g.
MS: GC-MS: 136 (M⁺) 97%

### ii) 1-Bromomethyl-3-ethylbenzene

A mixture of the product from step (i) (29 g) and phosphorus tribromide (6.7 ml) in toluene (300 ml) was heated at reflux for 4 hours. After cooling to room temperature, the toluene layer was decanted from the brown phosphorus residues and concentrated under reduced pressure. The crude product was dissolved in diethyl ether (300 ml), washed with water, saturated aqueous sodium bicarbonate solution, and saturated brine. The organic phase was collected, dried (MgSO₄) and evaporated under reduced pressure. Yield: 39g
1H NMR: δ (CDCl₃) 7.2 (m, 4H), 4.49 (s, 2H), 2.65 (q, 2H), 1.22 (t, 3H).

### iii) 5-(3-Ethylphenyl)-3-oxopentanoic acid, ethyl ester

To a stirred solution of ethyl acetoacetate (25 g) in tetrahydrofuran (800 ml) at 0°C was added LDA (2.0M solution in heptanes/tetrahydrofuran/ethylbenzene) (194 ml). After 40 min the product from step (ii) (39 g) was added dropwise and the mixture was stirred at 0°C for 1 hour. The reaction mixture was acidified with 2N HCl and the organic phase was concentrated under reduced pressure. The residue was dissolved in diethyl ether and washed with water and saturated brine. The organic phase was collected, dried (MgSO₄) and evaporated under reduced pressure. Purification was by chromatography eluting with 5-10% ethyl acetate in isohexane. Yield: 20.6g.
MS:APCI (-ve) 247 (M-1,100%)

### iv) 5-(3-Ethylphenyl)-2-hydroxyimino-3-oxo-pentanoic acid, ethyl ester

A solution of sodium nitrite (6.3 g) in water (10 ml) was added to a stirred solution of the product from step (iii) (20.6 g) in acetic acid (30 ml) at <30°C. The reaction mixture was stirred at room temperature for 18 hours. Water (150 ml) was added and the mixture was stirred for 2 hours and extracted with diethyl ether. The combined extracts were washed with water and saturated aqueous sodium bicarbonate solution. The organic phase was collected, dried (MgSO₄) and solvent evaporated under reduced pressure.Yield:20.9g
MS: APCI (-ve) 276 (M-1,100%)

### v) 2-Acetylamino-5-(3-ethylphenyl)-3-oxo-pentanoic acid, ethyl ester

To a stirred solution of the product from step (iv) (20.9g) in acetic acid (100 ml) and acetic anhydride (25 ml) was added zinc dust (30g) portionwise at a rate to maintain the internal temperature <40°C. The mixture was stirred for 18 hours and water (125 ml) was added at such a rate as to maintain the temperature at around 40°C. The mixture was stirred at room temperature for 1 hour and filtered. The filtrate was extracted with dichloromethane and the extract was washed with water and saturated aqueous sodium bicarbonate solution. The organic phase was collected, dried (MgSO₄) and evaporated under reduced pressure to give a yellow oil. Yield 21.1g.
MS:APCI(+ve) 306 (M+1, 50%)

### vi) 5-[2-(3-Ethylphenyl)ethyl]-2-methyloxazole-4-carboxylic acid, ethyl ester

Thionyl chloride (80 ml) was added to the product from step (v) (21.0 g) at 0°C. The mixture was stirred at room temperature for 1 hour and heated at reflux for 1 hour. The residue after evaporation was dissolved in ethyl acetate and washed with saturated sodium bicarbonate solution. The organic phase was dried (MgSO,) and evaporated. Yield: 19.2g.
MS: APCI (+ve) 288 (M+1, 100%)

### vii) 5-[2-(3-Ethylphenyl)ethyl]-2-methyloxazole-4-carboxylic acid

A solution of the product from step (vi) (19.0 g) and lithium hydroxide (5.6 g) in tetrahydrofuran (200 ml) and water (100 ml) was heated at reflux for 4 hours and concentrated under reduced pressure. The residue was suspended in water, acidified with 2N HCl and extracted with ethyl acetate. The combined extracts were washed with water and saturated brine. The organic phase was collected, dried (MgSO₄) and solvent evaporated to give a brown solid. Purification was by flash chromatography, eluting with 50% ethyl acetate in dichloromethane with 0.5% acetic acid. Yield 9.7 g.
MS: APCI(+ve) 260 (M+1) (100%)

### viii) 7-Ethyl-9,10-dihydro-2-methyl-4H-benzo[5,6]cyclohepta[1,2-d]oxazole-4-one

A solution of the product from step (vii) (9.5 g) in thionyl chloride (100 ml) was stirred at room temperature for 4 days. After removal of solvent under reduced pressure, the residue was dissolved in 1,2-dichloroethane (200 ml) and treated with aluminium chloride (23 g). The reaction mixture was heated at reflux for 1 hour and allowed to cool. The resultant suspension was partitioned between 10% hydrochloric acid and dichloromethane. The combined extracts were evaporated, triturated with acetonitrile, and filtered through a plug of celite. Evaporation afforded a residue which was purified by chromatography eluting with 50-100% ethyl acetate in isohexane. Yield 4.8g.
MS: APCI (+ve) 242 (M+1, 100%)

### ix) 7-Ethyl-9,10-dihydro-2-methyl-4H-benzo[5,6]cyclohepta[1,2-d]thiazole-4-one

Hydrogen sulphide gas was bubbled through a solution of potassium *tert*-butoxide (17.4 g) in dimethylformamide (75 ml) at 0°C, maintaining the temperature below 5°C, until a permanent blue colour appeared. The product from step (viii) was added to this solution, and the reaction mixture stirred at room temperature for 3 hours, before it was poured onto ice. The mixture was acidified to pH 4 with 10% hydrochloric acid and extracted with ethyl acetate. The combined extracts were dried (MgSO₄) and evaporated to give a brown solid. Purification was by chromatography, eluting with 50-100% ethyl acetate in isohexane.
Yield: 2.5 g.
MS: APCI (+ve) 258 (M+1, 100%)

### x) 7-(1-Bromoethyl)-2-methyl-4H-benzo[5,6]cyclohepta[1,2-d]thiazole-4-one

A stirred mixture of N-bromosuccinimide (2.1 g) and the product from step (ix) (2.0 g) in ethyl acetate (100 ml) was irradiated with a 500 Watt lamp for 3 hours at room temperature. The solvent was removed under reduced pressure and the residue redissolved in dichloromethane (100 ml). Triethylamine (0.82 ml) was added and the mixture stirred at room temperature for 2 hours. Evaporation of solvent under reduced pressure gave a residue which was purified by chromatography, eluting with 20% isohexane in ethyl acetate. Yield: 0.94 g.
1H NMR: δ (CDCl₃) 8.76 (d,1H), 7.74 (m, 2H), 7.23 (s, 2H), 5.29 (q, 1H), 2.84 (s, 3H), 2.11(d,3H).

### xi) (±)-7-Ethyl-2-methyl-4H-benzo[5,6]cyclohepta[1,2-d]thiazol-4-ol

A solution of sodium borohydride (0.31 g) and the product from step (x) (1.35 g) in 1,4-dioxan (40 ml) and dimethyl sulphoxide (10 ml) was stirred at room temperature for 18 hours. The reaction was quenched with water (4 ml) before solvent was removed under reduced pressure. The residue was dissolved in ethyl acetate which was washed with saturated aqueous sodium bicarbonate and saturated brine. The organic phase was collected, dried (MgSO₄) and evaporation of solvent under reduced pressure gave a yellow oil which was purified by chromatography, eluting with 20% isohexane in ethyl acetate. Yield: 0.45 g.
1H NMR: δ (CDCl₃) 7.60 (d,1H), 7.32 (dd, 1H), 7.26 (d, 1H), 7.09 (d, 1H), 6.90 (d, 1H), 5.74 (br s, 1H), 2.69 (s, 3H), 2.68 (q, 2H), 1.24 (t, 3H).

### xii) (±)-5-[7-Ethyl-2-methyl-4H-benzo[5,6]cyclohepta[1,2-d]thiazol-4-yl]-1-methyl-2,4(1H,3H)-pyrimidinedione

1-Methyluracil (0.064 g) was added to a solution of the product from step (xi) (0.10 g) in dichloromethane (2 ml), followed by trifluoroacetic acid (1 ml). The orange solution was stirred at room temperature for 10 minutes before the solvent was removed under reduced pressure. The residue was azeotroped twice with acetonitrile, and the crude product purified by chromatography , eluting with 0-5% methanol in ethyl acetate.
MS: APCI(+ve) 366 (M+1) (100%)

### xiii) (±)-1-Methyl-5-(2-methyl-7-ethyl-4H-benzo[5,6]cyclohepta[1,2-d]thiazol-4-yl)-3,4-dihydro-4-thioxo-2(1H)-pyrimidinone

A mixture of the product of step (xii) (0.070 g) and Lawesson's reagent (0.077 g) in 1,4-dioxane (5 ml) was heated at reflux for 16 hours. The solvent was evaporated under reduced pressure. Purification was by chromatography eluting with 2% ethanol in dichloromethane to give the title compound. Yield: 0.01 g.
MS: APCI(+ve): 382 (M+1, 100%).
1H NMR: δ (CDCl₃) 9.31 (br s,1H), 7.63 (d, 1H), 7.25 (dd, 1H), 7.18 (d, 1H), 7.13 (s, 1H), 6.98 (d, 1H), 6.81 (d, 1H), 6.26 (s, 1H), 3.28 (s, 3H), 2.68 (s, 3H), 2.64 (q, 2H), 1.23 (t, 3H).
MP: 265°C.

### Example 36

### (±)-1-Methyl-5-(2-methyl-7-n-propyl-4H-benzo[5,6]cyclohepta[1,2-d]thiazol-4-yl)-3,4-dihydro-4-thioxo-2(1H)-pyrimidinone

### i) 3-n-Propylphenylmethanol

The subtitle product was prepared from 1-bromo-3-*n*-propylbenzene (81.5g) according to the method of example 35 step (i) as a pale yellow oil. Yield: 26.7g
NMR: δ (CDCl₃): 7.35 -7.10 (m, 4H), 4.67 (s, 2H), 2.60 (t, 2H), 1.7 (quin, 2H), 0.90 (t, 3H)

### ii) 1-Bromomethyl-3-n-propylbenzene

The subtitle product was prepared from the product of step (i) (26.7g) according to the method of example 35 step (ii) as a colourless oil. Yield: 29.06g. Used directly in the next step.

### iii) 5-(3-n-Propylphenyl)-3-oxopentanoic acid, ethyl ester

The subtitle product was prepared from the product of step (ii) (29g) according to the method of example 35 step (iii). Purification was by flash chromatography eluting with 5-10% ethyl acetate in *iso*-hexane. Yield: 14.88g
MS: APCI(+ve); (263 (M+1), 245 ,100%), APCI-ve; (261 (M-1), 100%)

### iv) 5-(3-n-Propylphenyl)-2-hydroxyimino-3-oxo-pentanoic acid, ethyl ester

The subtitle product was prepared from the product of step (iii) (22.84g) according to the method of example 35 step (iv). Yield: 24.27g
MS:APCI(-ve); (290 (M-1, 100%)

### v) 2-Acetylamino-5-(3-n-propylphenyl)-3-oxo-pentanoic acid, ethyl ester

The subtitle product was prepared from the product of step (iv) (24.25g) according to the method of example 35 step (v). Yield: 23.1g
MS:APCI(+ve); (320 (M+1), 230, 100%), APCI-ve; (318 (M-1), 272, 100%)

### vi) 5-[2-(3-n-Propylphenyl)ethyl]-2-methyloxazole-4-carboxylic acid, ethyl ester

The subtitle product was prepared from the product of step (v) (23.1g) according to the method of example 35 step (vi). Yield: 15.95g
MS: APCI(+ve); 302 (M+1, 100%)

### vii) 5-[2-(3-n-Propylphenyl)ethyl]-2-methyloxazole-4-carboxylic acid

The subtitle product was prepared from the product of step (vi) (15.95g) according to the method of example 35 step (vii). Yield: 14.36g
MS:APCI(+ve); 274 (M+1, 256, 100%), APCI-ve; 272 (M-1, 100%)

### viii) 9,10-Dihydro-2-methyl-7-n-propyl-4H-benzo[5,6]cyclohepta[1,2-d]oxazol-4-one

The subtitle product was prepared from the product of step (vii) (13.72g) according to the method of example 35 step (viii). Yield: 6.79g
MS: APCI(+ve); (256 (M+1, 100%)

### ix) 9,10-Dihydro-2-methyl-7-n-propyl-4H-benzo[5,6]cyclohepta[1,2-d]thiazol-4-one

The subtitle product was prepared from the product of step (viii) (6.78g) according to the method of example 35 step (ix). Yield: 3.38g
MS: APCI(+ve); 272 (M+1, 100%)

### x) 2-Methyl-7-n-propyl-4H-benzo[5,6]cyclohepta[1,2-d]thiazol-4-one

The product from step (ix) (2.8g) and *N*-bromosuccinamide were dissolved in ethyl acetate (30ml) and irradiated with a 500 Watt halogen lamp at reflux for 2h. The solvent was evaporated under reduced pressure and the solid residue dissolved in dichloromethane (30ml) and treated with triethylamine (10ml). After 2h the solvents were removed under reduced pressure and the residue partioned between water and ethyl acetate. The organic layer was collected and dried over MgSO₄. The solvent was evaporated under reduced pressure and the product purified by flash chromatography. Yield: 3.1g
MS: APCI(+ve); 272 (M+1,100%)

### xi) (±)-2-Methyl-7-n-propyl-4H-benzo[5,6]cyclohepta[1,2-d]thiazol-4-ol

To a solution of the product from step (x) (3.1g) in dry dimethylsulphoxide (30ml) and dry 1,4-dioxane (90ml) was added sodium borohydride (0.7g). After stirring at room temperature for 24h the reaction mixture was quenched with water and extracted with ethyl acetate. The organic layer was washed with saturated sodium bicarbonate solution, then brine. The organic layer was collected and dried over magnesium sulphate and solvent removed under reduced pressure to leave a pale yellow gum. Yield: 1.4g
MS: APCI(+ve); 254 (M-17, 100%)

### xii) (±)-1-Methyl-5-(2-methyl-7-n-propyl-4H-benzo[5,6]cyclohepta[1,2-d]thiazol-4-yl)-2,4-(1H,3H)-pyrimidinedione

A mixture of the 1-methyluracil (0.5g) and the product from step (xi) in acetic acid (50ml) was heated at reflux 2h. The solvent was removed under reduced pressure and the residue partitioned between ethyl acetate/water. The organic layer collected and dried over MgSO₄. The solvent evaporated and the crude product purified by flash chromatography eluting with ethyl acetate. Yield: 0.06g
MS: APCI+ve; 380 (M+1, 100%)

### xiii) (±)-1-Methyl-5-(2-methyl-7-n-propyl-4H-benzo[5,6]cyclohepta[1,2-d]thiazol-4-yl)-3,4-dihydro-4-thioxo-2(1H)-pyrimidinone

The title product was prepared from the product of step (xii) according to the method of example 35 step (xiii). Purification was by flash chromatography eluting with 60/40 toluene/ethyl acetate. Yield: 0.03g
MS: APCI(+ve); 396 (M+1, 100%), APCI-Ve; 394 (M-1), 100%)
1HNMR δ (CDCl₃): 9.3 (bs, 1H), 7.64 (d, 1H), 7.22 (d, 1H), 7.18 (s, 1H), 7.17 (s, 1H), 7.0 - 6.80 (2xd, 2H), 6.26 (s, 1H), 3.37 (s, 3H), 2.68 (s, 3H), 2.58 (t, 2H), 1.60 (sextet, 2H), 0.96 (t, 3H)
MP: 235 °C

### Example 37

### (±)-1-Methyl-5-(2-methyl-4H-benzo[5,6]cyclohepta[1,2-d]imidazol-4-yl)-3,4-dihydro-4-thioxo-2(1H)-pyrimidinone

### i) 2-Methyl-4H-benzo[5,6]cyclohepta[1,2-d]oxazol-4-one

A stirred mixture of 9,10-dihydro-2-methyl-4*H*-benzo[5,6]cyclohepta[1,2-*d*]oxazol-4-one (9.52g) (J. Med. Chem., 1974, **17**, 1316) and *N*-bromosuccinimide (7.95g) in benzene (200ml) was irradiated with a 500 Watt halogen lamp for 2h. The mixture was then treated with *N,N*-diisopropylethylamine (15.5ml) and set at reflux for 1hour. The mixture was then partitioned between ethyl acetate and water. The organic phase was collected, dried (MgSO₄) and solvent evaporated under reduced pressure. Purification was by recrystallisation from ethyl acetate to give a buff solid. Yield:5.07g
MS: APCI(+ve); 212 (M+1, 100%)

### ii) 2-Methyl-1-(4-methoxyphenylmethyl)-4H-benzo[5,6]cyclohepta[1,2-d]imidazol-4-one

A stirred mixture of the product from step (i) (5.07g) and 4-methoxybenzylamine (12ml) was heated at 100 °C for 24h. The excess 4-methoxybenzylamine was distilled off under reduced pressure. The remaining residue was purified by recrystallisation from ethyl acetate to give the subtitle product as a pale yellow powder. Yield: 4.34g
MS: APCI(+ve); 331 (M+1, 100%)

### iii) 2-Methyl-1-(4-methoxyphenylmethyl)-4H-benzo[5,6]cyclohepta[1,2-d]imidazol-4-ol

To a stirred solution of the product from step (ii) (1g) in ethanol (40ml) and dichloromethane (20ml) was added sodium borohydride (0.28g). After 48h the solvent was evaporated (without heat) under reduced pressure and the residue partitioned between dilute aqueous sodium hydroxide solution and dichloromethane. The organic phase was collected, dried, (MgSO₄) and solvent evaporated under reduced pressure to leave a yellow foam. Yield: 1.08g
MS: APCI(+ve): 333 (M+1, 100%)

### iv) (±)-1-Methyl -5-(2-methyl-4H-benzo[5,6]cyclohepta[1,2-d]imidazol-4-yl)-2,4(1H,3H)-pyrimidinedione

A stirred solution of the product from step (iii) (0.819g) in trifluoroacetic acid (20ml) was heated at reflux for 1 h. 1-Methyluracil (0.311g) was added and the reflux continued for 5 days. The solvent was evaporated under reduced pressure. The residue was purified by chromatography eluting with 10% methanol in dichloromethane to give the subtitle product as pale green glass. Yield: 0.3g
MS: APCI(+ve): 321 (M+1,100%), APCI(-ve); 319 (M-1, 100%)

### v) (±)-1-Methyl -5-(2-methyl-4H-benzo[5,6]cyclohepta[1,2-d]imidazol-4-yl)-3,4-dihydro-4-thioxo-2(1H)-pyrimidinone

The title product was prepared from the product of step (iv) (0.1g) and Lawesson's reagent (0.378g) according to the method of example 34 step (iv). Purification was by chromatography eluting with 0-5% methanol in dichloromethane followed by reverse phase HPLC using a 40mm Novapak column eluting with 15-85% methanol in 0.1% aqueous ammonium acetate gradient over 9min. Yield: 0.015g
MS: APCI(+ve): 337 (M+1, 100%)
1HNMR δ (CDCl₃): 7.31-7.43 (m, 4H), 6.86 (d, 1H), 6.77 (s, 1H), 6.72 (d, 1H), 6.05 (s, 1H), 3.20 (s, 3H), 2.43 (s, 3H).
MP: 280 °C (dec)

### Example 38

### (±)-5-(7-Chloro-2-methyl-4H-benzo[5,6]cyclohepta[1,2-d]thiazol-4-yl)-3,4-dihydro-4-thioxo-2(1H)-pyrimidinone

### i) 7-Chloro-9,10-dihydro-2-methyl-4H-benzo[5,6]cyclohepta[1,2-d]thiazole-4-one

To a solution of the product from example 25 step (vi) (21g) in *N,N*-dimethylformamide (225ml) was added NaSH.H₂O (23.76g) and the whole heated at 80 °C for 1hour. After cooling to room temperature, 2M hydrochloric acid solution (250ml) was added. Nitrogen gas was then bubbled through the solution for 5min and the mixture then poured into water (2L) and extracted with chloroform (X2). The organic phases were collected, dried (MgSO₄) and solvent evaporated to leave a cream solid. Yield: 10.1g
MS: APCI(+ve):266/4 (M+1, 100%)

### ii) 7-Chloro-2-methyl-4H-benzo[5,6]cyclohepta[1,2-d]thiazole-4-one

A solution of the product from step (i) (6.05g) in 1,2-dichloroethane (85ml) with N-bromosuccinimide (4.1g) was irradiated with a 500 Watt halogen lamp for 1h. After cooling to room temperature, triethylamine (7ml) was added with further stirring for 0.5 hours. The solvents were evaporated under reduced pressure and the residue triturated with acetone, filtered and then further washed with water and then dried under vacuum to leave the subtitle compound as a cream solid. Yield: 3.41g
1HNMR δ (DMSO): 8.51 (d, 1H), 8.10(d, 1H), 7.80(dd, 1H), 7.64(d, 1H), 7.43(d, 1H), 2.78(s, 3H).

### iii) (±)-7-Chloro-2-methyl-4H-benzo[5,6]cyclohepta[1,2-d]thiazol-4-ol

Sodium borohydride (0.3g) was added portionwaise to a suspension of the product from step (ii) (1g) in a mixture of methanol (40ml) and dichloromethane (30ml) at room temperature. After 1hour the reaction mixture was partitioned between dichloromethane and brine. The organic phase was collected, dried (MgSO₄) and evaporated under reduced pressure to leave a beige foam. Yield: 1g. Used directly in the next step.

### iv) (±)-5-(7-Chloro-2-methyl-4H-benzo[5,6]cyclohepta[1,2-d]thiazol-4-yl)-2,4(1H,3H)-pyrimidinedione

The product from step (iii) (1g) and uracil (3g) were heated at 100 °C in acetic acid (200ml) for 16h. The solvent was evaporated under reduced pressure and the residue partitioned between ethyl acetate and brine. The organic phase was collected, dried (MgSO₄), and evaporated under reduced pressure to leave a beige solid. The solid was triturated with isohexane/ethyl acetate mixtures and filtered to leave a cream solid. Yield. 1.35g
Used directly in the next step.

### v) (±)-5-(7-Chloro-2-methyl-4H-benzo[5,6]cyclohepta[1,2-d]thiazol-4-yl)-3,4-dihydro-4-thioxo-2(1H)-pyrimidinone

The product from step (iv) (1.35g) and Lawesson's reagent (1.85g) were heated at reflux in dioxane (50ml) under nitrogen for 16h. The reaction mixture was partitioned between ethyl acetate and brine. The organic phase was collected, dried (MgSO₄), and evaporated under reduced pressure to leave a brown solid. The solid was triturated with ethyl acetate and filtered to leave a yellow solid. Yield. 0.9g
MS: APCI(+ve): 374 (M+1, 100%), APCI(-ve): 372 (M-1)
1HNMR δ (DMSO): 12.55(bs,1H), 11.51 (bd, 1H), 7.64(d, 1H), 7.62(s, 1H), 7.42(dd, 1H), 7.30(s, 1H), 7.10(dd, 2H), 6.0(s, 1H), 2.63(s, 3H)

### Example 39

### (5S, 2R)-5-(7-Chloro-2-methyl-4H-benzo[5,6]cyclohepta[1,2-d]thiazol-4-yl)-1-(2-pyrrolidinylmethyl)-3,4-dihydro-4-thioxo-2(1H)-pyrimidinone

### i) (±)-5-(7-Chloro-2-methyl-4H-benzo[5,6]cyclohepta[1,2-d]thiazol-4-yl)-4-methylthio-2(1H)-pyrimidinone

A suspension of the product from example 38, step (v) (1.8g) in ethanol (200ml) and sodium bicarbonate (0.5g) in water (15ml) was heated until a complete solution formed. After cooling to room temperature methyl iodide (0.33ml) was added and the whole stirred for 18h. Another aliquot of methyl iodide was added (0.2ml) followed by heating at 50 °C for 4h. The reaction mixture was concentrated under reduced pressure and then partitioned between dichloromethane and brine. The organic phase was collected, dried (MgSO₄) and evaporated under reduced pressure to leave a beige foam. Purification was by chromatography eluting with ethyl acetate/methanol mixtures to give the subtitle product as colourless solid. Yield 1.5g
MS:APCI(+ve);388 (M+1, 100%), APCI(-ve);386 (M-1,100%)

### ii) (R)- N-(1,1-Dimethylethoxycarbonyl)-2-[[(4-methylsulphonyl)oxy]methyl]pyrrolidine

p-Toluenesulphonyl chloride (1.04g) was added in portions to a stirred solution of (R)- N-(1,1-dimethylethoxycarbonyl)-2-pyrrolidinemethanol (1g) in pyridine (6ml) under nitrogen at 0 °C. The mixture was allowed to reach room temperature and further stirred for 20h. The reaction mixture was partitioned between diethyl ether and water. The organic phase was collected and further washed with 1N HCI, saturated copper sulphate solution, and brine. The organic phase was collected, dried (MgSO₄) and evaporated under reduced pressure to leave a colourless oil.
Yield:1.85g
MS: APCI(+ve); 297 (M-57, 100%)
1HNMR δ (CDCl₃):7.79 (d, 2H), 7.36(m, 2H), 4.10(m, 1H), 3.90 (m, 2H), 3.30(m, 2H), 1.6-2.0(m, 4H), 1.20 (2xS, 9H) rotamers

### iii) (5S,2R)-5-(7-Chloro-2-methyl-4H-benzo[5,6]cyclohepta[1,2-d]thiazol-4-yl)-1-(2-(1-(1,1-dimethylethoxycarbonyl)pyrrolidinylmethyl)-4-methylthio-2(1H)-pyrimidinone

To a solution of the product from step (i) (0.5g) in *N,N*-dimethylformamide (6ml) under nitrogen was added 60% sodium hydride (60mg). After 15min the product from step (ii) (0.5g) was added and the whole heated at 50°C for 14h. A further aliquot of the product from step (ii) (1g) was added and heating continued at 70°C for 24h. The reaction mixture was partitioned between ethyl acetate and brine. The organic phase was collected, dried (MgSO₄) and evaporated under reduced pressure to leave a yellow gum. Two diastereoisomers were separable by TLC using ethyl acetate as eluant.
Purification and isolation of the less polar diastereoisomer was by chromatography eluting with ethyl acetate to give the subtitle product as a colourless foam. Yield. 0.12g
MS: APCI(+ve); 572 (M+1, 30%), 471 (100%)

### iv) (5S,2R)-5-(7-Chloro-2-methyl-4H-benzo[5,6]cyclohepta[1,2-d]thiazol-4-yl)-1-(2-(pyrrolidinylmethyl)-4-methylthio-2(1H)-pyrimidinone trifluoroacetate.

Trifluoroacetic acid (4ml) was added to a solution of the product from step (iii) (0.12g) in dichloromethane (8ml) under nitrogen. After 1 h the volatiles were evaporated under reduced pressure to leave a pale yellow gum. Yield. 0.12g. Used directly in the next step.

### v) (5S,2R)-5-(7-Chloro-2-methyl-4H-benzo[5,6]cyclohepta[1,2-d]thiazol-4-yl)-1-(2-(pyrrolidinylmethyl)-3,4-dihydro-4-thioxo-2(1H)-pyrimidinone

The product from step (iv) (0.12g) was dissolved in dry pyridine (15ml) and triethylamine (10ml) and treated with hydrogen sulphide gas bubbled through for 35min. The volatiles were then removed under reduced pressure leaving a yellow gum. The title product was obtained by purification by chromatography eluting with ethyl acetate, methanol and triethylamine mixtures to give the product as a yellow solid. Yield. 0.05g
MS: APCI(+ve); 457 (M+1, 100%), APCI(-ve); 455 (M-1, 100%
1HNMR δ (CDCl₃): 7.67 (d, 1H), 7.50(s, 1H), 7.30(m, 2H), 6.90 (dd, 2H), 6.11 (s, 1H), 3.75 (m, 2H), 3.55 (m, 1H), 3.0 (m, 2H), 2.0-1.6 (m, 4H)
MP. 178 °C

### Example 40

### (5S,2S)-5-(7-Chloro-2-methyl-4H-benzo[5,6]cyclohepta[1,2-d]thiazol-4-yl)-1-(2-(pyrrolidinylmethyl)-3,4-dihydro-4-thioxo-2(1H)-pyrimidinone

### i) (S)- N-(1,1-Dimethylethoxycarbonyl)-2-[[(4-methylsulphonyl)oxy]methyl]pyrrolidine

The subtitle product was prepared from (S)- *N*-( (1,1-dimethylethoxycarbonyl)-2-pyrrolidinemethanol (2.2g) according to the method of example 39 step (ii) as a colourless oil. Yield:3.8g
Used directly in the next step.

### ii) (5S,2S)-5-(7-Chloro-2-methyl-4H-benzo[5,6]cyclohepta[1,2-d]thiazol-4-yl)-1-(2-(1-(1,1-dimethylethoxycarbonyl)pyrrolidinylmethyl)-4-methylthio-2(1H)-pyrimidinone

The subtitle compound was prepared from the products of example 39 step (i) (0.71g) and the product of step (i) (3.8g) according to the method of example 39 step (ii).
Two diastereoisomers were separable by TLC using ethyl acetate as eluant.
Purification and isolation of the more polar diastereoisomer was by chromatography eluting with ethyl acetate to give the subtitle product as a colourless foam. Yield. 0.16g
MS: APCI(+ve); 572 (M+1, 30%), 471 (100%)

### iii) (5S,2S)-5-(7-Chloro-2-methyl-4H-benzo[5,6]cyclohepta[1,2-d]thiazol-4-yl)-1-(2-pyrrolidinylmethyl)-4-methylthio-2(1H)-pyrimidinone trifluoroacetate

The subtitle product was prepared from the product of step (ii) according to the method of example 39 step (iv). Yield. 0.16g. Used directly in the next step.

### iv) (5S,2S)-5-(7-Chloro-2-methyl-4H-benzo[5,6]cyclohepta[1,2-d]thiazol-4-yl)-1-(2-(pyrrolidinylmethyl)-3,4-dihydro-4-thioxo-2(1H)-pyrimidinone

The title product was prepared from the product of step (iii) (0.16g) according to the method of example 39 step (v). Purification was by chromatography eluting with ethyl acetate,methanol and triethylamine mixtures to give the product as a yellow solid. Yield: 0.05g
MS: APCI(+ve); 457 (M+1, 100%), APCI(-ve); 455 (M-1, 100% 1HNMR δ (CDCl₃): 7.74(d, 1H), 7.50(s, 1H), 7.30 (m, 2H), 6.90(dd, 2H), 6.18(s, 1H), 3.85(dd, 1H), 3.42(m,1H), 3.30(m,1H), 3.0-2.8(m, 2H), 2.70 (s, 3H), 2.0-1.20(m, 4H),
MP. 235 °C

### Example 41

### (±)-5-(7-Chloro-1-((imidazol-4-yl)ethyl)-2-methyl-4H-benzo[5,6]cyclohepta[1,2-d]imidazol-4-yl)-1-methyl-3,4-dihydro-4-thioxo-2(1H)-pyrimidinone

### i) 7-Chloro-1-((imidazol-4-yl)ethyl)-2-methyl-4H-benzo[5,6]cyclohepta[1,2-d]imidazol-4-one

A mixture of the product from example 42 step (i) (1g) and histamine (3g) in 1-methyl-2-pyrrolidone (10ml) was stirred at 100 °C for 4 hours. The mixture was diluted with water (10ml) and the solid filtered. The solid was further washed with water and then dichloromethane to leave the subtitle product as a beige powder. Yield: 0.986g
MS: APCI(+ve): 339(M+1, 100%), APCI(-ve); 337(M-1, 100%)

### ii) (±)-7-Chloro-1-((imidazol-4-yl)ethyl)-2-methyl-4H-benzo[5,6]cyclohepta[1,2-d]imidazol-4-ol

The subtitle product was prepared from the product of step (i) (0.966g) and sodium borohydride (0.215g) according the method of example 71 step (ii).
Used directly in the next step.

### iii) (±)-5-(7-Chloro-1-((imidazol-4-yl)ethyl)-2-methyl-4H-benzo[5,6]cyclohepta[1,2-d]imidazol-4-yl)-1-methyl-2,4(3H,4H)-pyrimidinedione

The subtitle product was prepared from the product of step (ii) and 1-methyluracil (0.36g) according to the method of example 42 step (iv). Purification was by biotage chromatography on silica eluting with 5% methanol in dichloromethane with 1% 0.880 ammonia present to give a white solid. Yield: 0.756g
MS: APCI(+ve): 449(M+1, 100%), APCI(-ve); 447(M-1, 100%)

### iv) (±)-5-(7-Chloro-1-((imidazol-4-yl)ethyl)-2-methyl-4H-benzo[5,6]cyclohepta[1,2-d]imidazol-4-yl)-1-methyl-3,4-dihydro-4-thioxo-2(1H)-pyrimidinone

The title compound was prepared from the product of step (iii) (0.2g) and Lawesson's reagent (0.9g) according to the method of example 34 step (iv). Purification was by biotage chromatography on silica eluting with 5% methanol in dichloromethane with 1% 0.880 ammonia present to give a yellow glass. Yield: 0.044g
MS: APCI(+ve); 465 (M+1, 100%), APCI(-ve); 463(M-1, 100%)
1HNMR δ(DMSO): 12.6 (bs, 1H), 7.70(d, 1H), 7.6(s, 1H), 7.45(d, 1H), 7.34(dd, 1H), 7.17(s, 1H), 6.78(m, 3H), 5.74(s, 1H), 4.20(m, 2H), 3.22(s, 3H), 2.84(m, 2H), 2.17(s, 3H)

### Example 42

### (±)-5-(7-Chloro-1,2-dimethyl-4H-benzo[5,6]cyclohepta[1,2-d]imidazol-4-yl)-1-methyl-3,4-dihydro-4-thioxo-2(1H)-pyrimidinone

### i) 7-Chloro-2-methyl-4H-benzo[5,6]cyclohepta[1,2-d]oxazol-4-one

A stirred solution of the product from example 25, step (vi) (10g), and *N*-bromosuccinimide (7.19g) in benzene (500ml), was irradiated under a 500 Watt halogen lamp for 3.5h. The mixture was then treated with triethylamine (11.25ml), and further stirred for 16h. The mixture was diluted with dichloromethane, washed with water, organic phase collected, dried (MgSO₄), and evaporated under reduced pressure. The residue was recrystallised from ethyl acetate to give the subtitled product as a beige crystalline solid. Yield:6g
MS: APCI(+ve); 246 (M+1, 100%)

### ii) 7-Chloro-1,2-dimethyl-4H-benzo[5,6]cyclohepta[1,2-d]imidazol-4-one

A mixture of the product from step (i) (3.33g), and 40% w/v aqueous methylamine solution was heated at 40 °C for 4h. The solid was filtered and washed with water then dried under vacuum to give the subtitled product as a cream powder. Yield: 2.1g
MS: APCI(+ve); 261 (M+1, 100%)

### iii) (±)-7-Chloro-1,2-dimethyl-4H-benzo[5,6]cyclohepta[1,2-d]imidazol-4-ol

A mixture of the product from step (ii) (1.43g), and sodium borohydride (0.42g) in ethanol (100ml) and dichloromethane (20ml) were heated at 50 °C for 4h. The solvents were evaporated under reduced pressure and the residue was dissolved in dichloromethane, washed with dilute aqueous sodium hydroxide solution, dried (CaCl₂), and evaporated to give the subtitled product as a brown solid. Yield:1.12g
MS: APCI(+ve); 243 (M+1, 100%)

### iv) (±)-5-(7-Chloro-1,2-dimethyl-4H-benzo[5,6]cyclohepta[1,2-d]imidazol-4-yl)-1-methyl-2,4(1H,3H)-pyrimidinedione

A mixture of the product from step (iii) (0.91g), and 1-methyluracil (0.44g) in acetonitrile (40ml) was treated with boron trifluoride etherate (2.47g) and heated at 50 °C for 4h. The solution was treated with methanol (1ml) and evaporated. Purification was by chromatography on a biotage silica column, using 3% methanol/ 0.1% 0.880 ammonia in dichloromethane as eluant to give the subtitled product as a cream powder. Yield: 0.54g
MS: APCI(+ve); 369 (M+1, 100%)

### v) (±)-5-(7-Chloro-1,2-dimethyl-4H-benzo[5,6]cyclohepta[1,2-d]imidazol-4-yl)-1-methyl-3,4-dihydro-4-thioxo-2(1H)-pyrimidinone

A mixture of the product from step (iv) (0.535g), and phosphorus pentasulphide (0.967g) in 1,4-dioxane (10ml) was heated at 100 °C for 3h. The solvent evaporated and the residue dissolved in dichloromethane, washed with saturated aqueous sodium bicarbonate solution then with water, dried (MgSO₄) and evaporated. Purification was by chromatography on a biotage silica column, using 3% methanol/ 0.1% 0.880 ammonia in dichloromethane as eluant. Yield: 0.3g.
MS: APCI(+ve): 385(M+1, 100%), APCI(-ve): 383(M-1, 100%)
1HNMR δ(DMSO): 7.67(d, 1H), 7.44(d, 1H), 7.32(dd, 1H), 7.25(s, 1H), 6.93(d, 1H), 6.84(d, 1H), 5.75(s, 1H), 3.56(s, 3H), 3.21(s, 3H), 2.31(s, 3H)

### Example 43

### (S)-5-(7-Chloro-1,2-dimethyl-4H-benzo[5,6]cyclohepat[1,2-d]imidazol-4-yl)-1-methyl-3,4-dihydro-4-thioxo-2(1H)-pyrimidinone

The title compound was obtained from the product of example 42 step (v) (0.3g) by resolution of the racemate on HPLC (chiral-pak AD® column and ethanol as eluant). The title compound was the first enantiomer to be eluted off the column and was obtained as a yellow powder. Yield: (0.090g)
MS: APCI(+ve); 385 (M+1, 100%), APCI(-ve):383 (M-1, 100%)
1HNMR δ(DMSO): 7.67(d, 1H), 7.44(d, 1H), 7.32(dd, 1H), 7.25(s, 1H), 6.93(d, 1H), 6.84(d, 1H), 5.75(s, 1H), 3.56(s, 3H), 3.21(s, 3H), 2.31(s, 3H)

### Example 44

### (±)-5-(2,7-Dichloro-4H-benzo[5,6]cyclohepta[1,2-d]thiazol-4-yl)-1-methyl-2,4(1H,3H)-pyrimidinedione

### i) 2-Amino-5-(2-(3-chlorophenyl)ethyl)-4-thiazolecarboxylic acid, methyl ester

A solution of sodium methoxide (made from 1.43g of sodium in dry methanol (20ml) was added dropwise to a stirred solution of 2-(3-chlorophenyl)propanal (10..4g) and methyl dichloroacetate (6.4ml) in diethyl ether (30ml) at 0 °C under nitrogen.
After 1h the mixture was quenched with brine and the product extracted into diethyl ether. The organic phase was collected, dried (MgSO₄) and evaporated under reduced pressure to leave a yellow oil. The oil and thiourea (4.72g) were dissolved in methanol (40ml) and the mixture set at reflux for 4h. The solvent was removed under reduced pressure and the residue partitioned between ethyl acetate and saturated sodium bicarbonate solution. The organic phase was separated, washed with water, collected, dried (MgSO₄) and solvent evaporated under reduced pressure to leave a brown solid. The solid was triturated with isohexane and ethyl acetate mixtures and filtered to leave the subtitle product. Yield: 5.9g
MS: APCI(+ve); 297/9 (M+1, 100%)

### ii) 2-Chloro-5-(2-(3-chlorophenyl)ethyl)-4-thiazolecarboxylic acid, methyl ester

*Tert*-Butylnitrite (4.5ml) was added to a suspension of the product of step (i) (5.85g) and anhydrous copper(II)chloride (4.03g) in dry acetonitrile (50ml) at room temperature over 0.5 hours. After a further 1h the reaction mixture was quenched with 2M HCl and extracted with ethyl acetate. The organic phase was collected, dried (MgSO₄) and solvent evaporated under reduced pressure. Purification was by chromatography eluting with 10-15% ethyl acetate in isohexane. Yield: 3.98g
MS:APCI(+ve); 316/8 (M+1, 100%)

### iii) 2-Chloro-5-(2-(3-chlorophenyl)ethyl)-4-thiazolecarboxylic acid

The product from step (ii) (3.46g) was treated with sodium hydroxide (0.7g) in tetrahydrofuran (15ml) and water (15m) at room temperature. After 3h the mixture was partitioned between 2M HCl and ethyl acetate. The organic phase was collected, dried (MgSO₄) and solvent evaporated under reduced pressure to leave a colourless solid. Yield: 3.22g
MS:APCI(+ve); 300/2(M+1, 100%)

### iv) 2-Chloro-5-(2-(3-chlorophenyl)ethyl)-4-thiazolecarboxylic acid chloride

Oxalyl chloride (1.75ml) was added to a solution of the product from step (iii) (3.14g) in dichloromethane (30ml) at room temperature containing 2 drops of dimethylformamide. After 4h the volatiles were removed under reduced pressure to leave the subtitle product. Used directly in the next step.

### v) 2,7-Dichloro-9,10-dihydro-4H-benzo[5,6]cyclohepta[1,2-d]thiazol-4-one

Aluminium chloride (5.3g) was added to a solution of the product from step (iv) in dichloromethane (30ml) at room temperature. After 3h the mixture was poured onto 2M HCI / ice and the product extracted into ethyl acetate. The organic phase was collected, dried, (MgSO₄) and solvent evaporated under reduced pressure to leave a brown solid. This was triturated with isohexane/ ethyl acetate and filtered to leave the subtitle product as a beige solid. Yield: 1.93g
MS: APCI(+ve);284/6 (M+1), 248/50(100%)

### vi) 2,7-Dichloro-4H-benzo[5,6]cyclohepta[1,2-d]thiazol-4-one

The product from step (v) (1.72g) in 1,2-dichloroethane (30ml) was treated with N-bromosuccinimide (1.08g) with irradiation from a 500 Watt halogen lamp for 1.25h. Triethylamine (8ml) was then added and the mixture further stirred for 3h. The subtitle product was obtained as the precipitated solid by filtration. Yield: 1.27g
MS: APCI(+ve); 282/4 (M+1, 100%)

### vii) (±)-2,7-Dichloro-4H-benzo[5,6]cyclohepta[1,2-d]thiazol-4-ol

Sodium borohydride (0.159g) was added to a suspension of the product from step (vi) (1.27g) in methanol (20ml) and dichloromethane (5ml) at room temperature. After 3h the mixture was partitioned between 2N NaOH and dichloromethane. The organic phase was collected, dried, (MgSO₄) and solvent evaporated to leave a colourless foam. Yield: 1.27g Used directly in the next step.

### viii) (±)-5-(2,7-Dichloro-4H-benzo[5,6]cyclohepta[1,2-d]thiazol-4-yl)-1-methyl-2,4(1H,3H)-pyrimidinedione

The title product was prepared from the product of step (vii) (1.27g) and 1-methyluracil (1g) according to the method of example 38 step (iv). Purification was by chromatography eluting with 50-60% ethyl acetate in isohexane. Yield: 1.29g
MS: APCI(+ve); 392/4 (M+1, 100%)
1HNMR δ (DMSO): 11.28(s, 1H), 7.57-7.47(m, 3H), 7.12(d, 1H), 7.04(d, 1H), 6.87(s, 1H), 5.51(s, 1H), 3.14(s, 3H)

### Example 45

### (±)-5-(7-Chloro-4H-benzo[5,6]cyclohepta[1,2-d]thiazol-4-yl)-1-methyl-2,4(1H,3H)-pyrimidinedione

Zinc powder (4g) was added to a solution of the product from example 44 step (viii) (0.5g) in acetic acid. After 24h the solvent was evaporated under reduced pressure and the residue partitioned between saturated sodium bicarbonate solution and dichloromethane. The organic phase was collected, dried, (MgSO₄) and solvent evaporated to leave as a beige foam. Purification by trituration and filtration from isohexane/ethyl acetate mixtures gave the title product. Yield: 0.44g
MS: APCI(+ve); 358 (M+1, 100%)
1HNMR δ (DMSO): 11.28 (s, 1H), 9.12(s, 1H), 7.65-7.41(m, 3H), 7.13(d, 2H), 6.96(s, 1H), 5.57(s, 1H), 3.15(s, 3H).

### Example 46

### (±)-5-(7-Chloro-2-methoxy-4H-benzo[5,6]cyclohepta[1,2-d]thiazol-4-yl)-1-methyl-2,4(1H,3H)-pyrimidinedione

The product from example 44 step (viii) (0.1g) was heated with a solution of sodium methoxide (made from sodium (0.2g) in dry methanol (10ml)) at reflux for 3h. The mixture was partitioned between ethyl acetate and water. The organic phase was collected, dried (MgSO₄) and solvent evaporated under reduced pressure. Purification was by chromatography eluting with 70% ethyl acetate in isohexane to give the title product as a colourless solid. Yield: 0.025g
MS: APCI(+ve);388/90 (M+1, 100%)
1HNMR δ (DMSO): 11.27(s, 1H), 7.52-7.41(m, 3H), 6.97(d, 1H), 6.96(s, 1H), 6.90(d, 1H), 5.33(s, 1H), 3.16(s, 3H), 4.04(s, 3H)
MP: 150°C (dec)

### Example 47

### (±)-5-(7-Chloro-2-methylthio-4H-benzo[5,6]cyclohepta[1,2-d]thiazol-4-yl)-1-methyl-2,4(1H,3H)-pyrimidinedione

The product from example 44 step (viii) (0.05g) was treated with sodium methane thiolate (0.02g) in dimethylformamide (2ml) at room temperature. After 1h the mixture was was partitioned between ethyl acetate and water. The organic phase collected, dried (MgSO₄) and solvent evaporated under reduced pressure. Purification was by precipitation with ethyl acetate / isohexane mixtures and filtration to give the title product. Yield: 0.031g
MS:APCI(+ve); 404/6 (M+1, 100%)
1HNMR δ (DMSO): 11.28 (s, 1H), 7.53(d, 1H), 7.52(d, 1H), 7.44(dd, 1H), 7.07-6.98(d, 2H), 6.98(s, 1H), 5.47(s, 1H), 3.15(s, 3H), 2.69(s, 3H)
MP: 214-6°C

### Example 48

### (±)-5-(2-Amino-7-chloro-4H-benzo[5,6]cyclohepta[1,2-d]thiazol-4-yl)-1-methyl-2,4(1H,3H)-pyrimidinedione

The product from example 44 step (viii) (0.05g) was heated with 0.88 ammonia (2ml) and ethanol (1ml) in a sealed tube at 100 "C for 24h. The volatiles were removed under reduced pressure and the residue triturated with ethyl acetate and methanol mixtures to leave the title product as a yellow solid. Yield: 0.008g
MS: APCI(+ve): 373/5 (M+100%)
1HNMR δ (DMSO): 11.22 (s, 1H), 7.45-7.34(m, 3H), 7.30(s, 2H), 6.93(s, 1H), 6.67(d, 2H), 5.21(s, 1H), 3.15(s, 3H)
MP:>240 °C

### Example 49

### (±)-5-(7-Chloro-2-methylamino-4H-benzo[5,6]cyclohepta[1,2-d]thiazol-4-yl)-1-methyl-2,4(1H,3H)-pyrimidinedione

The product from example 44 step (viii) (0.05g) was heated with 40% aq. Methylamine (4ml) in ethanol (1ml) for 3 hours. The mixture was partitioned between ethyl acetate and water. The organic phase was collected, dried (MgSO₄) and solvent evaporated under reduced pressure. The residue was triturated with ethyl acetate /*iso*-hexane mixtures and the title product collected by filtration as a pale yellow solid. Yield:0.033g
MS:APCI(+ve);387/9;(M+1, 100%)
1HNMR δ (DMSO): 11.23(s, 1H), 7.83(brq, 1H), 7.46(d, 1H), 7.42(d, 1H), 7.36(dd, 1H), 6.95(s, 1H), 6.81(d, 1H), 6.74(d, 1H), 5.25(s, 1H), 3.15(s, 3H), 2.82(d, 3H)
MP: >240 °C

### Example 50

### (±)-5-(7-Chloro-2-dimethylamino-4H-benzo[5,6]cyclohepta[1,2-d]thiazol-4-yl)-1-methyl-2,4(1H,3H)-pyrimidinedione

The product from example 44 step (viii) (0.05g) was heated with 40% aq. dimethylamine (2ml) in ethanol (1ml) for 1hour. The mixture was partitioned between ethyl acetate and water. The organic phase was collected, dried (MgSO₄) and solvent evaporated under reduced pressure. The residue was triturated with ethyl acetate/ isohexane mixtures and the title product collected by filtration as a pale yellow solid. Yield:0.031g
MS: APCI(+ve);401/3 (M+1, 100%)
1HNMR δ (DMSO): 7.46(d, 1H), 7.42(d, 1H), 7.36(dd, 1H), 6.96(s, 1H), 6.86(d, 1H), 6.76(d, 1H), 5.28(s, 1H), 3.15(s, 3H), 3.04(s, 6H)
MP: >220 °C

### Example 51

### (±)-5-(7-Chloro-2-(pyrrolidin-1-yl)-4H-benzo[5,6]cyclohepta[1,2-d]thiazol-4-yl)-1-methyl-3,4-dihydro-4-thioxo-2(1H)-pyrimidinone

### i) (±)-5-(7-Chloro-2-(pyrrolidin-1-yl)-4H-benzo[5,6]cyclohepta[1,2-d]thiazol-4-yl)-1-methyl-2,4(1H,3H)-pyrimidinedione

The product from example 44 step (viii) (0.042g) was treated with pyrrolidine (0.1ml) in 1,4-dioxane (3ml) at reflux for 6h. The volatiles were removed under reduced pressure and the residue triturated with diethyl ether/isohexane mixtures to leave the subtitle product as a pale yellow solid. Yield:0.04g
MS:APCI(+ve); 427/9 (M+1, 100%)

### ii) (±)-5-(7-Chloro-2-(pyrrolidin-1-yl)-4H-benzo[5,6]cyclohepta[1,2-d]thiazol-4-yl)-1-methyl-3,4-dihydro-4-thioxo-2(1H)-pyrimidinone

The product from step (i) (0.04g) was treated with Lawesson's reagent (0.037g) according to the method of example 34 step (iv). Purification was by chromatography eluting with 1:1 ethyl acetate/isohexane to give the title product as a yellow solid. Yield: 0.016g
MS:APCI(+ve);443/5(M+1, 100%), APCI(-ve);441/3; (M-1, 100%)
1HNMR δ (CDCl₃): 11.60(bs, 1H), 7.70(d, 1H), 7.28(m, 2H), 7.10(s, 1H), 6.7(dd, 2H), 6.09(s, 1H), 6.30(bs, 2H), 3.27(s, 3H)

### Example 52

### (±)-5-(7-Chloro-2-(N4-methyl-piperazin-1-yl))-4H-benzo[5,6]cyclohepta[1,2-d]thiazol-4-yl)-1-methyl-3,4-dihydro-4-thioxo-2(1H)-pyrimidinone

### i) (±)-5-(7-Chloro-2-(N4-methyl-piperazin-1-yl))-4H-benzo[5,6]cyclohepta[1,2-d]thiazol-4-yl)-1-methyl-2,4(1H,3H)-pyrimidinedione

The subtitle compound was prepared from the product of example 44 step (viii) (0.225g) and *N*-methyl piperazine (0.32ml) according to the method of example 51 step (i). Purification was by precipitation using ethyl acetate and isohexane with filtration to give the subtitle product as yellow solid. Yield: 0.254g
MS: APCI(+ve); 456/8 (M+1, 100%)

### ii) (±)-5-(7-Chloro-2-(N4-methyl-piperazin-1-yl))-4H-benzo[5,6]cyclohepta[1,2-d]thiazol-4-yl)-1-methyl-3,4-dihydro-4-thioxo-2(1H)-pyrimidinone

The title product was prepared from the product of step (i) (0.225g) and Lawesson's reagent (0.77g) according to the method of example 34 step (iv).Purification was by chromatography eluting with dichloromethane/ethanol/triethylamine mixtures to give the title product as a yellow solid. Yield. 0.086g
MS:APCI(+ve); 472/4(M+1, 100%), APCI(-ve);(470/2; (M-1, 100%)
1HNMR δ (CDCl₃): 7.74(d, 1H), 7.32(dd, 1H), 7.30(s, 1H), 7.10(s, 1H), 6.72(dd, 2H), 6.02(s, 1H), 3.50(m, 4H), 3.30(s, 3H), 2.50(t, 4H), 2.35(s, 3H). rotamers

### Example 53

### (±)-5-(7-Chloro-2-(2-(4-morpholinyl)ethylamino)-4H-benzo[5,6]cyclohepta[1,2-d]thiazol-4-yl)-1-methyl-3,4-dihydro-4-thioxo-2(1H)-pyrimidinone

### i) (±)-5-(7-Chloro-2-(2-(4-morpholinyl)ethylamino)-4H-benzo[5,6]cyclohepta[1,2-d]thiazol-4-yl)-1-methyl-2,4(1H,3H)-pyrimidinedione

The subtitle product was prepared from the product of example 44 step (viii) (0.225g) and 4-(2-aminoethyl)morpholine (0.38ml) according to the method of example 51 step (i). Purification was by precipitation using diethyl ether with filtration to give the subtitle product as yellow solid. Yield: 0.162g
MS: APCI(+ve); 486/8 (M+1, 100%), APCI(-ve);484/6(M-1, 100%)

### ii) (±)-5-(7-Chloro-2-(2-(4-morpholinyl)ethylamino)-4H-benzo[5,6]cyclohepta[1,2-d]thiazol-4-yl)-1-methyl-3,4-dihydro-4-thioxo-2(1H)-pyrimidinone

The title product was prepared from the product of step (i) (0.162g) and Lawesson's reagent (0.7g) according to the method of example 34 step (iv). Purification was by chromatography eluting with dichloromethane/ethanol/triethylamine mixtures to give the title product as a yellow solid. Yield. 0.074g
MS:APCI(+ve); 502/4(M+1, 100%),
1HNMR δ (CDCl₃): 9.40(bs, 1H), 7.72(d, 1H), 7.30(dd, 1H), 7.26(s, 1H), 7.06(s, 1H), 6.71(dd, 2H), 6.07(s, 1H), 3.80(m,4H), 3.40(m, 2H), 3.30(s, 3H), 3.15(m, 2H), 2.50(m, 4H) rotamers

### Example 54

### (±)-5-(7-Chloro-4H-benzo[5,6]cyclohepta[1,2-d]thiazol-4-yl)-1-methyl-3,4-dihydro-4-thioxo-2(1H)-pyrimidinone

The title product was prepared from the product of example 45 step (i) (0.44g) and Lawesson's reagent (1.5g) according to the method of example 34 step (iv).
Purification was by chromatography eluting with 7:3 ethyl acetate/isohexane to give the title product as a yellow solid. Yield: 0.23g
MS: APCI(+ve); 374(M+1, 100%)
1HNMR δ (CDCl₃): 9.4(bs, 1H), 7.60(d, 1H), 8.78(s, 1H), 7.40(m, 3H), 7.0(s, 2H), 6.24(s, 1H), 3.35(s, 3H)

### Example 55

### (S)-5-(7-Chloro-4H-benzo[5,6]cyclohepta[1,2-d]thiazol-4-yl)-1-methyl-3,4-dihydro-4-thioxo-2(1H)-pyrimidinone

The title compound was obtained from the product of example 54 by resolution of the racemate on HPLC (chiral-pak AD® column and ethanol as eluant). The title compound was the second enantiomer to be eluted off the column and was obtained as a yellow powder.
MS:APCI(+ve); 374(M+1, 100%)
1HNMR δ (CDCl₃): 9.45(bs, 1H), 8.80(s, 1H), 7.60(d, 1H), 7.40(m, 2H), 7.0(s, 2H), 6.23(s, 1H), 3.35(s, 3H)

### Example 56

### (±)-5-(2-Amino-7-Chloro-4H-benzo[5,6]cyclohepta[1,2-d]thiazol-4-yl)-1-methyl-3,4-dihydro-4-thioxo-2(1H)-pyrimidinone

The title product was obtained from the product of example 48 (0.11g) and Lawesson's reagent (0.6g) according to the method of example 34 step (iv). Purification was by chromtography eluting with ethyl acetate to give the title product as yellow solid. Yield: 0.01g
MS:APCI(+ve); 389 (M+1, 100%), APCI(-ve); 387(M-1, 100%)
1HNMR δ (DMSO+CDCl₃): 11.60(bs, 1H), 8.0(bs, 1H), 7.70(d, 1H), 7.30(m, 2H), 7.10(s, 1H), 6.7(dd, 2H), 6.10(s, 1H), 3.27(s, 3H)

### Example 57

### (±)-5-(7-chloro-2-methyl-4H-benzo[5,6]cyclohepta[1,2-d]thiazol-4-yl)-1-methyl-3,4-dihydro-4-thioxo-2(1H)-pyrimidinone

A solution of the product of example 39 step (i) (0.1g) together with methyl iodide (0.040 ml) and a 1M solution of potassium tert-butoxide in tetrahydrofuran (0.52 ml) in *N,N*-dimethylformamide (10 ml) was heated at 80°C for 2h. The reaction mixture was cooled, diluted with ethyl acetate and washed with saturated aqueous ammonium chloride and brine. The organic phase was dried over magnesium sulphate, filtered and evaporated. The crude product was purified by chromatography eluting with 5% ethanol in ethyl acetate to give a yellow oil (86 mg). This material was dissolved in a saturated blue solution of sodium hydrosulphide in *N*,*N*-dimethylformamide (3 ml) and heated at 80°C for 3h. The reaction mixture was cooled, diluted with dichloromethane and washed with water. The organic phase was dried over magnesium sulphate, filtered and evaporated. The crude product was purified by chromatography eluting with 30% isohexane in ethyl acetate to give the title compound as a yellow solid. Yield: 0.02 g.
MS: APCI(+ve): 388 (M+1, 100%).
1H NMR: δ (CDCl₃) 9.30 (br s,1H), 7.68 (d, 1H), 7.36 (m, 2H), 7.17 (s, 1H), 6.91 (dd, 2H), 6.20 (s, 1H), 3.31 (s, 3H), 2.70 (s, 3H).
MP: 305°C.

### Example 58

### (±)-5-(7-Chloro-1-ethyl-2-methyl-4H-benzo[5,6]cyclohepta[1,2-d]imidazol-4-yl)-1-methyl-3,4-dihydro-4-thioxo-2(1H)-pyrimidinone

### i) (±)-5-(7-Chloro-1-ethyl-2-methyl-4H-benzo[5,6]cyclohepta[1,2-d]imidazol-4-yl)-1-methyl-2,4(1H,3H)-pyrimidinedione

A solution of the product of example 42 step (i) (0.15 g) and diethylamine (0.157 g of a 70% solution in water) in butanol (0.5 ml) and *N*-methylpyrrolidinone (0.5 ml) was heated at 80°C in a sealed tube for 24h. The reaction mixture was cooled, diluted with ethanol (3 ml), sodium borohydride (0.070 g) added, and heated at 50°C in a sealed tube for 3h. The reaction was diluted with dichloromethane (50 ml), washed with pH5 phosphate buffer solution and brine, dried over magnesium sulphate and evaporated. The crude material was redissolved in acetonitrile (3 ml) and 1-methyl uracil (0.115 g) and boron trifluoride etherate (0.225 ml) added. The reaction was heated at 80°C for 1h before quenching with methanol (2 ml). After removal of solvent, the crude product was purified by chromatography eluting with 0.5% 880 ammonia, 4% methanol in dichloromethane to give the subtitle compound as a colourless oil. Yield 0.068 g.
MS: APCI(+ve) 384 (M+1) (100%)

### ii)(±)-5-(7-Chloro-1-ethyl-2-methyl-4H-benzo[5,6]cyclohepta[1,2-d]imidazol-4-yl)-1-methyl-3,4-dihydro-4-thioxo-2(1H)-pyrimidinone

A mixture of the product of step (i) (0.065 g) and Lawesson's reagent (0.343 g) in 1,4-dioxane (5 ml) was heated at reflux for 20 hours. The solvent was evaporated under reduced pressure and the residue partitioned between dichloromethane and water. Purification was by chromatography eluting with 0.2% 880 ammonia, 4% methanol in dichloromethane to give the title compound. Yield: 0.047 g.
MS: APCI(+ve): 399 (M+1, 100%).
1H NMR: δ (CDCl₃) 9.37 (br s,1H), 7.75 (d, 1H), 7.29 (m, 2H), 7.03 (s, 1H), 6.77 (d, 1H), 6.66 (d, 1H), 6.01 (s, 1H), 3.98 (q, 2H), 3.26 (s, 3H), 2.43 (s, 3H), 1.33 (t, 3H).
MP: 260-262°C (dec).

### Example 59

### (±)-5-(7-Chloro-1-cyclopropyl-2-methyl-4H-benzo[5,6]cyclohepta[1,2-d]imidazol-4-yl)-1-methyl-3,4-dihydro-4-thioxo-2(1H)-pyrimidinone

### i) (±)-5-(7-Chloro-1-cyclopropyl-2-methyl-4H-benzo[5,6]cyclohepta[1,2-d]imidazol-4-yl)-1-methyl-2,4(1H,3H)-pyrimidinedione

A solution of the product of example 42 step (i) (0.15 g) and cyclopropylamine (0.140 g) in butanol (0.3 ml) and *N*-methylpyrrolidinone (0.3 ml) was heated at 80°C in a sealed tube for 24h. The reaction mixture was cooled, diluted with ethanol (3 ml), sodium borohydride (0.070 g) added, and heated at 50°C in a sealed tube for 3h. The reaction was diluted with dichloromethane (50 ml), washed with pH5 phosphate buffer solution and brine, dried over magnesium sulphate and evaporated. The crude material was redissolved in acetonitrile (3 ml) and 1-methyl uracil (0.115 g) and boron trifluoride etherate (0.225 ml) added. The reaction was heated at 80°C for 1h before quenching with methanol (2 ml). After removal of solvent, the crude product was purified by chromatography eluting with 0.5% 880 ammonia, 4% methanol in dichloromethane to give the title compound as a colourless oil.
Yield: 0.073 g.
MS: APCI(+ve): 395 (M+1,100%)

### ii) (±)-5-(7-Chloro-1-cyclopropyl-2-methyl-4H-benzo[5,6]cyclohepta[1,2-d]imidazol-4-yl)-1-methyl-3,4-dihydro-4-thioxo-2(1H)-pyrimidinone

A mixture of the product of step (i) (0.070 g) and Lawesson's reagent (0.350 g) in 1,4-dioxane (5 ml) was heated at reflux for 8 hours. The solvent was evaporated under reduced pressure and the residue partitioned between dichloromethane and water. Purification was by chromatography eluting with 0.2% 880 ammonia, 2% methanol in dichloromethane to give the title compound. Yield: 0.043 g.
MS: APCI(+ve): 411 (M+1, 100%).
1H NMR: δ (CDCl₃) 9.30 (br s,1H), 7.74 (d, 1H), 7.30 (m, 2H), 7.04 (s, 1H), 6.96 (d, 1H), 6.76 (d, 1H), 5.99 (s, 1H), 3.27 (s, 3H), 3.10 (m, 1H), 2.50 (s, 3H), 1.20 (m, 2H), 0.98 (m, 2H).
MP: 295-298°C (dec).

### Example 60

### (±)-5-(7-Chloro-2-methyl-4H-benzo[5,6]cyclohepta[1,2-d]thiazol-4-yl)-1-(2-hydroxyethyl-3,4-dihydro-4-thioxo-2(1H)-pyrimidinone

A solution of the product of example 39 step (i) (0.250 g) together with 2-bromoethanol (0.114 ml) and a 1M solution of potassium tert-butoxide in tetrahydrofuran (1.29 ml) in *N,N*-dimethylformamide (3 ml) was heated at 50°C for 20h. Sufficient sodium hydrosulphide was added to give a blue solution and the reaction mixture heated at 80°C for 4h. The reaction mixture was cooled, diluted with ethyl acetate and washed with saturated aqueous ammonium chloride. The organic phase was dried over magnesium sulphate, filtered and evaporated. The crude product was purified by chromatography eluting with 2% methanol in dichloromethane, followed by trituration with ethyl acetate to give the subtitle compound as a yellow solid. Yield: 0.045g.
MS: APCI(+ve): 418 (M+1, 100%).
1H NMR: δ (CDCl₃) 9.52 (br s,1H), 7.66 (d, 1H), 7.34 (m, 3H), 6.90 (dd, 2H), 6.16 (s, 1H), 3.83 (m, 4H), 2.69 (s, 3H).

### Example 61

### (S)-5-(7-chloro-2-methyl-4H-benzo[5,6]cyclohepta[1,2-d]thiazol-4-yl)-1-(2-hydroxyethyl)-3,4-dihydro-4-thioxo-2(1H)-pyrimidinone

The title compound was obtained from the product example 54 step by resolution of the racemate on HPLC (chiral-pak AD® column and ethanol as eluant). The title compound was the second enantiomer to be eluted off the column and was obtained as a yellow powder.
MS: APCI(+ve): 418 (M+1, 100%).
1H NMR: δ (CDCl₃) 9.52 (br s,1H), 7.66 (d, 1H), 7.34 (m, 3H), 6.90 (dd, 2H), 6.16 (s, 1H), 3.83 (m, 4H), 2.69 (s, 3H).

### Example 62

### (±)-5-(7-chloro-2-methyl-4H-benzo[5,6]cyclohepta[1,2-d]thiazol-4-yl)-1-(2-methoxyethyl)-3,4-dihydro-4-thioxo-2(1H)-pyrimidinone

A solution of the product of example 39 step (i) (0.120 g) together with 2-bromoethyl methyl ether (0.073 ml) and a 1M solution of potassium tert-butoxide in tetrahydrofuran (0.62 ml) in *N,N*-dimethylformamide (3 ml) was heated at 50°C for 20h. Sufficient sodium hydrosulphide was added to give a blue solution and the reaction mixture heated at 80°C for 4h. The reaction mixture was cooled, diluted with ethyl acetate and washed with saturated aqueous ammonium chloride. The organic phase was dried over magnesium sulphate, filtered and evaporated. The crude product was purified by chromatography eluting with 1% methanol in dichloromethane, followed by trituration with ethyl acetate to give the title compound as a yellow solid. Yield: 0.01 g.
MS: APCI(+ve): 432 (M+1, 100%).
1H NMR: δ (CDCl₃) 9.31 (br s, 1H), 7.71 (d, 1H), 7.34 (m, 3H), 6.90 (dd, 2H), 6.20 (s, 1H), 3.93 (m, 1H), 3.76 (m, 1H), 3.53 (m, 2H), 3.31 (s, 3H), 2.69 (s, 3H).

### Example 63

### (±)-5-(7-Chloro-2-[(phenylmethyl)amino]-4H-benzo[5,6]cyclohepta[1,2-d]thiazol-4-yl)-1-methyl-3,4-dihydro-4-thioxo-2(1H)-pyrimidinone

### i) (±)-5-(7-Chloro-2-[(phenylmethyl)amino]-4H-benzo[5,6]cyclohepta[1,2-d]thiazol-4-yl)-1-methyl-2,4(1H,3H)-pyrimidinedione

A mixture of the product from example 44 step (viii) (0.25g) and benzylamine (0.14ml) in dry 1,4-dioxane (3ml) were heated at 60°C for 36h. After this time benzylamine (0.14ml) and dry dimethylsulphoxide (2ml) were added and the mixture was heated at 60°C for a further 72h. The solvent was removed under reduced pressure. Purification was by trituration with diethyl ether and water. Yield: 0.17g.
MS: APCI(+ve): 465/463 (M+1, 100%).

### ii) (±)-5-(7-Chloro-2-[(phenylmethyl)amino]-4H-benzo[5,6]cyclohepta[1,2-d]thiazol-4-yl)-1-methyl-3,4-dihydro-4-thioxo-2(1H)-pyrimidinone

The title compound was prepared from the product of step (i) (0.14g) by the method of example 34 step (iv). Purification was by flash chromatography eluting with dichloromethane/20% ethyl acetate. Yield 0.106g.
MS: APCI(+ve): 481/479 (M+1, 100%).
1H NMR: δ (DMSO) 12.65(s,1H), 8.42(t,1H), 7.64(s,1H), 7.54(d,1H), 7.43(d,1H), 7.35(m,5H), 6.86(s,2H), 5.69(s,1H), 4.45(m,2H), 3.26(s,3H).

### Example 64

### (±)-5-(7-Chloro-2-(cyclobutylamino)-4H-benzo[5,6]cyclohepta[1,2-d]thiazol-4-yl)-1-methyl-3,4-dihydro-4-thioxo-2(1H)-pyrimidinone

### i) (±)-5-(7-Chloro-2-(cyclobutylamino)-4H-benzo[5,6]cyclohepta[1,2-d]thiazol-4-yl)-1-methyl-2,4(1H,3H)-pyrimidinedione

A mixture of the product from example 44 step (viii) (0.25g) and cyclobutylamine (0.54ml) in dry 1,4-dioxane (3ml) were heated at 60°C for 34h. The solvent was removed under reduced pressure and the residue partitioned between water and ethyl acetate. The organic extracts were dried (MgSO₄) and evaporated under reduced pressure. Yield 0.25g.
MS: APCI(+ve): 429/427 (M+1, 100%).

### ii) (±)-5-(7-Chloro-2-(cyclobutylamino)-4H-benzo[5,6]cyclohepta[1,2-d]thiazol-4-yl)-1-methyl-3,4-dihydro-4-thioxo-2(1H)-pyrimidinone

The title compound was prepared from the product of step (i) (0.21 g) by the method of example 34 step (iv). Purification was by flash chromatography eluting with dichloromethane/10% ethyl acetate. Yield 0.109g.
MS: APCI(+ve): 445/443 (M+1, 100%).
¹H NMR: δ (DMSO) 12.65(s,1H), 8.23(d,1H), 7.57(t,2H), 7.43(d,1H), 7.33(dofd,1H), 6.85(q,2H), 5.71(s,1H), 4.04(sextet,1H), 3.29(s,3H), 2.28(m,2H), 1.90(m,2H), 1.67(m,2H).

### Example 65

### (±)-5-(7-Chloro-2-(cyclopropylamino)-4H-benzo[5,6]cyclohepta[1,2-d]thiazol-4-yl)-1-methyl-3,4-dihydro-4-thioxo-2(1H)-pyrimidinone

### i) (±)-5-(7-Chloro-2-(cyclopropylamino)-4H-benzo[5,6]cyclohepta[1,2-d]thiazol-4-yl)-1-methyl-2,4(1H,3H)-pyrimidinedione

A mixture of the product from example 44 step (viii) (0.25g) and cyclopropylamine (0.44ml) in dry 1,4-dioxane (3ml) was heated at 100°C for 34h. The solvent was removed under reduced pressure and the residue partitioned between water and ethyl acetate. The organic extracts were dried (MgSO₄) and evaporated under reduced pressure. Yield: 0.25g.
MS: APCI(+ve): 415/413 (M+1, 100%).

### ii) (±)-5-(7-Chloro-2-(cyclopropylamino)-4H-benzo[5,6]cyclohepta[1,2-d]thiazol-4-yl)-1-methyl-3,4-dihydro-4-thioxo-2(1H)-pyrimidinone

The title compound was prepared from the product of step (i) (0.21g) by the method of example 51 step (ii). Purification was by flash chromatography eluting with dichloromethane/2% ethanol. Yield: 0.03g.
MS: APCI(+ve): 431/429 (M+1,100%)
1HNMR δ (DMSO): 8.29(s, 1H), 7.60(d, 1H), 7.53(s, 1H), 7.44(d, 1H), 7.34(dd, 1H), 6.91(d, 1H), 6.84(d, 1H), 5.79(s, 1H), 3.26(s, 3H), 0.72(m, 2H), 0.51(m, 2H)

### Example 66

### (±)-5-(7-Chloro-2-(1,3,4-thiadiazolylthio)-4H-benzo[5,6]cyclohepta[1,2-d]thiazol-4-yl)-1-methyl-2,4(1H,3H)-pyrimidinedione

To a mixture of the product from example 44 step (viii) (0.20g) and 2-mercapto-1,3,4-thiadiazole (0.120g) in dry dimethylformamide (2ml) was added 60% sodium hydride (0.041g). The mixture was stirred for 16h then heated at 55°C for 24h. The reaction mixture was diluted with water and neutralised by the addition of acetic acid. The aqueous was extracted with dichloromethane and the combined organic extracts were dried (MgSO₄). The solvent was removed under reduced pressure and the residue purified by flash chromatography eluting with dichloromethane/5% ethanol. Yield 0.135g.
MS: APCI(+ve): 476/474 (M+1, 100%).
¹H NMR: δ (CDCl₃) 9,18(s,1H), 8.26(s,1H), 7.52(d,1H), 7.40(d of d,1H), 7.36(d,1H), 6.96(d,1H), 6.85(d,1H),.6.64(s,1H), 5.79(s,1H), 3.23(s,3H).

### Example 67

### (±)-5-(7-Chloro-2-mercapto-4H-benzo[5,6]cyclohepta[1,2-d]thiazol-4-yl)-1-methyl-3,4-dihydro-4-thioxo-2(1H)-pyrimidinone

### i) (±)-5-(7-Chloro-2-(2-pyridinylthio)-4H-benzo[5,6]cyclohepta[1,2-d]thiazol-4-yl)-1-methyl-2,4(1H,3H)-pyrimidinedione

To a mixture of the product from example 44 step (viii) (0.20g) and 2-mercapto-pyridine (0.113g) in dry *N*,*N*-dimethylformamide (2ml) was added 60% sodium hydride (0.041g). The mixture was stirred for 16h then heated at 55°C for 2h. The reaction mixture was diluted with water and neutralised by the addition of acetic acid. The aqueous was extracted with dichloromethane and the combined organic extracts were dried (MgSO₄). The solvent was removed under reduced pressure and the residue purified by flash chromatography eluting with dichloromethane/3% ethanol. Yield: 0.15g.
MS: APCI(+ve): 469/467 (M+1, 100%).

### ii) (±)-5-(7-Chloro-2-mercapto-4H-benzo[5,6]cyclohepta[1,2-d]thiazol-4-yl)-1-methyl-3,4-dihydro-4-thioxo-1(2H)-pyrimidinone

The title compound was prepared from the product of step (i) (0.135g) by the method of example 34 step (iv). Purification was by flash chromatography eluting with dichloromethane/15% ethyl acetate. Yield: 0.038g.
MS: APCI(+ve): 408/406 (M+1, 100%).
¹H NMR: δ (DMSO) 13.43(s,1H), 12.68(s,1H), 7.55(m,3H), 7.04(d,1H), 6.82(s,1H), 6.69(d,1H), 5.83(s,1H), 3.19(s,3H).

### Example 68

### (±)-5-(7-Chloro-2-((imidazol-2-yl)thio)-4H-benzo[5,6]cyclohepta[1,2-d]thiazol-4-yl)-1-methyl-2,4-(1H,3H)-pyrimidinedione

A suspension of sodium hydride (60% in oil, 20mg) in *N,N*-dimethylformamide (2ml) was treated with a solution of 2-mercaptoimidazole (50mg) in *N,N*-dimethylformamide (2ml). After 1 hour at room temperature a solution of example 44 step (viii) (200mg) in *N,N*-dimethylformamide (2ml) was added. The reaction mixture was heated at 60°C for 18 hours, treated with a further equivalent of thiolate anion and heated at 60°C for a further 8 hours. The reaction mixture was partitioned between ethyl acetate and dilute acetic acid. The organic phase was washed with water and brine, dried (MgSO₄) and evaporated. The product was purified by flash chromatography eluting with 0-2% methanol in dichloromethane. Yield 0.13g.
MS: APCI (+ve) 456 (M+1, 100%)
1H NMR: δ(DMSO) 13.15 (br s, 1H), 11.28 (br s, 1H), 7.55-7.44 (m, 4H), 7.21 (s,1H), 6.99 (s,2H), 6.89 (s,1H), 5.47 (s,1H), 3.13 (s,3H).

### Example 69

### (±)-5-[7-Chloro-2-((1H-1,2,4-triazol-3-yl)thio)-4H-benzo[5,6]cyclohepta[1,2-d]thiazol-4-yl]-1-methyl-2,4(1H,3H)-pyrimidinedione

The title compound was prepared from example 44 step (viii) (200mg) and 3-mercapto-1,2,4-triazole (51mg) according to the method of example 68. Yield: 35mg.
MS: APCI (+ve) 457 (M+1, 100%)
1H NMR: δ(DMSO) 11.27 (br s, 1H), 8.79 (br s, 1H), 7.56-7.44 (m, 3H), 7.03 (s,2H), 5.50 (s,1H), 3.14 (s,3H).

### Example 70

### (±)-5-(7-Chloro-2-((imidazol-2-yl)thio)-4H-benzo[5,6]cyclohepta[1,2-d]thiazol-4-yl)-1-methyl-3,4-dihydro-4-thioxo-2(1H)-pyrimidinone

The product from example 68 (0.18g), and 1,2,4-triazole (0.3g) was dissolved in dry acetonitrile (10ml) and triethylamine(1ml) under nitrogen at 0 °C. Phosphorous oxychloride (0.072ml) was added and the mixture stirred for a further 5h. The reaction mixture was quenched with saturated sodium bicarbonate solution and extracted with dichloromethane (X3). The combined organic phases were collected, dried (MgSO₄) and solvents evaporated under reduced pressure to leave a colourless gummy solid.
A solution of this solid in methanol (10ml) was immediately added to a previously prepared saturated solution of hydrogen sulphide gas in methanol and triethylamine(prepared by bubbling hydrogen sulphide gas through a solution of triethylamine (0.115ml) in methanol (10ml) for 20 min at room temperature). After stirring for 20min the volatiles were renoved under reduced pressure leaving a yellow gum. Purification was by chromatography eluting with ethyl acetate to give the product as a yellow solid.
Yield: 0.16g
MS:APCI(+ve): 472(M+1, 100%), APCI(-ve);470(M-1, 100%)
1HNMR δ (CDCl₃): 9.62(bs, 1H), 7.56(d, 1H), 7.40(d+s, 2H), 7.11(s, 2H), 6.90(s, 1H), 6.80(dd, 2H), 6.39(s, 1H), 3.27(s, 3H)
MP: 190°C

### Example 71

### (±)-5-(1-(2-Acetyloxyethyl)-7-chloro-2-methyl-4H-benzo[5,6]cyclohepta[1,2-d]imidazol-4-yl)-1-methyl-3,4-dihydro-4-thioxo-2(1H)-pyrimidinone

### i) 7-Chloro-1-(2-hydroxyethyl)-2-methyl-4H-benzo[5,6]cyclohepta[1,2-d]imidazol-4-one

A mixture of the product from example 42 step (i) (1g) and ethanolamine(10ml) was stirred at 90 °C for 14 hours. The mixture was diluted with water (10ml) and the solid filtered. The solid was further washed with water and then dichloromethane to leave the subtitle product as a beige powder. Yield: 0.796g
MS: APCI(+ve): 289(M+1, 100%)

### ii) (±)-7-Chloro-1-(2-hydroxyethyl)-2-methyl-4H-benzo[5,6]cyclohepta[1,2-d]imidazol-4-ol

A mixture of the product of step (i) (0.79g) in ethanol (100ml) was treated with sodium borohydride (0.207g) followed by heating at reflux for 4hours. The mixture was treated with a few drops of acetone and then concentrated under reduced pressure to dryness. Used directly in the next step.
MS: APCI(+ve); 243 (M-17, 100%).

### iii) (±)-5-(7-Chloro-1-(2-hydroxyethyl)-2-methyl-4H-benzo[5,6]cyclohepta[1,2-d]imidazol-4-yl)-1-methyl-2,4(1H,3H)-pyrimidinedione

The subtitle compound was prepared from the product from step (ii) (1.85g) and 1-methyluracil (0.4g) according to the method of example 42 step (iv). Purification was by biotage chromatography on silica eluting with 5% methanol in dichloromethane with 1% 0.880 ammonia present. Yield: 0.7g
MS: APCI(+ve); 399 (M+1, 100%)

### iv) (±)-5-(1-(2-Acetyloxyethyl)-7-chloro-2-methyl-4H-benzo[5,6]cyclohepta[1,2-d]imidazol-4-yl)-1-methyl-2,4(1H,3H)-pyrimidinedione

A solution of the product of step (iv) (0.75g) in pyridine (5ml) was treated with acetic anhydride (0.35ml). After 4 hours the solvents were evaporated under reduced pressure. Purification was by biotage chromatography on silica eluting with 2-5% methanol in dichloromethane. Yield: 0.701g
MS: APCI(+ve); 441 (M+1, 100%), APCI(-ve); 439(M-1, 100%)

### v) (±)-5-(1-(2-Acetyloxyethyl)-7-chloro-2-methyl-4H-benzo[5,6]cyclohepta[1,2-d]imidazol-4-yl)-1-methyl-3,4-dihydro-4-thioxo-2(1H)-pyrimidinone

The title compound was prepared from the product of step (iv) (0.3g) and Lawesson's reagent (1.376g) according to the method of example 34 step (iv). Purification was by biotage chromatography on silica eluting with 3% methanol in dichloromethane with 1% 0.880 ammonia present to give a yellow glass. Yield: 0.259g
MS: APCI(+ve); 457(M+1, 100%), APCI(-ve); 455 (M-1, 100%)
1HNMR δ (DMSO): 12.62(s, 1H), 7.68(d, 1H), 7.46(s, 1H), 7.33(d, 1H), 7.25(s, 1H), 6.95(d, 1H), 6.86(d, 1H), 5.74(s, 1H), 4.41-4.11 (m, 4H), 3.22(s, 3H), 2.34(s, 3H), 1.89(s, 3H)

### Example 72

### (±)-5-(7-Chloro-1-(2-hydroxyethyl)-2-methyl-4H-benzo[5,6]cyclohepta[1,2-d]imidazol-4-yl)-1-methyl-3,4-dihydro-4-thioxo-2(1H)-pyrimidinone

The title compound was prepared from the product of example 71 step (v) (0.231 g) and lithium hydroxide monohydrate (0.045g) in methanol (20ml) and water (5ml) according to the method of example 1 step (ix) as a yellow powder. Yield: 0.144g
MS: APCI(+ve); 415 (M+1, 100%)
1HNMR δ(DMSO): 12.68(s, 1H), 7.73(d, 1H), 7.50(d, 1H), 7.39(dd, 1H), 7.29(s, 1H), 7.02(d, 1H), 6.88(d, 1H), 5.80(s, 1H), 5.02(t, 1H), 4.12-4.04( m, 2H), 3.72(-3.63(m ,2H), 3.26(s, 3H), 2.40(s, 3H),

### Example 73

### (±)-5-(9-Chloro-3-methylbenzo[f]oxepano[3,4-d]imidazol-6-yl)-1-methyl-3,4-dihydro-4-thioxo-2(1H)-pyrimidinone

### i) Ethyl 2-bromo-5-bromomethyl-1-methylimidazole-4-carboxylate

A mixture of ethyl 1,5-dimethylimidazole-4-carboxylate (*J. Org. Chem.*, 1982, **47,** 144) (3.58g) and N-bromosuccinimide (8.34g) in ethyl acetate was irradiated with a 500 Watt halogen lamp for 3h. The solvent was removed under reduced pressure and the residue purified by flash chromatography eluting with 5% ethyl acetate in dichloromethane.
Yield: 4.29g.
MS: APCI(+ve): 329/327 (M+1, 100%) /325

### ii) Ethyl 2-bromo-5-(3-chlorophenoxy)methyl -1-methylimidazole-4-carboxylate

To a solution of 3-chlorophenol (1.73g) in dry *N*,*N*-dimethylformamide (60ml) was added 60% sodium hydride (0.535g) in small portions. After the addition was complete the resultant solution was stirred at room temperature for 1h. The product of step (i) (4.0g) was added and the mixture was stirred at room temperature for 16h. The solvent was removed under reduced pressure and the residue was partitioned between water and dichloromethane. The organic phase was washed with saturated potassium carbonate solution (X2), saturated brine (X2) and dried (MgSO₄). The solvent was removed under reduced pressure. Yield: 4.8g.
MS: APCI(+ve): 377/375/373, 247/245 (100%).

### iii) 2-Bromo-5-(3-chlorophenoxy)methyl-1-methylimidazole-4-carboxylic acid

A mixture of the product from step (ii) (4.8g) and lithium hydroxide monohydrate (2.58g) in tetrahydrofuran/water 1:1 (40ml) was stirred at room temperature for 16h. The solvent was removed under reduced pressure, the residue triturated with water and the resultant solid collected by filtration. This solid was partitioned between ethyl acetate and dilute hydrochloric acid, the organic phase was dried (MgSO₄) and the solvent removed under reduced pressure. Yield 3.30g.
MS: APCI(+ve): 349/347/345, 331/329/327, 219 (100%)/217.

### iv) 4,9-Dichloro-3-methylbenzo[f]oxepano[3,4-d]imidazol-6-one

A mixture of the product from step (iii) (1g) and thionyl chloride (10ml) were heated at 70°C for 1h. The thionyl chloride was evaporated under reduced pressure and the residue dissolved in dichloromethane (60ml). Aluminium trichloride (1.55g) was added and the mixture was stirred under nitrogen for 48h. The reaction mixture was quenched with ice/water and the organic phase was separated. The aqueous layer was extracted with ethyl acetate, the combined organic extracts were dried (MgSO₄) and the solvent removed under reduced pressure. Yield 0.64g.
MS: APCI(+ve): 285/283 (M+1, 100%).

### v) (±)-4,9-Dichloro-3-methylbenzo[f]oxepano[3,4-d]imidazol-6-ol

To a solution of the product from step (iv) (0.64g) in dichloromethane/methanol 1:1 (20ml) was added sodium borohydride (0.11g). After 2h a further amount of sodium borohydride (0.11g) was added. After 1h water (20ml) was added and the organic solvents were removed under reduced pressure. The aqueous residue was extracted with ethyl acetate and the combined organic extracts were dried (MgSO₄) and the solvent evaporated under reduced pressure. Yield 0.52g.
MS: APCI(+ve): 287/285(M+1), 269/267(100%).

### vi) (±)-5-(4,9-Dichloro-3-methylbenzo[f]oxepano[3,4-d]imidazol-6-yl)-1-methyl-2,4(1H,3H)-pyrimidinedione

The subtitle compound was prepared from the product of step (v) (0.52g), 1-methyluracil (0.69g) and boron trifluoride diethyletherate (2.3ml) according to the method of example 42 step (iv). Purification was by flash chromatography eluting with 5% ethyl acetate in methanol. Yield: 0.08g.
MS: APCI(+ve): 397/395/393(M+1, 100%).

### vii) (±)-5-(9-Chloro-3-methylbenzo[f]oxepano[3,4-d]imidazol-6-yl)-1-methyl-2,4(1H,3H)-pyrimidinedione

The subtitle compound was prepared from the product of step (vi) (0.08g) and zinc dust (5g) according to the method of example 45. Purification was by flash chromatography eluting with dichloromethane/methanol/0.88 ammonia 93:5:2. Yield 0.068g.
MS: APCI(+ve): 361/359(M+1, 100%).

### vii) (±)-5-(9-Chloro-3-methylbenzo[f]oxepano[3,4-d]imidazol-6-yl)-1-methyl-3,4-dihydro-4-thioxo-2(1H)-pyrimidinone

The title compound was prepared from the product of step (vii) (0.053g) by the method of example 34 step (iv). Purification was by flash chromatography eluting with dichloromethane/methanol/0.88 ammonia 94:5:1. Yield: 0.023g.
MS: APCI(+ve): 377/375(M+1, 100%).
1H NMR: δ (DMSO): 12.66(s,1H), 7.66(d,1H), 7.55(s.1H), 7.51(s,1H), 7.20(d,1H), 7.14(dofd,1H), 5.64(s,1H), 5.52(d,1H), 4.92(d,1H), 3.48(s,3H), 3.28(s,3H).

### PHARMACOLOGICAL DATA

The following examples describe the assay used to determine how strongly the compounds of the invention bind to P2-purinoceptor 7-TM G-protein coupled receptors. The assay used a human P2Y₂ receptor clone which was isolated from HL60 cells cDNA and then stably transfected into a Jurkat cell line (using methods described in "Cloning and Characterisation of a Bovine P_{2Y} Receptor" Henderson *et al* (1995), 212, 2, 648-656; Parr *et al* Proc. Natl. Acad. Sci USA (1994), 91, 3275-3279 and Proc Natl Acad Sci USA (1994), 91, 13067). The cloned receptor mediates an increase in intracellular calcium in the cell line, which possesses no endogenous nucleotide receptor of its own.
1. The transfected Jurkat cells were maintained at a concentration of from about 1 x 10⁵ to 10 x 10⁵ cells/ml in RPMI containing 4% heat inactivated bovine serum, 2% penicillin/streptomycin and 1% glutamine. The cells were incubated at 37°C in an atmosphere of air with 5% CO₂.
   The cells were spun down at 1000 r.p.m. for 5 minutes and resuspended in 10ml basal salt solution (BSS) containing 125 mM of NaCl, 5 mM of KCl, 1 mM of MgCl, 1.5 mM of CaCl₂, 25 mM of HEPES, 5 mM of glucose and 1 mg/ml of bovine serum albumin, having a pH of 7.3. The concentration of cells was determined using a Technicon cell counter. From 0.75 x 10⁸ to 1 x 10⁸ cells were spun down, resuspended to a concentration of 3.3 x 10⁷ cells/ml in BSS and incubated with either 17 µM fluo-3AM or 17µM Fura-2AM at 37°C for 35 minutes with vigorous shaking. The dye used was dependent upon the fluorescence and absorption properties of the compounds of the invention. In general for compounds of formula (I) wherein Q¹ is a S atom, fluo-3AM was used and for compounds wherein Q¹ is an O atom, either fluo-3AM or fura-2AM were used. The cells were again spun down and washed once with the same volume of BSS before being resuspended in BSS to a concentration of 1 x 10⁶ cells/ml ready for testing.
   When fluo-3AM was used as the dye, the cell solution was left at room temperature to recover for approximately 30 minutes before testing.
   Fura-2AM loaded cells were divided into aliquots of about 10 ml and were warmed to 37°C for 10 minutes before testing.
   Calcium responses were measured on a SPEX Fluomax using 508 nm excitation and 525 nm emission wavelengths at room temperature for Fluo-3AM loaded cells and 340/380 nm excitation and 510 nm emission wavelengths for Fura-2AM loaded cells. Each cuvette contained 2 ml of cells and was stirred at high speed throughout the test. Basal fluorescence was measured for 5 seconds before 20µl of a 10⁻²-10⁻⁶M solution of the test compound in water was added to the 2ml solution of the cells. The response was calibrated by the addition of Triton-X-100 (68 µl, 10% solution) and then EGTA (180 µl, 0.5 M solution). For each compound the response was compared to that of UTP.
   or
2. The appropriate number of cells (3 x 10⁷ per plate) were spun down at 1000 r.p.m. for 5 minutes and resuspended in 10 ml of fresh RPMI at a concentration of 1 x 10⁷ cell.ml⁻ ¹. The concentration of cells was determined using a Coulter cell counter. The cells were then incubated with 5 µM Fluo3-AM (TEFLABS) for 45 min. with vigorous shaking, 2 U.ml⁻¹ of apyrase (Sigma) was added after 30 min. The cells were spun down again, washed twice with 50 ml of BSS (basal salt solution containing 125 mM NaCl, 5 mM KCl, 1 mM MgCl₂, 1.5 mM CaCl₂, 25 mM HEPES, 5 mM glucose and 1 mg.ml⁻¹ bovine serum albumin, pH ∼ 7.3) before being resuspended in BSS to a concentration of 3 x 10⁶ cells.ml⁻ ¹. The cells were then plated out (100 µl of cell suspension per well) in black, clear bottomed 96-well plates (Costar) before being spun down at 1000 r.p.m. for 5 minutes. Following a 15 min. recovery the plates were read at room temperature on a Fluorometric Imaging Plate Reader (FLIPR, Molecular Devices Corp.). Compound affinities were calculated either from inhibition of a UTP [A₅₀] or from the shift of a UTP concentration-effect curve.

The compounds exemplified have pA2 or PK_{B} values greater than 4.0.

## Claims

1. A compound of formula I or a salt thereof: 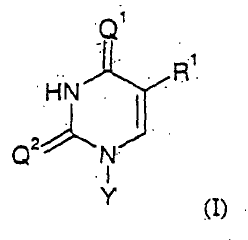 where
Y is hydrogen, C₁₋₄alkyl, C₁₋₄alkyl optionally substituted by hydroxy, alkoxy, amino; alkylamino, dialkylamino, phenyl, nitrogen and/or oxygen or optionally substituted by a C₃₋₈cycloalkyl ring which optionally contains 1 to 3 heteroatoms and optionally substituted by C₁₋₄ alkyl; or Y is a group of formula (i): 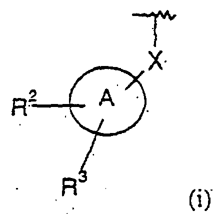
where X is a bond or CH₂
where
A is a pyridine, pyrimidine, thiazole, oxazole, thiophene, furan or pyridazine ring:
R¹ is a group of formula (ii): 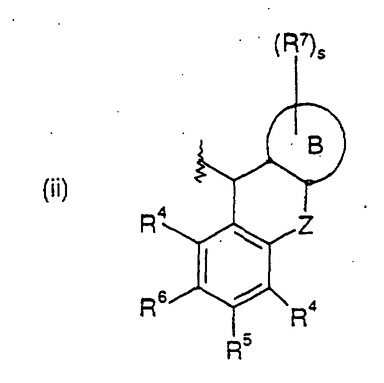 where
R⁴ is hydrogen, halogen, C₁₋₃alkoxy, C₁₋₃alkylthio or C₁₋₃alkyl (optionally substituted by one or more fluorine atoms);
R⁵ is hydrogen, hydroxy, halogen, C₁₋₃alkylthio, C₁₋₄alkyl (optionally substituted by one or more fluorine atoms), C₃₋₄cycloalkyl, MeOCH₂, MeSCH₂, phenyl, pyridyl, or C₁₋₃alkoxy; or R⁴ and R⁵ are -(CH₂)t- where t is 3 or 4 forming a fused ring;
R⁶ is hydrogen, halogen, C₁₋₃alkoxy, C₁₋₃alkylthio, or C₁₋₃alkyl(optionally substituted by one or more fluorine atoms); or R⁵ and R⁶ are -(CH₂)t- where t is 3 or 4 forming a fused ring;
R⁷ is hydrogen, hydroxy, C₁₋₃alkoxy, amino, hydroxyC₁₋₃alkyl, -NHC₁₋₃alkyl, -NC₁₋₃dialkyl, -NHC₃₋₈cycloalkyl, -N₃₋₈cycloalkyl, -NHphenyl, -NHC₁₋₃alkylphenyl, (heterocycle)C₁₋₃alkyl-, (heterocycle)C₁₋₃alkylthio-, (heterocycle)C₁₋₃alkyloxy-, (heterocycle)C₁₋₃alkylamino-, (heterocycle)thio-, (heterocycle)oxy-, (heterocycle)amino-, C₁₋₃alkylthio-, cyano, thiol, C₁₋₃alkyl (optionally substituted by one or more fluorine atoms), -C₁₋₃alkylamino, -C₁₋₃alkylaminoalkyl, carboxamidoC₁₋₃alkyl, acetoxyC₁₋₃alkyl, or C₃₋₄cycloalkyl;
S is 1 or 2;
B is a pyrrole, thiophene, furan, oxazole, thiazole, pyridine, pyrimidine, pyridazine, pyrazine or triazine ring;
Z is -CH=CH-, -CH₂CH₂-, or CH₂O ;
R² is hydrogen, NO₂, NH₂, N(C₁₋₆alkyl)₂, CO₂H, CH₂OH, halogen, CO₂C₁₋₆alkyl, C₁₋₈alkyl optionally interrupted by one or more oxygen, nitrogen or sulphur atoms and optionally substituted by CO₂H or R² is hydroxy, imidazol-1-ylCH₂-, phenyl optionally substituted by CH₂CO₂H or CONR⁹R¹⁰ where R⁹ and R¹⁰ are independently hydrogen, C₁₋₆alkyl optionally substituted by hydroxy or CO₂H and/or optionally interrupted by oxygen, nitrogen or sulphur;
R³ is hydrogen, R¹¹CO₂H, R¹¹PO(OH)₂, R¹²tetrazol-5-yl, COR¹³, NR¹⁴R¹⁵, CH₂NR¹⁶CH₂CO₂H, C₁₋₈alkyl optionally interrupted by one or more oxygen, sulphur or nitrogen atoms and optionally substituted by CO₂H or R³ is a group of formula (iii): 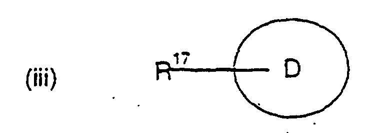
where D *is* a 4,5 or 6 membered saturated ring containing a nitrogen optionally substituted by hydroxy and substituted by CO₂H or CONH-het where het is tetrazol-5-yl or a thiazole or a thiadiazole ring substituted by CH₂CO₂H or D is a phenyl ring or a 5 membered aromatic heterocylic ring containing 1-3 heteroatoms selected from nitrogen, oxygen or sulphur optionally substituted by one or more groups selected from CF₃, CO₂H, CH₂OH, C₁₋₆alkyl optionally interrupted by one or more oxygen atoms, (CH₂)PCO₂H, C(CO₂H)=NOMe, tetrazol-5-yl, CH₂tetrazol-5-yl, CH₂CON(CH₂CO₂H)₂, or CH₂COR¹⁸;
where R¹¹ is OCH₂, (CH₂)p, SCH₂, CONHCH₂, NHCH(R¹⁹) or NR²⁰(CH₂)p;
R¹² is a bond, (CH₂)p, OCH₂, SCH₂, CONH, CONHCH₂, CONHCH₂CONH, NHCH₂CONH, NHCH(R⁹);
R¹³ is OH, N(CH₂CO₂H)₂, NHS(O)₂R²¹ or a group of formula (iv): 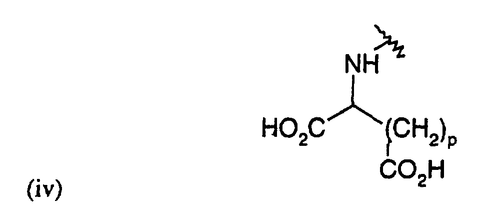
R¹⁴ and R¹⁵ are independently hydrogen, CH₂CO₂H, CHPh₂ or C(=S)CH₂CH₂CO₂H;
R¹⁶ is hydrogen, C₁₋₆alkyl or CO₂CH₂Ph;
R¹⁷ is a bond, sulphur atom, CONH, CH₂, CH₂O, OCH₂, a group -NR²²CH(CO₂H)CH₂-group or a group CONR²²(CH₂)pCONR²³ or NR²²(CH₂)pCONR²³;
R¹⁸ is a group of formula (iv) as defined above or a group of formula (v): 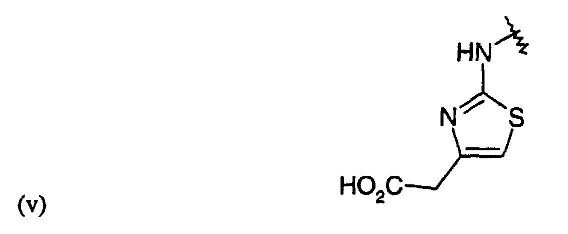
R¹⁹ is hydrogen, C₁₋₆ alkyl optionally substituted by hydroxy and/or optionally interrupted by oxygen, nitrogen or sulphur;
R²⁰ is hydrogen or C₁₋₆ alkyl;
p is 1 or 2;
R²¹ is NH₂ or C₁₋₆alkyl optionally interrupted by oxygen or nitrogen;
R²² and R²³ are independently hydrogen, C₁₋₆alkyl; and
Q¹ and Q² each independently represent an O or S atom.

2. A compound according to claim 1 in which Y is hydrogen, methyl, 2-(NN-dimethylamino)ethyl,2-(N(pyrrolidinyl)ethyl, 2-(pyrrolidinyl)methyl,, (1-methyl)-1H-imidazole-2-ylmethyl, hydroxyethyl, methoxyethyl a group of formula (i) and X is a bond or CH₂ group.

3. A compound according to claim 1 or 2 in which A is furan, oxazole, pyrimidine or phenyl.

4. A compound according to any one of claims 1 to 3 in which R⁴ is hydrogen and R⁶ is hydrogen or methyl.

5. A compound according to any one of claims I to 4 in which R⁵ is hydrogen, chlorine, hydroxy or C₁₋₆ alkyl.

6. A compound according to any one of claims 1 to 5 in which R⁷ is C₁₋₃ alkyl, hydrogen, chlorine, methoxy, methylthio, amino, methylamino, dimethylamino, pyrrolidin-1-yl, N-4-methyl-piperazin-1-yl, 2-(2-(4-morpholinyl)ethylamino, cyclopropyl, benzylamino, cyclobutylamino, cyclopropylamino, imidazol-2-ylthio, thiadiazol-2-ylthio, thiol, triazol-2-ylthio, imidazol-1-ylethoxy, acetoxyethyl, hydroxyethyl or imidazol-4-ylethyl.

7. A compound according to any one of claims 1 to 6 in which B is pyridine, oxazole, thiazole or imidazole.

8. A compound according to any one of claims 1 to 7 in which Z is CH=CH, CH₂CH₂ or -CH₂O-.

9. A compound according to any one of claims 1 to 8 in which R² is hydrogen or CO₂H.

10. A compound according to any one of claims 1 to 9 in which R³ is hydrogen, a group of formula (iii) where D is a 4-membered saturated ring containing a nitrogen atom and substituted by CO₂H, or D is a 5-membered aromatic heterocycle containing 2 or 3 heteroatoms selected from nitrogen and sulphur and optionally substituted by CH₂CO₂H or CF₃ and R¹³ is a bond, CONH, NHCH₂CONH or CH₂.

11. A compound according to any one of claims 1 to 10 in which Q¹ is S and Q² is O or S.

12. A compound according to claim 1 which is:
(±)-5-[[5-(2-Ethyl-7-methyl-4*H*-benzo[5,6]cyclohepta[1,2-*d*]oxazol-4-yl)-3,4-dihydro-2,4-dioxo-1(2*H*)-pyrimidinyl]methyl]-2-furancarboxylic acid,
(±)-2-[[5-(2-Ethyl-7-methyl-4*H*-benzo[5,6]cyclohepta[1,2-*d*]oxazol-4-yl)-3,4-dihydro-2-oxo-4-thioxo-1(2*H*)-pyrimidinyl]methyl]-4-oxazolecarboxylic acid,
(±)-2-[2-[[4-[5-(7-Ethyl-2-methyl-4*H*-benzo[5,6]cyclohepta[1,2-*d*]oxazol-4-yl)-3,4-dihydro-2,4-dioxo-1(2*H*)-pyrimidinyl]pyrimidin-2-yl]amino]acetylamino]-4- thiazoleacetic acid,
(±)-5-[[3,4-Dihydro-5-(2-methyl-4*H*-benzo[5,6]cyclohepta[1,2-*d*]oxazol-4-yl)-2-oxo-4-thioxo-1(2*H*)-pyrimidinyl]methyl]-2-furancarboxylic acid,
(±)-*N*-[4-[3,4-Dihydro-5-(7-hydroxy-2-methyl-4*H*-benzo[5,6]cyclohepta[1,2-*d*]oxazol-4-yl)-2,4-dioxo-1(2*H*)-pyrimidinyl]pyrimidin-2-yl]-3-azetidinecarboxylic acid,
(±)-2-[[5-(2-Ethyl-7-methyl-4*H*-benzo[5,6]cyclohepta[1,2-*d*]oxazol-4-yl)-3,4-dihydro-2-oxo-4-thioxo-1(2*H*)-pyrimidinyl]methyl]-*N*-[5-(trifluoromethyl)-1*H*-1,2,4-triazol-3-yl]-4-oxazolecarboxamide,
(±)-3-[[5-(7-Ethyl-2-methyl-4*H*-benzo[5,6]cyclohepta[1,2-*d*]oxazol-4-yl)-3,4-dihydro-2-oxo-4-thioxo-1(2*H*)-pyrimidinyl]methyl]-5-[imidazol-1-ylmethyl]benzoic acid,
(±)-2-[[5-(7-Ethyl-2-methyl-4*H*-benzo[5,6]cyclohepta[1,2-*d*]oxazol-4-yl)-3,4-dihydro-2-oxo-4-thioxo-1(2*H*)-pyrimidinyl]methyl]-4-oxazolecarboxylic acid,
(±)-3-[[5-(7-Ethyl-2-methyl-4*H*-benzo[5,6]cyclohepta[1,2-*d*]oxazol-4-yl)-3,4-dihydro-2-oxo-4-thioxo-1(2*H*)-pyrimidinyl]methyl]benzoic acid,
(±)-3-[[5-(7-Ethyl-2-methyl-4*H*-benzo[5,6]cyclohepta[1,2-*d*]oxazol-4-yl)-3,4-dihydro-2-oxo-4-thioxo-1(2*H*)pyrimidinyl]methyl]-*N*-[5-(trifluoromethyl)-1*H*-1,2,4-triazol-3-yl]benzenecarboxamide,
(±)-3-[[5-(7-Ethyl-9,10-dihydro-2-methyl-4*H*-benzo[5,6]cyclohepta[1,2-*d*]oxazol-4-yl)-3,4-dihydro-2-oxo-4-thioxo-1(2*H*)-pyrimidinyl]methyl]benzoic acid,
(±)-3-[[5-(7-Ethyl-2-methyl-4*H*-benzo[5,6]cyclohepta[1,2-*d*]thiazol-4-yl)-3,4-dihydro-2,4-dioxo-1(2*H*)-pyrimidinyl]methyl]benzoic acid,
(±)-3-[[5-(2,7-Dimethyl-10*H*-benzo[4,5]cyclohepta[1,2-*d*]oxazol-10-yl)-3,4-dihydro-2-oxo-4-thioxo-1(2*H*)-pyrimidinyl]methyl]benzoic acid,
(±)-5-(2,7-Dimethyl-4*H*-benzo[5,6]cyclohepta[1,2-*d*]oxazol-4-yl)-1-(3-methyl-5-(5-trifluoromethyl)- 1*H*-1,2,4-triazol-3-yl)phenylmethyl-3,4-dihydro-4-thioxo-2(1*H*)pyrimidinone,
(±)-3-[5-(2,7-Dimethyl-4*H*-benzo[5,6]cyclohepta[1,2-*d*]oxazol-4-yl)-3,4-dihydro-2-oxo-4-thioxo-1(2*H*)-pyrimidinylmethyl]benzoic acid,
(±)-1-Methyl-5-(2,7-dimethyl-4*H*-benzo[5,6]cyclohepta[1,2-*d*]oxazol-4-yl)-3,4-dihydro-4-thioxo-2(1*H*)-pyrimidinone,
(±)-1-Methyl-5-(2,6-dimethyl-4*H*-benzo[5,6]cyclohepta[1,2-*d*]oxazol-4-yl)-3,4-dihydro-4-thioxo-2(1*H*)-pyrimidinone,
(±)-1-Methyl-5-(2,7-dimethyl-4*H*-benzo[5,6]cyclohepta[1,2-*d*]thiazol-4-yl)-3,4-dihydro-4-thioxo-2(1*H*)-pyrimidinone,
(±)-1-Methyl-5-(2,7-dimethyl-10*H*-benzo[4,5]cyclohepta[1,2-*d*]oxazol-10-yl)-2,4-(1*H*,3*H*)-pyrimidinedione,
(±)-1-Methyl-5-(2,7-dimethyl-10H-benzo[4,5]cyclohepta[1,2-*d*]oxazol-10-yl)-3,4-dihydro-2-oxo-4-thioxo-1(2H)-pyrimidine,
(±)-1-Methyl-5-(2,7-dimethyl-10*H*-benzo[4,5]cyclohepta[1,2-*d*]thiazol-10-yl)-3,4-dihydro-4-thioxo-2(1*H*)-pyrimidinone,
(±)-5-[8-Methyl-5*H*-benzo[4,5]cyclohepta[1,2-b]pyridin-5-yl]-3,4-dihydro-4-thioxo-2(1*H*)-pyrimidinone,
(±)-1-Methyl-5-[8-Methyl-5*H*-benzo[4,5]cyclohepta[1,2-b]pyridin-5-yl]-3,4-dihydro-4-thioxo-2(1*H*)-pyrimidine,
(±)-1-Methyl-5-[2,7-dimethyl-4*H*-benzo[5,6]cyclohepta[1,2-d]thiazol-4-yl]-2,4(1*H*,3*H*)-pyrimidinedione,
(±)-5-[7-Chloro-2-methyl-4*H*-benzo[5,6]cyclohepta[1,2-*d*]oxazol-4-yl]-1-methyl-2,4(1*H*,3*H*)-pyrimidinedione,
(±)-5-[7-Chloro-2-methyl-4*H*-benzo[5,6]cyclohepta[1,2-*d*]oxazol-4-yl]-1-methyl-3,4-dihydro-4-thioxo-2(1*H*)-pyrimidinone,
(S)- 5-[7-Chloro-2-methyl-4*H*-benzo[5,6]cyclohepta[1,2-*d*]oxazol-4-yl]-1-methyl-3,4-dihydro-4-thioxo-2(1*H*)-pyrimidinone,
(±) -5-(7-Chloro-2-(2-(imidazol-1-yl)ethoxy)-4*H*-benzo[5,6]cyclohepta[1,2-*d*]thiazol-4-yl)-1-methyl-3,4-dihydro-4-thioxo-2(1*H*)-pyrimidinone,
(±)-1-[(1-Methyl)-1*H*-imidazol-2-ylmethyl]-5-(2,6-dimethyl-4*H*-benzo[5,6]cyclohepta[1,2-*d*]oxazol-4-yl)-3,4-dihydro-4-thioxo-2(1*H*)-pyrimidinone,
(±)-5-(2,7-Dimethyl-4*H*-benzo[5,6]cyclohepta[1,2-*d*]thiazol-4-yl)-1-(2-(*N*-pyrrolidinyl)ethyl)-3,4-dihydro-4-thioxo-2(1*H*)-pyrimidinone,
(±)-5-(2-Ethyl-7-methyl-4*H*-benzo[5,6]cyclohepta[1,2-*d*]thiazol-4-yl)-3,4-dihydro-4-thioxo-2(1*H*)-pyrimidinone,
(±) 5-(2-Ethyl-7-methyl-4*H*-benzo[5,6]cyclohepta[1,2-*d*]thiazol-4-yl)-1-*N*-methyl)imidazol-2-ylmethyl)-3,4-dihydro-4-thioxo-2(1*H*)-pyrimidinone,
(±)-5-(2-Ethyl-7-methyl-4*H*-benzo[5,6]cyclohepta[1,2-*d*]thiazol-4-yl)-1-(2-(*N*,*N*-dimethyl)ethyl)-3,4-dihydro-4-thioxo-2(1*H*)-pyrimidinone,
(±)-5-[2-methyl-4*H*-benzo[5,6]cyclohepta[1,2-*d*]oxazol-4-yl)-1-methyl-3,4-dihydro-4-thioxo-2(1*H*)-pyrimidinone,
(±)-1-Methyl-5-(2-methyl-7-ethyl-4*H*-benzo[5,6]cyclohepta[1,2-*d*]thiazol-4-yl)-3,4-dihydro-4-thioxo-2(1*H*)-pyrimidinone,
(±)-1-Methyl-5-(2-methyl-7-n-propyl-4*H*-benzo[5,6]cyclohepta[1,2-*d*]thiazol-4-yl)-3,4-dihydro-4-thioxo-2(1*H*)-pyrimidinone,
(±)-1-Methyl-5-(2-methyl-4*H*-benzo[5,6]cyclohepta[1,2-*d*]imidazol-4-yl)-3,4-dihydro-4-thioxo-2(1*H*)-pyrimidinone,
(±)-5-(7-Chloro-2-methyl-4*H*-benzo[5,6]cyclohepta[1,2-*d*]thiazol-4-yl)-3,4-dihydro-4-thioxo-2(1*H*)-pyrimidinone,
(5S, 2R)-5-(7-Chloro-2-methyl-4*H*-benzo[5,6]cyclohepta[1,2-*d*]thiazol-4-yl)-1-(2-pyrrolidinylmethyl)-3,4-dihydro-4-thioxo-2(1H)-pyrimidinone,
(5S,2S)-5-(7-Chloro-2-methyl-4*H*-benzo[5,6]cyclohepta[1,2-*d*]thiazol-4-yl)-1-(2-(pyrrolidinylmethyl)-3,4-dihydro-4-thioxo-2(1*H*)-pyrimidinone,
(±)-5-(7-Chloro-1-((imidazol-4-yl)ethyl)-2-methyl-4*H*-benzo[5,6]cyclohepta[1,2-*d*]imidazol-4-yl)-1-methyl-3,4-dihydro-4-thioxo-2(1*H*)-pyrimidinone,
(±)-5-(7-Chloro-1,2-dimethyl-4*H*-benzo[5,6]cyclohepta[1,2-*d*]imidazol-4-yl)-1-methyl-3,4-dihydro-4-thioxo-2(1*H*)-pyrimidinone,
(S)-5-(7-Chloro-1,2-dimethyl-4*H*-benzo[5,6]cyclohepat[1,2-*d*]imidazol-4-yl)-1-methyl-3,4-dihydro-4-thioxo-2(1*H*)-pyrimidinone,
(±)-5-(2,7-Dichloro-4*H*-benzo[5,6]cyclohepta[1,2-*d*]thiazol-4-yl)-1-methyl-2,4(1*H*,3*H*)-pyrimidinedione,
(±)-5-(7-Chloro-4*H*-benzo[5,6]cyclohepta[1,2-*d*]thiazol-4-yl)-1-methyl-2,4(1*H*,3*H*)-pyrimidinedione
(±)-5-(7-Chloro-2-methoxy-4*H*-benzo[5,6]cyclohepta[1,2-*d*]thiazol-4-yl)-1-methyl-2,4(1*H*,3*H*)-pyrimidinedione,
(±)-5-(7-Chloro-2-methylthio-4*H*-benzo[5,6]cyclohepta[1,2-*d*]thiazol-4-yl)-1-methyl-2,4(1*H*,3*H*)-pyrimidinedione,
(±)-5-(2-Amino-7-chloro-4*H*-benzo[5,6]cyclohepta[1,2-*d*]thiazol-4-yl)-1-methyl-2,4(1*H*,3*H*)-pyrimidinedione,
(±)-5-(7-Chloro-2-methylamino-4*H*-benzo[5,6]cyclohepta[1,2-*d*]thiazol-4-yl)-1-methyl-2,4(1*H*,3*H*)-pyrimidinedione,
(±)-5-(7-Chloro-2-dimethylamino-4*H*-benzo[5,6]cyclohepta[1,2-*d*]thiazol-4-yl)-1-methyl-2,4(1*H*,3*H*)-pyrimidinedione,
(±)-5-(7-Chloro-2-(pyrrolidin-1-yl)-4*H*-benzo[5,6]cyclohepta[1,2-*d*]thiazol-4-yl)-1-methyl-3,4-dihydro-4-thioxo-2( 1*H*)-pyrimidinone,
(±)-5-(7-Chloro-2-(*N*4-methyl-piperazin-1-yl))-4*H*-benzo[5,6]cyclohepta[1,2-*d*]thiazol-4-yl)-1-methyl-3,4-dihydro-4-thioxo-2(1H)-pyrimidinone,
(±)-5-(7-Chloro-2-(2-(4-morpholinyl)ethylamino)-4*H*-benzo[5,6]cyclohepta[1,2-*d*]thiazol-4-yl)-1-methyl-3,4-dihydro-4-thioxo-2(1*H*)-pyrimidinone,
(±)-5-(7-Chloro-4*H*-benzo[5,6]cyclohepta[1,2-*d*]thiazol-4-yl)-1-methyl-3,4-dihydro-4-thioxo-2(1*H*)-pyrimidinone,
(S)-5-(7-Chloro-4*H*-benzo[5,6]cyclohepta[1,2-*d*]thiazol-4-yl)-1-methyl-3,4-dihydro-4-thioxo-2(1*H*)-pyrimidinone,
(±)-5-(2-Amino-7-Chloro-4*H*-benzo[5,6]cyclohepta[1,2-*d*]thiazol-4-yl)-1-methyl-3,4-dihydro-4-thioxo-2(1*H*)-pyrimidinone,
(±)-5-(7-chloro-2-methyl-4*H*-benzo[5,6]cyclohepta[1,2-*d*]thiazol-4-yl)-1-methyl-3,4-dihydro-4-thioxo-2(1*H*)-pyrimidinone,
(±)-5-(7-Chloro-1-ethyl-2-methyl-4*H*-benzo[5,6]cyclohepta[1,2-*d*]imidazol-4-yl)-1-methyl-3,4-dihydro-4-thioxo-2(1*H*)-pyrimidinone,
(±)-5-(7-Chloro-1-cyclopropyl-2-methyl-4*H*-benzo[5,6]cyclohepta[1,2-*d*]imidazol-4-yl)-1-methyl-3,4-dihydro-4-thioxo-2(1*H*)-pyrimidinone,
(±)-5-(7-Chloro-2-methyl-4*H*-benzo[5,6]cyclohepta[1,2-*d*]thiazol-4-yl)-1-(2-hydroxyethyl-3,4-dihydro-4-thioxo-2(1H)-pyrimidinone,
(S)-5-(7-chloro-2-methyl-4*H*-benzo[5,6]cyclohepta[1,2-*d*]thiazol-4-yl)-1-(2-hydroxyethyl)-3,4-dihydro-4-thioxo-2(1*H*)-pyrimidinone,
(±)-5-(7-chloro-2-methyl-4*H*-benzo[5,6]cyclohepta[1,2-*d*]thiazol-4-yl)-1-(2-methoxyethyl)-3,4-dihydro-4-thioxo-2(1*H*)-pyrimidinone,
(±)-5-(7-Chloro-2-[(phenylmethyl)amino]-4*H*-benzo[5,6]cyclohepta[1,2-*d*]thiazol-4-yl)-1-methyl-3,4-dihydro-4-thioxo-2(1*H*)-pyrimidinone,
(±)-5-(7-Chloro-2-(cyclobutylamino)-4*H*-benzo[5,6]cyclohepta[1,2-*d*]thiazol-4-yl)-1-methyl-3,4-dihydro-4-thioxo-2(1H)-pyrimidinone,
(±)-5-(7-Chloro-2-(cyclopropylamino)-4*H*-benzo[5,6]cyclohepta[1,2-*d*]thiazol-4-yl)-1-methyl-3,4-dihydro-4-thioxo-2(1*H*)-pyrimidinone,
(±)-5-(7-Chloro-2-(1,3,4-thiadiazolylthio)-4*H*-benzo[5,6]cyclohepta[1,2-*d*]thiazol-4-yl)-1-methyl-2,4(1*H*,3*H*)-pyrimidinedione,
(±)-5-(7-Chloro-2-mercapto-4*H*-benzo[5,6]cyclohepta[1,2-*d*]thiazol-4-yl)-1-methyl-3,4-dihydro-4-thioxo-2(1*H*)-pyrimidinone,
(±)-5-(7-Chloro-2-((imidazol-2-yl)thio)-4*H*-benzo[5,6]cyclohepta[1,2-*d*]thiazol-4-yl)-1-methyl-2,4-(1*H*,3*H*)-pyrimidinedione,
(±)-5-[7-Chloro-2-((1*H*-1,2,4-triazol-3-yl)thio)-4*H*-benzo[5,6]cyclohepta[1,2-*d*]thiazol-4-yl]-1-methyl-2,4(1*H*,3*H*)-pyrimidinedione,
(±)-5-(7-Chloro-2-((imidazol-2-yl)thio)-4*H*-benzo[5,6]cyclohepta[1,2-*d*]thiazol-4-yl)-1-methyl-3,4-dihydro-4-thioxo-2(1*H*)-pyrimidinone,
(±)-5-(1-(2-Acetyloxyethyl)-7-chloro-2-methyl-4*H*-benzo[5,6]cyclohepta[1,2-*d*]imidazol-4-yl)-1-methyl-3,4-dihydro-4-thioxo-2(1*H*)-pyrimidinone,
(±)-5-(7-Chloro-1-(2-hydroxyethyl)-2-methyl-4*H*-benzo[5,6]cyclohepta[1,2-*d*]imidazol-4-yl)-1-methyl-3,4-dihydro-4-thioxo-2(1*H*)-pyrimidinone,
(±)-5-(9-Chloro-3-methylbenzo[*f*]oxepano[3,4-*d*]imidazol-6-yl)-1-methyl-3,4-dihydro-4-thioxo-2(1*H*)-pyrimidinone,
and pharmaceutically acceptable salts thereof.

13. A compound according to any one of claims 1 to 12 for use in therapy.

14. A compound according to any one of claims 1 to 12 for use as an anti-inflammatory agent.

15. A pharmaceutical composition comprising a compound of formula I or a salt or solvate thereof as defined in any one of claims 1 to 12 in association with a pharmaceutically acceptable adjuvant, diluent or carrier.

16. A process for the preparation of compounds of formula I which comprises:
(a) reacting a compound of formula (II): 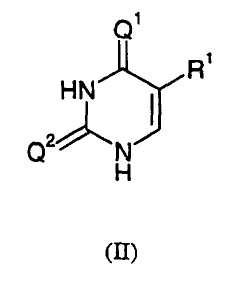 where Q¹ and Q² are as defined in formula (I) and R¹ is as defined in formula (I) or is a protected derivative thereof with a compound of formula (III): 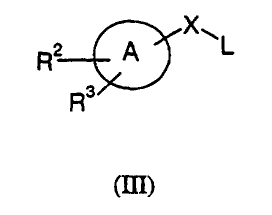 where R², R³ and A are as defined in formula (I) or are protected derivatives thereof, X is as defined in formula (I) and L is a leaving group, or
(b) reacting a compound of formula (IV): 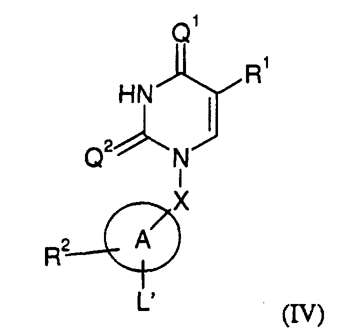 where Q¹, Q², R¹ and X are as defined in formula (I), R² and A are as defined in formula (I) or are protected derivatives thereof and L' is a leaving group with a compound of formula (V), (VI) or (VII):
HNR²²-(CH₂)ₚ₋CONR²³-D (V)
HNR²²-CH(CO₂H)-CH₂-D (VI) 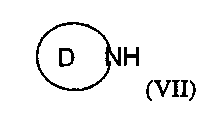
where R²², R²³ and p are as defined in formula (I) and D is as defined in formula (I) or is a protected derivative thereof, or
(c) when R³ is a group -R¹¹-CO₂H and R¹¹ is SCH₂ or NR²⁰(CH₂)ₚ, reacting a compound of formula (iv) as defined above with a compound H-R¹¹-CO₂R²⁴ where R¹¹ is SCH₂ or NR²⁰(CH₂)ₚ and R²⁴ is hydrogen or an ester forming group;
and optionally thereafter (a), (b) or (c) in any order:
• removing any protecting groups
• converting the compound of formula (I) into a further compound of formula (I)
• forming a salt

## Patentansprüche

1. Verbindungen der Formel I oder deren Salze: 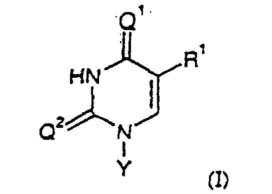 wobei
Y für Wasserstoff, für C₁₋₄Alkyl oder für gegebenenfalls durch Hydroxy, Alkoxy, Amino, Alkylamino, Dialkylamino, Phenyl, Stickstoff und/oder Sauerstoff substituiertes oder gegebenenfalls durch einen C₃₋₈-Cycloalkylring, der gegebenenfalls 1 bis 3 Heteroatome enthält und gegebenenfalls durch C₁₋₄-Alkyl substituiert ist, substituiertes C₁₋₄-Alkyl steht; oder Y für eine Gruppe der Formel (i) steht: 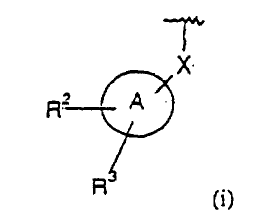
wobei X für eine Bindung oder CH₂ steht,
wobei A für einen Pyridin-, Pyrimidin-, Thiazol-, Oxazol-, Thiophen-, Furan- oder Pyridazinring steht;
R¹ für eine Gruppe der Formel (ii) steht: 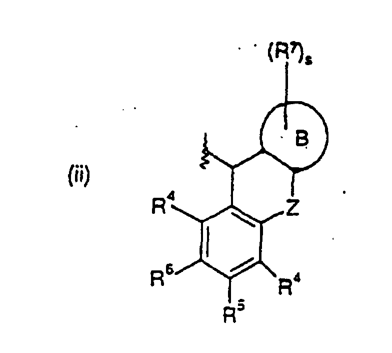 wobei
R⁴ für Wasserstoff, Halogen, C₁₋₃-Alkoxy, C₁₋₃-Alkylthio oder C₁₋₃-Alkyl (gegebenenfalls substituiert durch ein oder mehrere Fluoratome) steht;
R⁵ für Wasserstoff, Hydroxy, Halogen, C₁₋₃-Alkylthio, C₁₋₄-Alkyl (gegebenenfalls substituiert durch ein oder mehrere Fluoratome), C₃₋₄-Cycloalkyl, MeOCH₂, MeSCH₂, Phenyl, Pyridyl oder C₁₋₃-Alkoxy steht;
oder R⁴ und R⁵ für -(CH₂)t- stehen, wobei t für 3 oder 4 steht, und einen ankondensierten Ring bilden;
R⁶ für Wasserstoff, Halogen, C₁₋₃-Alkoxy, C₁₋₃-Alkylthio oder C₁₋₃-Alkyl (gegebenenfalls substituiert durch ein oder mehrere Fluoratome) steht; oder R⁵ und R⁶ für -(CH₂)t- stehen, wobei t für 3 oder 4 steht, und einen ankondensierten Ring bilden;
R⁷ für Wasserstoff, Hydroxy, C₁₋₃-Alkoxy, Amino, Hydroxy-C₁₋₃-alkyl, -NH-C₁₋₃-Alkyl, -N-C₁₋₃-Dialkyl, -NH-C₃₋₈-Cycloalkyl, -N-C₃₋₈-Cycloalkyl, -NH-Phenyl, -NH-C₁₋₃-Alkylphenyl, (Heterozyklus) -C₁₋₃-alkyl, (Heterozyklus) -C₁₋₃-alkylthio, (Heterozyklus) -C₁₋₃-alkyloxy, (Heterozyklus)-C₁₋₃-alkylamino, (Heterozyklus)-thio, (Heterozyklus)-oxy, (Heterozyklus)-amino, C₁₋₃-Alkylthio, Cyano, Thiol, C₁₋₃-Alkyl (gegebenenfalls substituiert durch ein oder mehrere Fluoratome), C₁₋₃-Alkylamino, C₁₋₃-Alkylaminoalkyl, Carboxamido-C₁₋₃-alkyl, Acetoxy-C₁₋₃-alkyl oder C₃₋₄-Cycloalkyl steht;
S für 1 oder 2 steht;
B für einen Pyrrol-, Thiophen-, Furan-, Oxazol-, Thiazol-, Pyridin-, Pyrimidin-, Pyridazin-, Pyrazin- oder Triazinring steht;
Z für -CH=CH-, -CH₂CH₂- oder CH₂O steht;
R² für Wasserstoff, NO₂, NH₂, N(C₁₋₆-Alkyl)₂, CO₂H, CH₂OH, Halogen, CO₂-C₁₋₆-Alkyl oder C₁₋₈-Alkyl, gegebenenfalls unterbrochen durch ein oder mehrere Sauerstoff-, Stickstoff- oder Schwefelatome und gegebenenfalls substituiert durch CO₂H, steht, oder R² für Hydroxy, Imidazol-1-yl-CH₂- oder Phenyl, gegebenenfalls substituiert durch CH₂CO₂H oder CONR⁹R¹⁰, wobei R⁹ und R¹⁰ unabhängig voneinander für Wasserstoff, C₁₋₆-Alkyl, gegebenenfalls substituiert durch Hydroxy oder CO₂H und/oder gegebenenfalls unterbrochen durch Sauerstoff, Stickstoff oder Schwefel, stehen, steht;
R³ für Wasserstoff, R¹¹CO₂H, R¹¹PO(OH)₂, R¹²-Tetrazol-5-yl, COR¹³, NR¹⁴R¹⁵, CH₂NR¹⁶CH₂CO₂H oder C₁₋₈-Alkyl, gegebenenfalls unterbrochen durch ein oder mehrere Sauerstoff-, Schwefel- oder Stickstoffatome und gegebenenfalls substituiert durch CO₂H, steht, oder R³ für eine Gruppe der Formel (iii) steht: 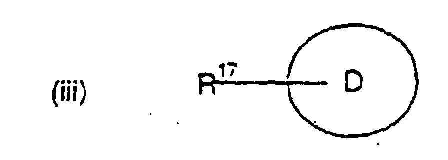
wobei D für einen 4-, 5- oder 6-gliedrigen gesättigten, ein Stickstoffatom enthaltenden Ring, der gegebenenfalls durch Hydroxy substituiert ist und durch CO₂H oder CONH-Het substituiert ist, wobei Het für Tetrazol-5-yl oder einen Thiazol- oder einen Thiadiazolring, der durch CH₂CO₂H substituiert ist, steht, steht oder D für einen Phenylring oder einen 5-gliedrigen aromatischen heterozyklischen Ring mit 1-3 Heteroatomen ausgewählt aus Stickstoff, Sauerstoff oder Schwefel, der gegebenenfalls durch eine oder mehrere Gruppen ausgewählt aus CF₃, CO₂H, CH₂OH, C₁₋₆-Alkyl, das gegebenenfalls durch ein oder mehrere Sauerstoffatome unterbrochen ist, (CH₂)pCO₂H, C(CO₂H)=NOMe, Tetrazol-5-yl, CH₂-Tetrazol-5-yl, CH₂CON (CH₂CO₂H)₂ oder CH₂COR¹⁸ substituiert ist, steht;
wobei R¹¹ für OCH₂, (CH₂)p, SCH₂, CONHCH₂, NHCH(R¹⁹) oder NR²⁰(CH₂)p steht;
R¹² für eine Bindung, (CH₂)p, OCH₂, SCH₂, CONH, CONHCH₂, CONHCH₂CONH, NHCH₂CONH oder NHCH(R⁹) steht;
R¹³ für OH, N(CH₂CO₂H)₂, NHS(O)₂R²¹ oder eine Gruppe der Formel (iv) steht: 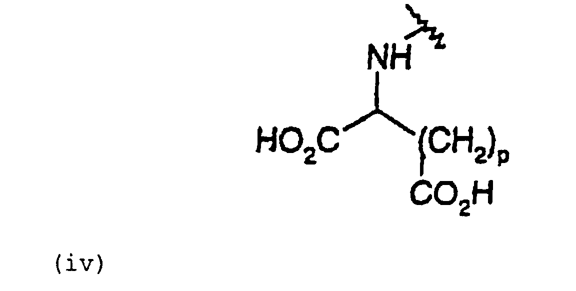
R¹⁴ und R¹⁵ unabhängig voneinander für Wasserstoff, CH₂CO₂H, CHPh₂ oder C(=S)CH₂CH₂CO₂H stehen;
R¹⁶ für Wasserstoff, C₁₋₆-Alkyl oder CO₂CH₂Ph steht;
R¹⁷ für eine Bindung, ein Schwefelatom, CONH, CH₂, CH₂O, OCH₂, eine Gruppe -NR²²CH(CO₂H)CH₂- oder eine Gruppe CONR²²(CH₂)pCONR²³ oder NR²²(CH₂)pCONR²³ steht;
R¹⁸ für eine wie oben definierte Gruppe der Formel (iv) oder eine Gruppe der Formel (v) steht: 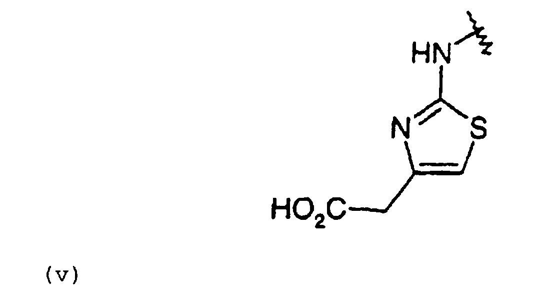
R¹⁹ für Wasserstoff, C₁₋₆-Alkyl, gegebenenfalls substituiert durch Hydroxy und/oder gegebenenfalls unterbrochen durch Sauerstoff, Stickstoff oder Schwefel, steht;
R²⁰ für Wasserstoff oder C₁₋₆-Alkyl steht;
p für 1 oder 2 steht;
R²¹ für NH₂ oder C₁₋₆-Alkyl, gegebenenfalls unterbrochen durch Sauerstoff oder Schwefel, steht;
R²² und R²³ unabhängig voneinander für Wasserstoff oder C₁₋₆-Alkyl stehen; und
Q¹ und Q² jeweils unabhängig voneinander für ein O- oder S-Atom stehen.

2. Verbindungen nach Anspruch 1, in denen Y für Wasserstoff, Methyl, 2-(N,N-Dimethylamino)ethyl, 2-(N-Pyrrolidinyl)ethyl, 2-(Pyrrolidinyl)methyl, (1-Methyl)-1H-imidazol-2-ylmethyl, Hydroxyethyl, Methoxyethyl oder eine Gruppe der Formel (i) steht und X für eine Bindung oder eine CH₂-Gruppe steht.

3. Verbindungen nach Anspruch 1 oder 2, in denen A für Furan, Oxazol, Pyrimidin oder Phenyl steht.

4. Verbindungen nach einem der Ansprüche 1 bis 3, in denen R⁴ für Wasserstoff und R⁶ für Wasserstoff oder Methyl steht.

5. Verbindungen nach einem der Ansprüche 1 bis 4, in denen R⁵ für Wasserstoff, Chlor, Hydroxy oder C₁₋₆-Alkyl steht.

6. Verbindungen nach einem der Ansprüche 1 bis 5, in denen R⁷ für C₁₋₃-Alkyl, Wasserstoff, Chlor, Methoxy, Methylthio, Amino, Methylamino, Dimethylamino, Pyrrolidin-1-yl, N-4-Methylpiperazin-1-yl, 2-(2-(4-Morpholinyl)ethylamino, Cyclopropyl, Benzylamino, Cyclobutylamino, Cyclopropylamino, Imidazol-2-ylthio, Thiadiazol-2-ylthio, Thiol, Triazol-2-ylthio, Imidazol-1-ylethoxy, Acetoxyethyl, Hydroxyethyl oder Imidazol-4-ylethyl steht.

7. Verbindungen nach einem der Ansprüche 1 bis 6, in denen B für Pyridin, Oxazol, Thiazol oder Imidazol steht.

8. Verbindungen nach einem der Ansprüche 1 bis 7, in denen Z für CH=CH, CH₂CH₂ oder -CH₂O- steht.

9. Verbindungen nach einem der Ansprüche 1 bis 8, in denen R² für Wasserstoff oder CO₂H steht.

10. Verbindungen nach einem der Ansprüche 1 bis 9, in denen R³ für Wasserstoff oder eine Gruppe der Formel (iii) steht, wobei D für einen 4-gliedrigen gesättigten, ein Stickstoffatom enthaltenden Ring, der durch CO₂H substituiert ist, steht, oder D für einen 5-gliedrigen aromatischen Heterozyklus steht, der 2 oder 3 Heteroatome ausgewählt aus Stickstoff und Schwefel enthält und gegebenenfalls durch CH₂CO₂H oder CF₃ substituiert ist, und R¹³ für eine Bindung, CONH, NHCH₂CONH oder CH₂ steht.

11. Verbindungen nach einem der Ansprüche 1 bis 10, in denen Q¹ für S und Q² für O oder S steht.

12. Verbindungen nach Anspruch 1, bei denen es sich um:
(±)-5-[[5-(2-Ethyl-7-methyl-4*H*-benzo[5,6]cyclohepta[1,2-*d*]oxazol-4-yl)-3,4-dihydro-2,4-dioxo-1(2*H*)-pyrimidinyl]methyl]-2-furancarbonsäure,
(±)-2-[[5-(2-Ethyl-7-methyl-4*H*-benzo[5,6]cyclohepta[1,2-*d*]oxazol-4-yl)-3,4-dihydro-2-oxo-4-thioxo-1(2*H*)-pyrimidinyl]methyl]-4-oxazolcarbonsäure,
(±)-2-[2-[[4-[5-(7-Ethyl-2-methyl-4*H*-benzo[5,6]cyclohepta[1,2-*d*]oxazol-4-yl)-3,4-dihydro-2,4-dioxo-1(2*H*)-pyrimidinyl]pyrimidin-2-yl]amino]acetylamino]-4-thiazolessigsäure,
(±)-5-[[3,4-Dihydro-5-(2-methyl-4*H*-benzo[5,6]cyclohepta[1,2-d]oxazol-4-yl)-2-oxo-4-thioxo-1(2*H*)-pyrimidinyl]methyl]-2-furancarbonsäure,
(±)-*N*-[4-[3,4-Dihydro-5-(7-hydroxy-2-methyl-4*H*-benzo[5,6]cyclohepta[1,2-*d*]oxazol-4-yl)-2,4-dioxo-1(2*H*)-pyrimidinyl]pyrimidin-2-yl]-3-azetidincarbonsäure,
(±)-2-[[5-(2-Ethyl-7-methyl-4*H*-benzo[5,6]cyclohepta[1,2-*d*]oxazol-4-yl)-3,4-dihydro-2-oxo-4-thioxo-1(2*H*)-pyrimidinyl]methyl]-*N*-[5-(trifluormethyl)-1*H*-1,2,4-triazol-3-yl]-4-oxazolcarbonsäureamid,
(±)-3-[[5-(7-Ethyl-2-methyl-4*H*-benzo[5,6]cyclohepta[1,2-*d*]oxazol-4-yl)-3,4-dihydro-2-oxo-4-thioxo-1(2*H*)-pyrimidinyl]methyl]-5-[imidazol-1-ylmethyl]benzoesäure,
(±)-2-[[5-(7-Ethyl-2-methyl-4*H*-benzo[5,6]cyclohepta[1,2-*d*]oxazol-4-yl)-3,4-dihydro-2-oxo-4-thioxo-1(2*H*)-pyrimidinyl]methyl]-4-oxazolcarbonsäure,
(±)-3-[[5-(7-Ethyl-2-methyl-4*H*-benzo[5,6]cyclohepta[1,2-*d*]oxazol-4-yl)-3,4-dihydro-2-oxo-4-thioxo-1 (2*H*)-pyrimidinyl]methyl]benzoesäure,
(±)-3-[[5-(7-Ethyl-2-methyl-4*H*-benzo[5,6]cyclohepta[1,2-*d*]oxazol-4-yl)-3,4-dihydro-2-oxo-4-thioxo-1(2*H*)-pyrimidinyl]methyl]-N-[5-(trifluormethyl)-1*H*-1,2,4-triazol-3-yl]benzolcarbonsäureamid,
(±)-3-[[5-(7-Ethyl-9,10-dihydro-2-methyl-4*H*-benzo[5,6]cyclohepta[1,2-*d*]oxazol-4-yl)-3,4-dihydro-2-oxo-4-thioxo-1(2*H*)-pyrimidinyl]methyl]benzoesäure,
(±)-3-[[5-(7-Ethyl-2-methyl-4*H*-benzo[5,6]cyclohepta[1,2-*d*]thiazol-4-yl)-3,4-dihydro-2,4-dioxo-1(2*H*)-pyrimidinyl]methyl]benzoesäure,
(±)-3-[[5-(2,7-Dimethyl-10*H*-benzo[4,5]cyclohepta[1,2-*d*]oxazol-10-yl)-3,4-dihydro-2-oxo-4-thioxo-1(2*H*)-pyrimidinyl]methyl]benzoesäure,
(±)-5-(2,7-Dimethyl-4*H*-benzo[5,6]cyclohepta [1,2-*d*]oxazol-4-yl)-1-(3-methyl-5-(5-trifluormethyl)-1*H*-1,2,4-triazol-3-yl)phenylmethyl-3,4-dihydro-4-thioxo-2(1*H*)-pyrimidinon,
(±)-3-[5-(2,7-Dimethyl-4*H*-benzo[5,6]cyclohepta[1,2-*d*]oxazol-4-yl)-3,4-dihydro-2-oxo-4-thioxo-1(2*H*)-pyrimidinylmethyl]benzoesäure,
(±)-1-Methyl-5-(2,7-dimethyl-4*H*-benzo[5,6]cyclohepta[1,2-*d*]oxazol-4-yl)-3,4-dihydro-4-thioxo-2(1H)-pyrimidinon,
(±)-1-Methyl-5-(2,6-dimethyl-4*H*-benzo[5,6]cyclohepta[1,2-*d*]oxazol-4-yl) -3,4-dihydro-4-thioxo-2(1*H*)-pyrimidinon,
(±)-1-Methyl-5-(2,7-dimethyl-4*H*-benzo[5,6]cyclohepta[1,2-*d*]thiazol-4-yl)-3,4-dihydro-4-thioxo-2(1*H*)-pyrimidinon,
(±)-1-Methyl-5-(2,7-dimethyl-10*H*-benzo[4,5]cyclohepta[1,2-*d*]oxazol-10-yl)-2,4-(1*H*, 3*H*)-pyrimidindion,
(±)-1-Methyl-5-(2,7-dimethyl-10*H*-benzo[4,5]cyclohepta[1,2-*d*]oxazol-10-yl)-3,4-dihydro-2-oxo-4-thioxo-1(2*H*)-pyrimidin,
(±)-1-Methyl-5- (2,7-dimethyl-10*H*-benzo[4,5]cyclohepta[1,2-*d*]thiazol-10-yl)-3,4-dihydro-4-thioxo-2(1H)-pyrimidinon,
(±)-5-[8-Methyl-5*H*-benzo[4,5]cyclohepta[1,2-*b*]pyridin-5-yl]-3,4-dihydro-4-thioxo-2(1*H*)-pyrimidinon,
(±)-1-Methyl-5-[8-methyl-5*H*-benzo[4,5]cyclohepta[1,2-*b*]pyridin-5-yl]-3,4-dihydro-4-thioxo-2(1*H*)-pyrimidin,
(±)-1-Methyl-5-[2,7-dimethyl-4*H*-benzo[5,6]cyclohepta[1,2-*d*]thiazol-4-yl]-2,4-(1*H*,3*H*)-pyrimidindion,
(±)-5-[7-Chlor-2-methyl-4*H*-benzo[5,6]cyclohepta[1,2-*d*]oxazol-4-yl]-1-methyl-2,4-(1*H*,3*H*)-pyrimidindion,
(±)-5-[7-Chlor-2-methyl-4*H*-benzo[5,6]cyclohepta[1,2-*d*]oxazol-4-yl]-1-methyl-3,4-dihydro-4-thioxo-2(1*H*)-pyrimidinon,
(S)-5-[7-Chlor-2-methyl-4*H*-benzo[5,6]cyclohepta[1,2-*d*]oxazol-4-yl]-1-methyl-3,4-dihydro-4-thioxo-2(1*H*)-pyrimidinon,
(±)-5-(7-Chlor-2-(2-(imidazol-1-yl)ethoxy)-4*H*-benzo[5,6]cyclohepta[1,2-*d*]thiazol-4-yl)-1-methyl-3,4-dihydro-4-thioxo-2(1H)-pyrimidinon,
(±)-1-[(1-Methyl)-1*H*-imidazol-2-ylmethyl]-5-(2,6-dimethyl-4*H*-benzo[5,6]cyclohepta[1,2-*d*]oxazol-4-yl)-3,4-dihydro-4-thioxo-2(1*H*)-pyrimidinon,
(±)-5-(2,7-Dimethyl-4*H*-benzo[5,6]cyclohepta[1,2-*d*]thiazol-4-yl)-1-(2-(*N*-pyrrolidinyl)ethyl)-3,4-dihydro-4-thioxo-2(1*H*)-pyrimidinon,
(±)-5-(2-Ethyl-7-methyl-4*H*-benzo[5,6]cyclohepta[1,2-*d*]thiazol-4-yl)-3,4-dihydro-4-thioxo-2(1*H*)-pyrimidinon,
(±)-5-(2-Ethyl-7-methyl-4*H*-benzo[5,6]cyclohepta[1,2-*d*]thiazol-4-yl)-1-(*N*-methyl)imidazol-2-ylmethyl)-3,4-dihydro-4-thioxo-2(1*H*)-pyrimidinon,
(±)-5-(2-Ethyl-7-methyl-4*H*-benzo[5,6]cyclohepta[1,2-*d*]thiazol-4-yl)-1-(2-(*N*,*N*-dimethyl)ethyl)-3,4-dihydro-4-thioxo-2(1*H*)-pyrimidinon,
(±)-5-[2-Methyl-4*H*-benzo[5,6]cyclohepta[1,2-d]oxazol-4-yl]-1-methyl-3,4-dihydro-4-thioxo-2(1H)-pyrimidinon,
(±)-1-Methyl-5-(2-methyl-7-ethyl-4*H*-benzo[5,6]cyclohepta[1,2-*d*]thiazol-4-yl)-3,4-dihydro-4-thioxo-2(1*H*)-pyrimidinon,
(±)-1-Methyl-5-(2-methyl-7-*n*-propyl-4*H*-benzo[5,6]cyclohepta[1,2-*d*]thiazol-4-yl)-3,4-dihydro-4-thioxo-2(1*H*)-pyrimidinon,
(±)-1-Methyl-5- (2-methyl-4*H*-benzo[5,6]cyclohepta[1,2-*d*]imidazol-4-yl)-3,4-dihydro-4-thioxo-2(1*H*)-pyrimidinon,
(±)-5- (7-Chlor-2-methyl-4*H*-benzo[5,6]cyclohepta[1,2-*d*]thiazol-4-yl)-3,4-dihydro-4-thioxo-2(1*H*)-pyrimidinon,
(5S,2R) -5- (7-Chlor-2-methyl-4*H*-benzo[5,6]cyclohepta[1,2-*d*]thiazol-4-yl)-1-(2-pyrrolidinylmethyl)-3,4-dihydro-4-thioxo-2(1*H*)-pyrimidinon,
(5S,2S)-5-(7-Chlor-2-methyl-4*H*-benzo[5,6]cyclohepta[1,2-*d*]thiazol-4-yl)-1-(2-pyrrolidinylmethyl)-3,4-dihydro-4-thioxo-2(1*H*)-pyrimidinon,
(±)-5-(7-Chlor-1-((imidazol-4-yl)ethyl)-2-methyl-4*H*-benzo[5,6]cyclohepta[1,2-*d*]imidazol-4-yl)-1-methyl-3,4-dihydro-4-thioxo-2(1*H*)-pyrimidinon,
(±)-5-(7-Chlor-1,2-dimethyl-4*H*-benzo[5,6]cyclohepta[1,2-*d*]imidazol-4-yl)-1-methyl-3,4-dihydro-4-thioxo-2(1*H*)-pyrimidinon,
(S)-5-(7-Chlor-1,2-dimethyl-4*H*-benzo[5,6]cyclohepta[1,2-*d*]imidazol-4-yl)-1-methyl-3,4-dihydro-4-thioxo-2(1*H*)-pyrimidinon,
(±)-5-(2,7-Dichlor-4*H*-benzo[5,6]cyclohepta[1,2-*d*]thiazol-4-yl)-1-methyl-2,4(1*H*,3*H*)-pyrimidindion,
(±)-5-(7-Chlor-4*H*-benzo[5,6]cyclohepta[1,2-*d*]thiazol-4-yl)-1-methyl-2,4(1*H*,3*H*)-pyrimidindion,
(±)-5-(7-Chlor-2-methoxy-4*H*-benzo[5,6]cyclohepta[1,2-*d*]thiazol-4-yl)-1-methyl-2,4(1*H*,3*H*)-pyrimidindion,
(±)-5-(7-Chlor-2-methylthio-4*H*-benzo[5,6]cyclohepta[1,2-*d*]thiazol-4-yl)-1-methyl-2,4(1*H*,3*H*)-pyrimidindion,
(±)-5-(2-Amino-7-chlor-4*H*-benzo[5,6]cyclohepta[1,2-*d*]thiazol-4-yl)-1-methyl-2,4(1*H*,3*H*)-pyrimidindion,
(±)-5-(7-Chlor-2-methylamino-4*H*-benzo[5,6]cyclohepta[1,2-*d*]thiazol-4-yl)-1-methyl-2,4(1*H*,3*H*)-pyrimidindion,
(±)-5-(7-Chlor-2-dimethylamino-4*H*-benzo[5, 6] cyclohepta [1, 2-*d*] thiazol-4-yl) -1-methyl-2,4(1*H*,3*H*)-pyrimidindion,
(±)-5-(7-Chlor-2- (pyrrolidin-1-yl) -4*H*-benzo[5,6]cyclohepta[1,2-*d*]thiazol-4-yl)-1-methyl-3,4-dihydro-4-thioxo-2(1*H*)-pyrimidinon,
(±)-5-(7-Chlor-2-(*N*4-methylpiperazin-1-yl))-4*H*-benzo[5,6]cyclohepta[1,2-*d*]thiazol-4-yl)-1-methyl-3,4-dihydro-4-thioxo-2(1*H*)-pyrimidinon,
(±)-5-(7-Chlor-2-(2-(4-morpholinyl)ethylamino-4H-benzo[5,6]cyclohepta[1,2-d]thiazol-4-yl)-1-methyl-3,4-dihydro-4-thioxo-2(1*H*)-pyrimidinon,
(±)-5-(7-Chlor-4*H*-benzo[5,6]cyclohepta[1,2-*d*]thiazol-4-yl)-1-methyl-3,4-dihydro-4-thioxo-2(1*H*)-pyrimidinon,
(S)-5-(7-Chlor-4*H*-benzo[5,6]cyclohepta[1,2-*d*]thiazol-4-yl)-1-methyl-3,4-dihydro-4-thioxo-2(1*H*)-pyrimidinon,
(±)-5-(2-Amino-7-chlor-4*H*-benzo[5,6]cyclohepta[1,2-*d*]thiazol-4-yl)-1-methyl-3,4-dihydro-4-thioxo-2(1*H*)-pyrimidinon,
(±)-5-(7-Chlor-2-methyl-4*H*-benzo[5,6]cyclohepta[1,2-*d*]thiazol-4-yl)-1-methyl-3,4-dihydro-4-thioxo-2(1*H*)-pyrimidinon,
(±)-5-(7-Chlor-1-ethyl-2-methyl-4*H*-benzo[5,6]cyclohepta[1,2-*d*]imidazol-4-yl) -1-methyl-3,4-dihydro-4-thioxo-2(1*H*)-pyrimidinon,
(±)-5-(7-Chlor-1-cyclopropyl-2-methyl-4*H*-benzo[5,6]cyclohepta[1,2-*d*]imidazol-4-yl) -1-methyl-3,4-dihydro-4-thioxo-2(1*H*)-pyrimidinon,
(±)-5-(7-Chlor-2-methyl-4*H*-benzo[5,6]cyclohepta[1,2-*d*]thiazol-4-yl)-1-(2-hydroxyethyl)-3,4-dihydro-4-thioxo-2(1*H*)-pyrimidinon,
(S)-5-(7-Chlor-2-methyl-4*H*-benzo[5,6]cyclohepta[1,2-*d*]thiazol-4-yl)-1-(2-hydroxyethyl)-3,4-dihydro-4-thioxo-2(1*H*)-pyrimidinon,
(±)-5-(7-Chlor-2-methyl-4*H*-benzo[5,6]cyclohepta[1,2-*d*]thiazol-4-yl)-1-(2-methoxyethyl)-3,4-dihydro-4-thioxo-2(1*H*)-pyrimidinon,
(±)-5-(7-Chlor-2-[(phenylmethyl)amino]-4*H*-benzo[5,6]cyclohepta[1,2-*d*]thiazol-4-yl)-1-methyl-3,4-dihydro-4-thioxo-2(1*H*)-pyrimidinon,
(±)-5-(7-Chlor-2-(cyclobutylamino)-4*H*-benzo[5,6]cyclohepta[1,2-*d*]thiazol-4-yl)-1-methyl-3,4-dihydro-4-thioxo-2(1*H*)-pyrimidinon,
(±)-5-(7-Chlor-2- (cyclopropylamino) -4H-benzo[5,6]cyclohepta[1,2-*d*]thiazol-4-yl)-1-methyl-3,4-dihydro-4-thioxo-2(1*H*)-pyrimidinon,
(±)-5-(7-Chlor-2-(1,3,4-thiadiazolylthio)-4*H*-benzo[5,6]cyclohepta[1,2-*d*]thiazol-4-yl)-1-methyl-2,4(1*H*,3*H*)-pyrimidindion,
(±)-5-(7-Chlor-2-mercapto-4*H*-benzo[5,6]cyclohepta[1,2-*d*]thiazol-4-yl)-1-methyl-3,4-dihydro-4-thioxo-2(1*H*)-pyrimidinon,
(±)-5-(7-Chlor-2-((imidazol-2-yl)thio)-4*H*-benzo[5,6]cyclohepta[1,2-*d*]thiazol-4-yl)-1-methyl-2,4(1*H*,3*H*)-pyrimidindion,
(±)-5-[7-Chlor-2-((1*H*-1,2,4-triazol-3-yl)thio)-4*H*-benzo[5,6]cyclohepta[1,2-*d*]thiazol-4-yl]-1-methyl-2,4(1*H*,3*H*)-pyrimidindion,
(±)-5-(7-Chlor-2-((imidazol-2-yl)thio)-4*H*-benzo[5,6]cyclohepta[1,2-*d*]thiazol-4-yl)-1-methyl-3,4-dihydro-4-thioxo-2(1*H*)-pyrimidinon,
(±)-5-(1-(2-Acetyloxyethyl)-7-chlor-2-methyl-4*H*-benzo[5,6]cyclohepta[1,2-*d*]imidazol-4-yl)-1-methyl-3,4-dihydro-4-thioxo-2(1*H*)-pyrimidinon,
(±)-5-(7-Chlor-1-(2-hydroxyethyl)-2-methyl-4*H*-benzo[5,6]cyclohepta[1,2-*d*]imidazol-4-yl)-1-methyl-3,4-dihydro-4-thioxo-2(1*H*)-pyrimidinon,
(±)-5-(9-Chlor-3-methylbenzo[*f*]oxepano[3,4-d]imidazol-6-yl)-1-methyl-3,4-dihydro-4-thioxo-2(1*H*)-pyrimidinon,
und deren pharmazeutisch unbedenkliche Salze handelt.

13. Verbindungen nach einem der Ansprüche 1 bis 12 zur Verwendung in der Therapie.

14. Verbindungen nach einem der Ansprüche 1 bis 12 zur Verwendung als entzündungshemmendes Mittel.

15. Pharmazeutische Zusammensetzungen, enthaltend eine Verbindung der Formel I oder ein Salz oder Solvat davon wie in einem der Ansprüche 1 bis 12 definiert in Verbindung mit einem pharmazeutisch unbedenklichen Adjuvans, Verdünnungsmittel oder Trägerstoff.

16. Verfahren zur Herstellung von Verbindungen der Formel I, bei dem man:
(a) eine Verbindung der Formel (II): 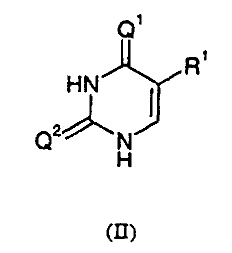 wobei Q¹ und Q² wie unter Formel (I) definiert sind und R¹ wie unter Formel (I) definiert ist oder ein geschütztes Derivat davon ist, mit einer Verbindung der Formel (III): 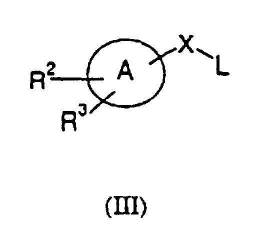 wobei R², R³ und A wie unter Formel (I) definiert sind oder geschützte Derivate davon sind, X wie unter Formel (I) definiert ist und L für eine Abgangsgruppe steht, umsetzt, oder
(b) eine Verbindung der Formel (IV): 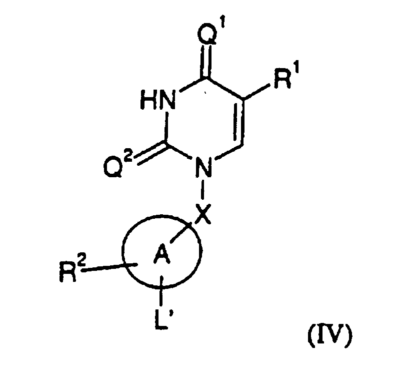 wobei Q¹, Q², R¹ und X wie unter Formel (I) definiert sind, R² und A wie unter Formel (I) definiert sind oder geschützte Derivate davon sind und L' für eine Abgangsgruppe steht, mit einer Verbindung der Formel (V), (VI) oder (VII) :
HNR²²-(CH₂)ₚ₋CONR²³-D (V)
HNR²²-CH(CO₂H)-CH₂-D (VI) 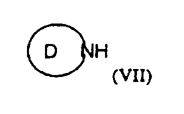
wobei R²², R²³ und p wie unter Formel (I) definiert sind und D wie unter Formel (I) definiert ist oder ein geschütztes Derivat davon ist, umsetzt, oder
(c) falls R³ für eine Gruppe -R¹¹-CO₂H und R¹¹ für SCH₂ oder NR²⁰(CH₂)ₚ steht, eine Verbindung der Formel (iv), wie oben definiert, mit einer Verbindung H-R¹¹-CO₂R²⁴, wobei R¹¹ für SCH₂ oder NR²⁰(CH₂)ₚ und R²⁴ für Wasserstoff oder eine esterbildende Gruppe steht, umsetzt;
und gegebenenfalls anschließend an (a), (b) oder (c) in beliebiger Reihenfolge:
• etwaige vorhandene Schutzgruppen abspaltet
• die Verbindung der Formel (I) in eine andere Verbindung der Formel (I) umwandelt
• ein Salz bildet.

## Revendications

1. Composé de formule I ou sel de celui-ci : 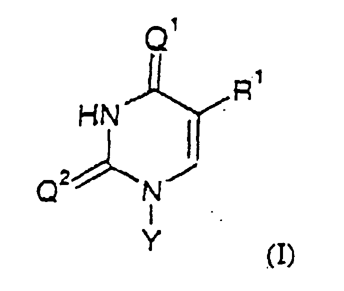 dans laquelle
Y est un hydrogène, un alkyle en C₁₋₄, un alkyle en C₁₋₄ éventuellement substitué par un hydroxy, un alcoxy, un amino, un alkylamino, un dialkylamino, un phényle, un azote et/ou un oxygène ou éventuellement substitué par un noyau cycloalkyle en C₃₋₈ renfermant éventuellement de 1 à 3 hétéroatomes et éventuellement substitué par un alkyle en C₁₋₄ ; ou Y est un groupe de formule (i) : 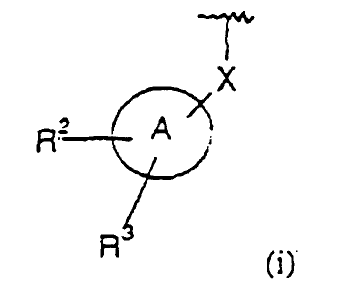
dans laquelle X est une liaison ou CH₂
où A est un noyau pyridine, pyrimidine, thiazole, oxazole, thiophène, furane ou pyridazine ;
R¹ est un groupe de formule (ii) : 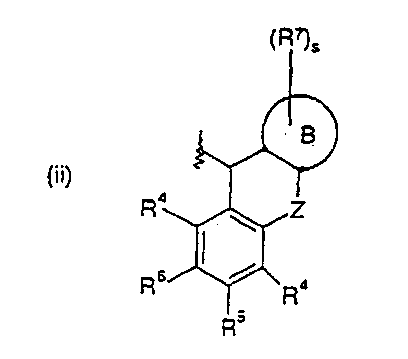 dans laquelle
R⁴ est un hydrogène, un halogène, un alcoxy en C₁₋₃, un alkylthio en C₁₋₃ ou un alkyle en C₁₋₃ (éventuellement substitué par un ou plusieurs atomes de fluor) ;
R⁵ est un hydrogène, un hydroxy, un halogène, un alkylthio en C₁₋₃, un alkyle en C₁₋₄ (éventuellement substitué par un ou plusieurs atomes de fluor), un cycloalkyle en C₃₋₄, MeOCH₂, MeSCH₂, un phényle, un pyridyle ou un alcoxy en C₁₋₃ ;
ou R⁴ et R⁵ sont -(CH₂)t- où t vaut 3 ou 4, en formant un noyau condensé ;
R⁶ est un hydrogène, un halogène, un alcoxy en C₁₋₃, un alkylthio en C₁₋₃ ou un alkyle en C₁₋₃ (éventuellement substitué par un ou plusieurs atomes de fluor) ; ou R⁵ et R⁶ sont -(CH₂)t- où t vaut 3 ou 4, en formant un noyau condensé ;
R⁷ est un hydrogène, un hydroxy, un alcoxy en C₁₋₃, un amino, un hydroxy-C₁₋₃-alkyle, -NHC₁₋₃-alkyle, -NC₁₋₃-dialkyle, -NHC₃₋₈-cycloalkyle, -NC₃₋₈-cycloalkyle, -NHphényle, -NHC₁₋₃-alkylphényle, un (hétérocycle)C₁₋₃-alkyle, un (hétérocycle)C₁₋₃-alkylthio, un (hétérocycle)C₁₋₃-alkyloxy, un (hétérocycle)C₁₋₃-alkylamino, un (hétérocycle)thio, un (hétérocycle)oxy, un (hétérocycle)amino, un alkylthio en C₁₋₃, un cyano, un thiol, un alkyle en C₁₋₃ (éventuellement substitué par un ou plusieurs atomes de fluor), un alkylamino en C₁₋₃, un C₁₋₃-alkylaminoalkyle, un carboxamido-C₁₋₃-alkyle, un acétoxy-C₁₋₃-alkyle ou un cycloalkyle en C₃₋₄ ;
S vaut 1 ou 2 ;
B est un noyau pyrrole, thiophène, furane, oxazole, thiazole, pyridine, pyrimidine, pyridazine, pyrazine ou triazine ;
Z est -CH=CH-, -CH₂CH₂- ou CH₂O ;
R² est un hydrogène, NO₂, NH₂, N(C₁₋₆alkyle)₂, CO₂H, CH₂OH, un halogène, CO₂C₁₋₆-alkyle, un alkyle en C₁₋₈ éventuellement interrompu par un ou plusieurs atomes d'oxygène, d'azote ou de soufre et éventuellement substitué par CO₂H, ou R² est un hydroxy, un imidazol-1-ylCH₂-, un phényle éventuellement substitué par CH₂CO₂H ou CONR⁹R¹⁰ où R⁹ et R¹⁰ sont indépendamment un hydrogène, un alkyle en C₁₋₆ éventuellement substitué par un hydroxy ou CO₂H et/ou éventuellement interrompu par un oxygène, un azote ou un soufre ;
R³ est un hydrogène, R¹¹CO₂H, R¹¹PO(OH)₂, R¹²tétrazol-5-yle, COR¹³, NR¹⁴R¹⁵, CH₂NR¹⁶CH₂CO₂H, un alkyle en C₁₋₈ éventuellement interrompu par un ou plusieurs atomes d'oxygène, de soufre ou d'azote et éventuellement substitué par CO₂H, ou R³ est un groupe de formule (iii) : 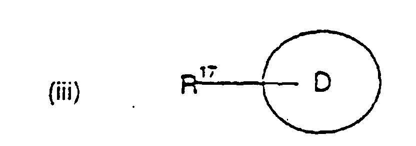
dans laquelle D est un noyau saturé à 4, 5 ou 6 chaînons renfermant un azote éventuellement substitué par un hydroxy et substitué par CO₂H ou CONH-hét où hét est un tétrazol-5-yle ou un noyau thiazole ou thiadiazole substitué par CH₂CO₂H, ou D est un noyau phényle ou un noyau hétérocyclique aromatique à 5 chaînons renfermant de 1 à 3 hétéroatomes choisis parmi l'azote, l'oxygène ou le soufre éventuellement substitué par un ou plusieurs groupes choisis parmi CF₃, CO₂H, CH₂OH, un alkyle en C₁₋₆, éventuellement interrompu par un ou plusieurs atomes d'oxygène, (CH₂)pCO₂H, C(CO₂H)=NOMe, un tétrazol-5-yle, CH₂tétrazol-5-yle, CH₂CON(CH₂CO₂H)₂ ou CH₂COR¹⁸ ;
où R¹¹ est OCH₂, (CH₂)p, SCH₂, CONHCH₂, NHCH(R¹⁹) ou NR²⁰(CH₂)p ;
R¹² est une liaison, (CH₂)p, OCH₂, SCH₂, CONH, CONHCH₂, CONHCH₂CONH, NHCH₂CONH, NHCH(R⁹) ;
R¹³ est OH, N(CH₂CO₂H)₂, NHS(O)₂R²¹ ou un groupe de formule (iv) : 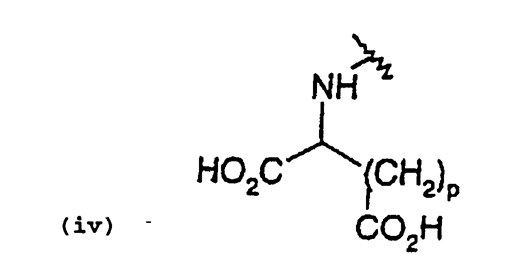
R¹⁴ et R¹⁵ sont indépendamment un hydrogène, CH₂CO₂H, CHPh₂ ou C(=S)CH₂CH₂CO₂H ;
R¹⁶ est un hydrogène, un alkyle en C₁₋₆ ou CO₂CH₂Ph ;
R¹⁷ est une liaison, un atome de soufre, CONH, CH₂, CH₂O, OCH₂, un groupe -NR²²CH(CO₂H)CH₂- ou un groupe CONR²²(CH₂)pCONR²³ ou NR²²(CH₂)pCONR²³ ;
R¹⁸ est un groupe de formule (iv) telle que définie ci-dessus ou un groupe de formule (v) : 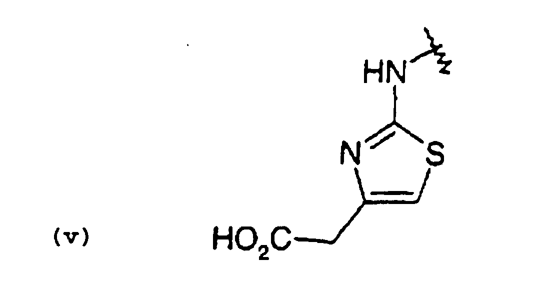
R¹⁹ est un hydrogène, un alkyle en C₁₋₆ éventuellement substitué par un hydroxy et/ou éventuellement interrompu par un oxygène, un azote ou un soufre ;
R²⁰ est un hydrogène ou un alkyle en C₁₋₆ ;
p vaut 1 ou 2 ;
R²¹ est NH₂ ou un alkyle en C₁₋₆ éventuellement interrompu par un oxygène ou un azote ;
R²² et R²³ sont indépendamment un hydrogène, un alkyle en C₁₋₆ ; et
Q¹ et Q² représentent chacun indépendamment un atome O ou S.

2. Composé selon la revendication 1, dans lequel Y est un hydrogène, un méthyle, un 2-(NN-diméthylamino)éthyle, un 2-(N(pyrrolidinyl)éthyle, un 2-(pyrrolidinyl)méthyle, un (1-méthyl)-1H-imidazole-2-ylméthyle, un hydroxyéthyle, un méthoxyéthyle ou un groupe de formule (i), et X est une liaison ou un groupe CH₂.

3. Composé selon la revendication 1 ou 2, dans lequel A est un furane, un oxazole, une pyrimidine ou un phényle.

4. Composé selon l'une quelconque des revendications 1 à 3, dans lequel R⁴ est un hydrogène et R⁶ est un hydrogène ou un méthyle.

5. Composé selon l'une quelconque des revendications 1 à 4, dans lequel R⁵ est un hydrogène, un chlore, un hydroxy ou un alkyle en C₁₋₆.

6. Composé selon l'une quelconque des revendications 1 à 5, dans lequel R⁷ est un alkyle en C₁₋₃, un hydrogène, un chlore, un méthoxy, un méthylthio, un amino, un méthylamino, un diméthylamino, un pyrrolidin-1-yle, un N-4-méthyl-pipérazin-1-yle, un 2-(2-(4-morpholinyl)éthylamino, un cyclopropyle, un benzylamino, un cyclobutylamino, un cyclopropylamino, un imidazol-2-ylthio, un thiadiazol-2-ylthio, un thiol, un triazol-2-ylthio, un imidazol-1-yléthoxy, un acétoxyéthyle, un hydroxyéthyle ou un imidazol-4-yléthyle.

7. Composé selon l'une quelconque des revendications 1 à 6, dans lequel B est une pyridine, un oxazole, un thiazole ou un imidazole.

8. Composé selon l'une quelconque des revendications 1 à 7, dans lequel Z est CH=CH, CH₂CH₂ ou -CH₂O-.

9. Composé selon l'une quelconque des revendications 1 à 8, dans lequel R² est un hydrogène ou CO₂H.

10. Composé selon l'une quelconque des revendications 1 à 9, dans lequel R³ est un hydrogène, un groupe de formule (iii) dans laquelle D est un noyau saturé à 4 chaînons renfermant un atome d'azote et substitué par CO₂H, ou D est un hétérocycle aromatique à 5 chaînons renfermant 2 ou 3 hétéroatomes choisis parmi l'azote et le soufre et éventuellement substitué par CH₂CO₂H ou CF₃ et R¹³ est une liaison, CONH, NHCH₂CONH ou CH₂.

11. Composé selon l'une quelconque des revendications 1 à 10, dans lequel Q¹ est S et Q² est O ou S.

12. Composé selon la revendication 1, qui est :
l'acide (±) -5-[[5-(2-éthyl-7-méthyl-4*H*-benzo[5, 6]cyclohepta[1,2-*d*]oxazol-4-yl)-3,4-dihydro-2,4-dioxo-1(2*H*)-pyrimidinyl]méthyl]-2-furanecarboxylique,
l'acide (±)-2-[[5- (2-éthyl-7-méthyl-4*H*-benzo[5, 6]cyclohepta[1,2-*d*]oxazol-4-yl)-3,4-dihydro-2-oxo-4-thioxo-1(2*H*)-pyrimidinyl]méthyl]-4-oxazolecarboxylique,
l'acide (±)-2-[2-[[4-[5-(7-éthyl-2-méthyl-4*H*-benzo[5,6]-cyclohepta[1,2-*d*]oxazol-4-yl)-3,4-dihydro-2,4-dioxo-1 (2*H*)-pyrimidinyl]pyrimidin-2-yl]amino]acétylamino]-4-thiazoleacétique,
l'acide (±)-5-[[3,4-dihydro-5-(2-méthyl-4*H*-benzo[5, 6]-cyclohepta[1,2-*d*]oxazol-4-yl)-2-oxo-4-thioxo-1(2*H*)-pyrimidinyl]méthyl]-2-furanecarboxylique,
l'acide (±)-N-[4-[3,4-dihydro-5-(7-hydroxy-2-méthyl-4*H*-benzo[5, 6]cyclohepta[1,2-*d*]oxazol-4-yl)-2,4-dioxo-1(2*H*)-pyrimidinyl]pyrimidin-2-yl]-3-azétidinecarboxylique,
le (±)-2-[[5-(2-éthyl-7-méthyl-4*H*-benzo[5,6]cyclohepta-[1,2-*d*]oxazol-4-yl)-3,4-dihydro-2-oxo-4-thioxo-1(2*H*)-pyrimidinyl]méthyl]-*N*-[5-(trifluorométhyl)-1*H*-1,2,4-triazol-3-yl]-4-oxazolecarboxamide,
l'acide (±)-3-[[5-(7-éthyl-2-méthyl-4*H*-benzo[5, 6]cyclohepta[1,2-*d*]oxazol-4-yl)-3,4-dihydro-2-oxo-4-thioxo-1 (2*H*)-pyrimidinyl]méthyl]-5-[imidazol-1-ylméthyl]benzoïque
l'acide (±)-2-[[5-(7-éthyl-2-méthyl-4*H*-benzo[5,6]cyclohepta[1,2-*d*]oxazol-4-yl)-3,4-dihydro-2-oxo-4-thioxo-1(2*H*)-pyrimidinyl]méthyl]-4-oxazolecarboxylique,
l'acide (±)-3-[[5-(7-éthyl-2-méthyl-4*H*-benzo[5,6]cyclohepta[1,2-*d*]oxazol-4-yl)-3,4-dihydro-2-oxo-4-thioxo-1 (2*H*)-pyrimidinyl]méthyl]benzoïque,
le (±)-3-[[5-(7-éthyl-2-méthyl-4*H*-benzo[5,6]cyclohepta[1,2-*d*]oxazol-4-yl)-3,4-dihydro-2-oxo-4-thioxo-1(2*H*)-pyrimidinyl]méthyl]-*N*-[5-(trifluorométhyl)-1*H*-1,2,4-triazol-3-yl]benzènecarboxamide,
l'acide (±)-3-[[5-(7-éthyl-9,10-dihydro-2-méthyl-4*H*-benzo[5,6]cyclohepta[1,2-*d*]oxazol-4-yl)-3,4-dihydro-2-oxo-4-thioxo-1(2*H*)-pyrimidinyl]méthyl]benzoïque,
l'acide (±)-3-[[5-(7-éthyl-2-méthyl-4*H*-benzo[5, 6]cyclohepta[1,2-*d*]thiazol-4-yl)-3,4-dihydro-2,4-dioxo-1(2*H*)-pyrimidinyl]méthyl]benzoïque,
l'acide (±)-3-[[5-(2,7-diméthyl-10*H*-benzo[4,5]cyclohepta[1,2-*d*]oxazol-10-yl)-3,4-dihydro-2-oxo-4-thioxo-1 (2*H*)-pyrimidinyl]méthyl]benzoïque,
la (±)-5-(2,7-diméthyl-4*H*-benzo[5,6]cyclohepta[1,2-*d*]oxazol-4-yl)-1-(3-méthyl-5-(5-trifluorométhyl)-1*H*-1,2,4-triazol-3-yl)phénylméthyl-3,4-dihydro-4-thioxo-2(1*H*)pyrimidinone,
l'acide (±)-3-[5- (2,7-diméthyl-4*H*-benzo[5,6]cyclohepta[1,2-*d*]oxazol-4-yl)-3,4-dihydro-2-oxo-4-thioxo-1 (2*H*)-pyrimidinylméthyl]benzoïque,
la (±)-1-méthyl-5-(2,7-diméthyl-4*H*-benzo[5,6]cyclohepta[1,2-*d*]oxazol-4-yl)-3,4-dihydro-4-thioxo-2(1*H*) -pyrimidinone,
la (±)-1-méthyl-5-(2,6-diméthyl-4*H*-benzo[5,6]cyclohepta[1,2-*d*]oxazol-4-yl)-3,4-dihydro-4-thioxo-2(1*H*)-pyrimidinone,
la (±)-1-méthyl-5-(2,7-diméthyl-4*H*-benzo[5,6]cyclohepta[1,2-*d*]thiazol-4-yl)-3,4-dihydro-4-thioxo-2(1*H*)-pyrimidinone,
la (±)-1-méthyl-5-(2,7-diméthyl-10*H*-benzo[4,5]cyclohepta[1,2-*d*]oxazol-10-yl)-2,4-(1*H*,*3H*)-pyrimidinedione,
la (±)-1-méthyl-5-(2,7-diméthyl-10*H*-benzo[4,5]cyclohepta[1,2-*d*]oxazol-10-yl)-3,4-dihydro-2-oxo-4-thioxo-1(2H)-pyrimidine,
la (±)-1-méthyl-5-(2,7-diméthyl-10*H*-benzo[4,5]cyclohepta[1,2-*d*]thiazol-10-yl)-3,4-dihydro-4-thioxo-2(1*H*)-pyrimidinone,
la (±)-5-[8-méthyl-5*H*-benzo[4, 5]cyclohepta[1,2-*b*]pyridin-5-yl]-3,4-dihydro-4-thioxo-2(1*H*)-pyrimidinone,
la (±)-1-méthyl-5-[8-méthyl-5*H*-benzo[4, 5]cyclohepta[1,2-b]pyridin-5-yl]-3,4-dihydro-4-thioxo-2(1*H*)-pyrimidine,
la (±)-1-méthyl-5-[2,7-diméthyl-4*H*-benzo[5, 6]cyclohepta[1,2-*d*]thiazol-4-yl]-2,4(1*H*, 3*H*) -pyrimidinedione,
la (±) -5-[7-chloro-2-méthyl-4*H*-benzo[5, 6]cyclohepta[1,2-*d*]oxazol-4-yl]-1-méthyl-2,4(1*H*, *3H*)-pyrimidinedione,
la (±) -5-[7-chloro-2-méthyl-4*H*-benzo[5,6]cyclohepta[1,2-*d*]oxazol-4-yl]-1-méthyl-3,4-dihydro-4-thioxo-2(1*H*)-pyrimidinone,
la (S)-5-[7-chlore-2-méthyl-4H-benzo[5, 6]cyclohepta[1,2-*d*]oxazol-4-yl]-1-méthyl-3,4-dihydro-4-thioxo-2(1*H*)-pyrimidinone,
la (±)-5-(7-chloro-2-(2-(imidazol-1-yl)éthoxy)-4*H*-benzo[5, 6]cyclohepta[1,2-*d*]thiazol-4-yl)-1-méthyl-3,4-dihydro-4-thioxo-2(1*H*)-pyrimidinone,
la (±)-1-[(1-méthyl)-1*H*-imidazol-2-ylméthyl]-5-(2,6-diméthyl-4H-benzo[5, 6]cyclohepta[1,2*-*d]oxazol-4-yl)-3,4-dihydro-4-thioxo-2(1*H*)-pyrimidinone,
la (±)-5-(2,7-diméthyl-4*H*-benzo[5,6]cyclohepta[1,2-*d*]thiazol-4-yl)-1-(2-(*N*-pyrrolidinyl)éthyl)-3,4-dihydro-4-thioxo-2(1*H*)-pyrimidinone,
la (±)-5-(2-éthyl-7-méthyl-4*H*-benzo[5,6]cyclohepta[1,2-*d*]thiazol-4-yl)-3,4-dihydro-4-thioxo-2(1*H*)-pyrimidinone,
la (±)-5-(2-éthyl-7-méthyl-4*H*-benzo[5,6]cyclohepta[1,2-*d*]thiazol-4-yl)-1-(*N*-méthyl)imidazol-2-ylméthyl)-3,4-dihydro-4-thioxo-2(1H)-pyrimidinone,
la (±)-5-(2-éthyl-7-méthyl-4*H*-benzo[5,6]cyclohepta[1,2-*d*]thiazol-4-yl)-1-(2-(*N,N*-diméthyl)éthyl)-3,4-dihydro-4-thioxo-2(1*H*)-pyrimidinone,
la (±)-5-[2-méthyl-4*H*-benzo[5,6]cyclohepta[1,2-*d*]oxazol-4-yl)-1-méthyl-3,4-dihydro-4-thioxo-2(1*H*)-pyrimidinone,
la (±)-1-méthyl-5-(2-méthyl-7-éthyl-4*H*-benzo[5,6]cyclohepta[1,2-*d*]thiazol-4-yl)-3,4-dihydro-4-thioxo-2(1*H*)-pyrimidinone,
la (±)-1-méthyl-5-(2-méthyl-7-*n*-propyl-4*H*-benzo[5,6]cyclohepta[1,2-*d*]thiazol-4-yl)-3,4-dihydro-4-thioxo-2(1*H*)-pyrimidinone,
la (±)-1-méthyl-5-(2-méthyl-4*H*-benzo[5,6]cyclohepta[1,2-*d*]imidazol-4-yl)-3,4-dihydro-4-thioxo-2(1*H*)-pyrimidinone,
la (±)-5-(7-chloro-2-méthyl-4*H*-benzo[5,6]cyclohepta[1,2-*d*]thiazol-4-yl)-3,4-dihydro-4-thioxo-2(1*H*)-pyrimidinone,
la (5S,2R)-5-(7-chloro-2-méthyl-4H-benzo[5,6]cyclohepta[1,2-*d*]thiazol-4-yl)-1-(2-pyrrolidinylméthyl)-3,4-dihydro-4-thioxo-2(1*H*)-pyrimidinone,
la (5S,2S)-5-(7-chloro-2-méthyl-4*H*-benzo[5,6]cyclohepta[1,2-d]thiazol-4-yl)-1-(2-pyrrolidinylméthyl)-3,4-dihydro-4-thioxo-2(1*H*)-pyrimidinone,
la (±)-5-(7-chloro-1-((imidazol-4-yl)éthyl)-2-méthyl-4*H*-benzo[5,6]cyclohepta[1,2-*d*]imidazol-4-yl)-1-méthyl-3,4-dihydro-4-thioxo-2(1*H*)-pyrimidinone,
la (±)-5-(7-chloro-1,2-diméthyl-4*H*-benzo[5, 6]cyclohepta[1,2-*d*]imidazol-4-yl)-1-méthyl-3,4-dihydro-4-thioxo-2(1*H*)-pyrimidinone,
la (S)-5-(7-chloro-1,2-diméthyl-4*H*-benzo[5, 6]cyclohepta[1,2-*d*]imidazol-4-yl)-1-méthyl-3,4-dihydro-4 thioxo-2(1*H*)-pyrimidinone,
la (±)-5- (2,7-dichloro-4*H*-benzo[5, 6]cyclohepta[1,2-*d*]thiazol-4-yl)-1-méthyl-2,4(1*H*,3*H*)-pyrimidinedione,
la (±)-5-(7-chloro-4*H*-benzo[5,6]cyclohepta[1,2-*d*]thiazol-4-yl)-1-méthyl-2,4(1*H*,3*H*)-pyrimidinedione,
la (±)-5-(7-chlore-2-méthoxy-4*H*-benzo[5, 6]cyclohepta[1,2-*d*]thiazol-4-yl)-1-méthyl-2,4(1*H*, 3*H*)-pyrimidinedione,
la (±)-5-(7-chloro-2-méthylthio-4*H*-benzo[5,6]cyclohepta[1,2-*d*]thiazol-4-yl)-1-méthyl-2,4(1*H*,3*H*)-pyrimidinedione,
la (±)-5-(2-amino-7-chloro-4H-benzo[5,6]cyclohepta[1,2-d]thiazol-4-yl)-1-méthyl-2,4(1*H*,3*H*)-pyrimidinedione,
la (±)-5-(7-chloro-2-méthylamino-4*H*-benzo[5,6]cyclohepta[1,2-*d*]thiazol-4-yl)-1-méthyl-2,4(1*H*,3*H*)-pyrimidinedione,
la (±)-5-(7-chloro-2-diméthylamino-4*H*-benzo[5,6]cyclohepta[1,2-*d*]thiazol-4-yl)-1-méthyl-2,4(1*H*,3*H*)-pyrimidinedione,
la (±)-5-(7-chloro-2-(pyrrolidin-1-yl)-4*H*-benzo[5,6]cyclohepta[1,2-*d*]thiazol-4-yl)-1-méthyl-3,4-dihydro-4-thioxo-2(1H)-pyrimidinone,
la (±)-5-(7-chloro-2-(*N*4-méthyl-pipérazin-1-yl))-4*H*-benzo[5, 6]cyclohepta[1,2-*d*]thiazol-4-yl)-1-méthyl-3,4-dihydro-4-thioxo-2(1*H*)-pyrimidinone,
la (±)-5-(7-chloro-2-(2-(4-morpholinyl)éthylamino)-4*H*-benzo[5, 6]cyclohepta[1,2-*d*]thiazol-4-yl)-1-méthyl-3,4-dihydro-4-thioxo-2(1*H*)-pyrimidinone,
la (±)-5-(7-chloro-4*H*-benzo[5,6]cyclohepta[1,2-*d*]thiazol-4-yl)-1-méthyl-3,4-dihydro-4-thioxo-2(1*H*)-pyrimidinone,
la (S)-5-(7-chloro-4*H*-benzo[5,6]cyclohepta[1,2-*d*]thiazol-4-yl)-1-méthyl-3,4-dihydro-4-thioxo-2(1*H*)-pyrimidinone,
la (±)-5-(2-amino-7-chloro-4*H*-benzo[5,6]cyclohepta[1,2-*d*]thiazol-4-yl)-1-méthyl-3,4-dihydro-4-thioxo-2(1*H*)-pyrimidinone,
la (±)-5-(7-chloro-2-méthyl-4*H*-benzo[5, 6]cyclohepta[1,2-*d*]thiazol-4-yl)-1-méthyl-3,4-dihydro-4-thioxo-2(1*H*)-pyrimidinone,
la (±)-5-(7-chloro-1-éthyl-2-méthyl-4*H*-benzo[5,6]cyclohepta[1,2-*d*]imidazol-4-yl)-1-méthyl-3,4-dihydro-4-thioxo-2(1*H*)-pyrimidinone,
la (±)-5-(7-chloro-1-cyclopropyl-2-méthyl-4*H*-benzo[5,6]-cyclohepta[1,2-*d*]imidazol-4-yl)-1-méthyl-3,4-dihydro-4-thioxo-2(1*H*)-pyrimidinone,
la (±)-5-(7-chloro-2-méthyl-4*H*-benzo[5,6]cyclohepta[1,2-*d*]thiazol-4-yl)-1-(2-hydroxyéthyl-3,4-dihydro-4-thioxo-2(1*H*)-pyrimidinone,
la (S)-5-(7-chloro-2-méthyl-4*H*-benzo[5,6]cyclohepta[1,2-*d*]thiazol-4-yl)-1-(2-hydroxyéthyl)-3,4-dihydro-4-thioxo-2(1*H*)-pyrimidinone,
la (±)-5-(7-chloro-2-méthyl-4*H*-benzo[5,6]cyclohepta[1,2-*d*]thiazol-4-yl)-1-(2-méthoxyéthyl)-3,4-dihydro-4-thioxo-2(1*H*)-pyrimidinone,
la (±)-5-(7-chlore-2-[(phénylméthyl)amine]-4*H*-benzo[5,6]-cyclohepta[1,2-*d*]thiazol-4-yl)-1-méthyl-3,4-dihydro-4-thioxo-2(1*H*)-pyrimidinone,
la (±)-5-(7-chloro-2-(cyclobutylamino)-4*H*-benzo[5, 6]-cyclohepta[1,2-*d*]thiazol-4-yl)-1-méthyl-3,4-dihydro-4-thioxo-2(1*H*)-pyrimidinone,
la (±)-5-(7-chloro-2-(cyclopropylamino)-4*H*-benzo[5, 6]-cyclohepta[1,2-*d*]thiazol-4-yl)-1-méthyl-3,4-dihydro-4-thioxo-2(1*H*)-pyrimidinone,
la (±)-5-(7-chloro-2-(1,3,4-thiadiazolylthio)-4*H*-benzo-[5,6]cyclohepta[1,2-*d*]thiazol-4-yl)-1-méthyl-2,4- (1*H*, 3*H*)-pyrimidinedione,
la (±)-5-(7-chloro-2-mercapto-4*H*-benzo[5,6]cyclohepta-[1,2-*d*]thiazol-4-yl)-1-méthyl-3,4-dihydro-4-thioxo-2(1H)-pyrimidinone,
la (±)-5-(7-chloro-2-((imidazol-2-yl)thio)-4*H*-benzo-[5,6]cyclohepta[1,2-*d*]thiazol-4-yl)-1-méthyl-2,4-(1*H*, 3*H*)-pyrimidinedione,
la (±)-5-[7-chloro-2-((1*H*-1,2,4-triazol-3-yl)thio)-4*H*-benzo[5,6]cyclohepta[1,2-*d*]thiazol-4-yl]-1-méthyl-2,4-(1*H*, 3*H*)-pyrimidinedione,
la (±)-5-(7-chloro-2-((imidazol-2-yl)thio)-4*H*-benzo-[5,6]cyclohepta[1,2-*d*]thiazol-4-yl)-1-méthyl-3,4-dihydro-4-thioxo-2(1*H*)-pyrimidinone,
la (+)-5-(1-(2-acétyloxyéthyl)-7-chloro-2-méthyl-4*H*-benzo[5,6]cyclohepta[1,2-*d*]imidazol-4-yl)-1-méthyl-3,4-dihydro-4-thioxo-2(1*H*)-pyrimidinone,
la (+)-5-(7-chloro-1-(2-hydroxyéthyl)-2-méthyl-4H-benzo[5,6]cyclohepta[1,2-*d*]imidazol-4-yl)-1-méthyl-3,4-dihydro-4-thioxo-2(1*H*)-pyrimidinone,
la (±)-5-(9-chloro-3-méthylbenzo[*f*]oxépano[3,4-d]imidazol-6-yl)-1-méthyl-3,4-dihydro-4-thioxo-2(1*H*)-pyrimidinone,
et leurs sels pharmaceutiquement acceptables.

13. Composé selon l'une quelconque des revendications 1 à 12 destiné à être utilisé en thérapie.

14. Composé selon l'une quelconque des revendications 1 à 12 destiné à être utilisé en tant qu'agent anti-inflammatoire.

15. Composition pharmaceutique comprenant un composé de formule I ou un sel ou solvate de celui-ci, comme défini dans l'une quelconque des revendications 1 à 12, en association avec un adjuvant, diluant ou support pharmaceutiquement acceptable.

16. Procédé de préparation de composés de formule I comprenant :
(a) la réaction d'un composé de formule (II) : 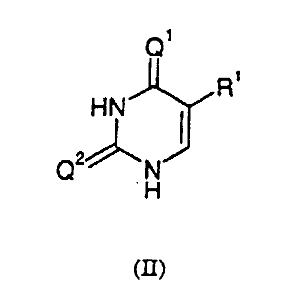 dans laquelle Q¹ et Q² sont tels que définis à la formule (I) et R¹ est tel que défini à la formule (I) ou est un dérivé protégé de celui-ci, avec un composé de formule (III) : 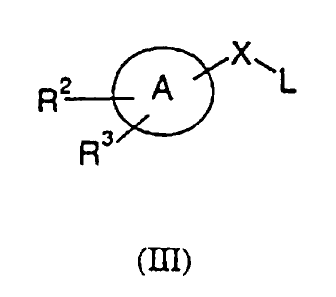 dans laquelle R², R³ et A sont tels que définis à la formule (I) ou sont des dérivés protégés de celui-ci, X est tel que défini à la formule (I) et L est un groupe partant, ou
b) la réaction d'un composé de formule (IV) : 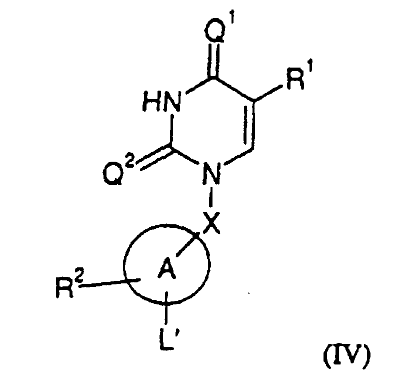 dans laquelle Q¹, Q², R¹ et X sont tels que définis à la formule (I), R² et A sont tels que définis à la formule (I) ou sont des dérivés protégés de ceux-ci et L' est un groupe partant, avec un composé de formules (V), (VI) ou (VII) :
HNR²²-(CH₂)ₚ₋CONR²³-D (V)
HNR²²-CH(CO₂H)-CH₂-D (VI) 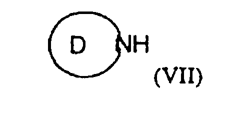
dans lesquelles R²², R²³ et p sont tels que définis à la formule (I) et D est tel que défini à la formule (I) ou est un dérivé protégé de celui-ci, ou
c) lorsque R³ est un groupe -R¹¹-CO₂H et R¹¹ est SCH₂ ou NR²⁰(CH₂)p, la réaction d'un composé de formule (iv) telle que définie ci-dessus, avec un composé H-R¹¹-CO₂R²⁴ dans lequel R¹¹ est SCH₂ ou NR²⁰(CH₂)ₚ et R²⁴ est un hydrogène ou un groupe formant un ester ;
et éventuellement après (a), (b) ou (c), dans un ordre quelconque :
• l'élimination de tous groupes protecteurs
• la transformation du composé de formule (I) en un autre composé de formule (I)
• la formation d'un sel.
